# EUROPEAN PATENT APPLICATION

(11) **EP 4 488 276 A1**
(43) Date of publication of application: **08.01.2025**
(21) Application number: 23763073.6
(22) Date of filing: 02.03.2023
(51) Int. Cl.: C07D 471/04, A61K 31/437, A61K 31/416, A61K 31/519, A61P 35/00, C07D 209/08, C07D 231/56, C07D 487/04

(54) **NOVEL HETEROCYCLIC COMPOUND AND PHARMACEUTICAL COMPOSITION COMPRISING SAME**

(30) Priority: 04.03.2022 KR 20220028075
(71) Applicant: Samjin Pharmaceutical Co., Ltd., Seoul 04054 (KR)
(72) Inventor: KWON, Ho Seok, Suwon-Si Gyeonggi-do 16557 (KR); LEE, Jae Woong, Suwon-Si Gyeonggi-do 16713 (KR); KIM, Hyun Tae, Suwon-si Gyeonggi-do 16687 (KR); LEE, Janghyun, Incheon 22762 (KR); LEE, Hyemi, Bucheon-si Gyeonggi-do 14588 (KR); HYUN, Joo Young, Gwangju-si Gyeonggi-do 12771 (KR); YOO, Jong Seok, Seoul 07251 (KR); LEE, Jeongmin, Seoul 05503 (KR); KIM, So Hee, Seoul 08295 (KR); KIM, Li Kyung, Seoul 07632 (KR)
(74) Representative: Gille Hrabal Partnerschaftsgesellschaft mbB Patentanwälte
(86) International application number: PCT/IB2023/051929
(87) International publication number: WO 2023/166450

(57) **Abstract**

The present invention relates to a compound represented by chemical formula I, a stereoisomer thereof, a pharmaceutically acceptable salt thereof, a hydrate or a solvate thereof, and a pharmaceutical composition comprising same.

## Description

### Technical Field

The present invention relates to a novel heterocyclic compound, a stereoisomer thereof, a pharmaceutically acceptable salt thereof, a hydrate thereof or a solvate thereof, and a pharmaceutical composition including the same.

### Background Art

A hippo signaling pathway is a major signaling system which regulates the proliferation of normal cells in the human body and the growth, size and the like of tissues and organs such as the skin, muscle, lung, liver, etc. The hippo signaling pathway is known as a tumor suppressor in normal cells or tissues, but it has been reported to be involved in diseases such as tumors, thus suggesting that the hippo signaling pathway plays an important role in cancer development and growth.

In addition, it has been reported that the hippo signaling pathway may also cross-talk with other signaling systems, such as the Wnt, EGFR, TGF-beta signaling system or the like, which have been known to be involved in tumors, such that the hippo signaling pathway may be involved in various carcinomas in the human body.

The hippo signaling pathway starts from a kinase cascade consisting of MST-1/2 and LATS-1/2 and is activated by phosphorylating a YAP or TAZ protein, which is a transcription activation factor.

When the hippo signaling pathway is not activated, the YAP protein is not phosphorylated, but moves from a cytoplasm into a nucleus and binds to TEA domain (TEAD) proteins TEAD1, 2, 3, and 4 present in the nucleus so as to act as a transcription factor of genes involved in cell proliferation, such as CTGF, Cyr61, FGF 1, and the like, thereby inducing an expression thereof. On the contrary, when the hippo signaling pathway is activated, the YAP protein is phosphorylated and the phosphorylated YAP protein is ubiquitinated in the cytoplasm and is degraded in proteasome or binds to a VGLL4 protein, which is a tumor suppressor, and thus may not move into the nucleus, resulting in the inhibition of the expression of genes involved in cell growth because of failing to bind to the TEAD proteins.

Recent reports have shown that the cause of abnormal cell proliferation, such as various tumors, is the abnormal expression of related genes which act on the hippo signaling pathway, leading to the abnormal regulation of the hippo signaling pathway (Cell 2011 Nov 11; 147(4): 759-72, Folia Histochem Cytobiol. 2015; 53(2):105-19). In particular, it has been reported that activation of the hippo signaling pathway is inhibited by about 50% in patients with mesothelioma with poor prognosis due to genetic variation in NF2 proteins (Transl Lung Cancer Res. 2014; 3: 75-83, Cancer Res. 1995; 55: 1227-31).

In order to inhibit the overexpression of genes involved in cell proliferation due to the abnormal regulation of the hippo signaling pathway, inhibiting the binding of YAP proteins and TEAD proteins in the cell nucleus is known as the only method. This is because MST-1/2 kinase and LATS-1/2 kinase, which are the upper proteins of the hippo signaling pathway, phosphorylate YAP proteins to inhibit a translocation thereof into the nucleus, and thus if the function of these kinases is inhibited, phosphorylation of the YAP proteins is not achieved, and thus YAP proteins are transferred into the nucleus, and then bind to TEAD proteins, thereby further promoting the overexpression of genes. In addition, YAP proteins have a very flexible structure without a specific tertiary structure, and thus hardly become a target of a therapeutic material, and for this reason, TEAD proteins have currently become an only target capable of inhibiting the hippo signaling pathway. Furthermore, recent studies have shown that S-palmitoylation of TEAD proteins per se is a very essential element in the stability and function of TEAD proteins (Cell Rep. 2020 Jun 23;31(12):107809).

Accordingly, various methods have been attempted to interfere with the binding between YAP proteins and TEAD proteins or to inhibit the function of TEAD proteins by binding to TEAD proteins.

TEAD proteins composed of TEAD1, 2, 3, and 4 have a very high similarity of about 70% in amino acid composition between each other. All of the TEAD proteins are combined with a fatty acid called palmitate in a specific cysteine group and undergo a protein post-translational modification process called palmitoylation. This process is known to be necessary for expressing genes by enhancing the structural stability of TEAD proteins or promoting the binding with YAP or TAZ proteins (Chan, P. et al. Nat. Chem. Biol. (2016) 12: 282-289; Noland, C. L. et al. Structure (2016) 24: 179-186; Mesrouze, Y. et al. Protein Sci. (2017) 26: 2399-2409).

While researches have been recently conducted on peptides which mimic a part of the YAP protein binding to the TEAD proteins, these peptide materials showed a strong binding force with TEAD proteins in in vitro experiments (Furet, P. et al. Bioorg. Med. Chem. Lett. (2019) 29: 2316-2319), but resulted in a failure to actually develop a therapeutic agent due to insufficient intracellular permeability and in vivo instability. In order to solve the problems of these peptides, attempts have been made to develop low molecular weight compounds which have excellent intracellular permeability and stability and bind to an interface in which the YAP protein interacts with TEAD proteins (Zhou, W. et al. Anal. Biochem. (2019) 586: 113413), but these low molecular weight compounds have not been developed as a therapeutic substance for inhibiting the hippo signaling pathway due to a weak YAP-TEAD inhibitory effect.

Studies to date show that it is very difficult to develop therapeutic substances with mechanisms for inhibiting an interface in the TEAD protein, which directly binds to the YAP protein. In order to solve this difficulty, it has been recently found that a palmitoylation process becomes more important in TEAD proteins, a palmitate fatty acid is a low molecular weight compound of 16 carbons, and a palmitoylation site is very similar in all TEAD proteins, and thus a strategy for developing a low molecular weight compound which binds to the palmitoylation site of TEAD protein has been reported.

It is known that a variety of low molecular weight compounds including flufenamic acid may inhibit the palmitoylation of TEAD protein (Pobbati, A. V. et al. Structure (2015) 23: 2076-2086), and it has been reported that these low molecular weight compounds inhibit the proliferation of cancer cells by inhibiting the palmitoylation of TEAD protein, thereby exhibiting stronger efficacy than the existing substances mentioned above (Holden, J. K. et al. Cell Rep. (2020) 31: 107809; Kaneda, A. et al. Am. J. Cancer Res. (2020) 10: 4399-4415).

In conclusion, the hippo signaling pathway is the signaling system which is significantly involved in diseases caused by excessive cell proliferation, such as cancer. In order to inhibit gene overexpression caused by the hippo signaling pathway, TEAD proteins located at the lowest level of the signaling system have been suggested as a sole therapeutic target. Accordingly, there is a recent demand for the development of low molecular weight compounds which inhibit the palmitoylation of TEAD proteins and thus inhibit gene overexpression caused by the hippo signaling pathway.

### [Related Art Reference]

### [Non-Patent Document]

(Non-Patent Document 0001) Cell 2011 Nov 11; 147(4):759-72
(Non-Patent Document 0002) Folia Histochem Cytobiol. 2015; 53(2):105-119
(Non-Patent Document 0003) Transl Lung Cancer Res. 2014; 3: 75-83
(Non-Patent Document 0004) Cancer Res. 1995; 55: 1227-31
(Non-Patent Document 0005) Cell Rep. 2020 Jun 23; 31(12):107809
(Non-Patent Document 0006) Chan, P. et al. Nat. Chem. Biol. (2016) 12: 282-289
(Non-Patent Document 0007) Noland, C. L. et al. Structure (2016) 24: 179-186
(Non-Patent Document 0008) Mesrouze, Y. et al. Protein Sci. (2017) 26: 2399-2409

### Detailed Description of the Invention

### Technical Problem

The present invention may provide a compound represented by formula I, a stereoisomer thereof, a pharmaceutically acceptable salt thereof, a hydrate thereof or a solvate thereof.

The present invention may provide a pharmaceutical composition including a compound represented by formula I, a stereoisomer thereof, a pharmaceutically acceptable salt thereof, a hydrate thereof or a solvate thereof as an active ingredient.

The present invention may provide a pharmaceutical composition including a compound represented by formula I, a stereoisomer thereof, a pharmaceutically acceptable salt thereof, a hydrate thereof or a solvate thereof for preventing or treating cancer.

The present invention may provide a pharmaceutical composition including a compound represented by formula I, a stereoisomer thereof, a pharmaceutically acceptable salt thereof, a hydrate thereof or a solvate thereof for preventing or treating cancer, which is caused by hyperproliferation of cells, by binding to a TEAD protein which is located at a lowest level of a signaling system of hippo signaling, which is one of the most important signaling systems for cell generation and growth, and functions as a switch for controlling the expression of genes involved in cell generation and growth, so as to inhibit the function of protein and inhibit the expression of genes.

The present invention may provide a use of a compound represented by formula I, a stereoisomer thereof, a pharmaceutically acceptable salt thereof, a hydrate thereof or a solvate thereof, or a pharmaceutical composition including the same for preventing or treating cancer.

The present invention may provide a use of a compound represented by formula I, a stereoisomer thereof, a pharmaceutically acceptable salt thereof, a hydrate thereof or a solvate thereof, or a pharmaceutical composition including the same in preparation of a medicament for preventing or treating cancer.

### Technical Solution

The present invention may provide a novel TEAD inhibitory substance for inhibiting the palmitoylation of TEAD protein, in which the novel TEAD inhibitory substance is structurally completely different from the palmitoylation inhibitory substances of the TEAD protein reported so far and has excellent intracellular permeability and in vivo stability. In addition, the present invention may contribute to the development of a therapeutic agent for diseases involving the hippo signaling pathway, such as anticancer drugs, etc., using the novel substance.

Hereinafter, the present invention will be described in more detail. All the combinations of various elements disclosed in the present invention fall within the scope of the present invention. In addition, it cannot be seen that the scope of the present invention is limited to the specific description below.

### Compound represented by formula I, stereoisomer thereof, pharmaceutically acceptable salt thereof, hydrate thereof or solvate thereof

The present invention may provide a compound according to any one of (1) to (6) below, a stereoisomer thereof, a pharmaceutically acceptable salt thereof, a hydrate thereof or a solvate thereof.
**(1)** A compound represented by formula I below, a stereoisomer thereof, a pharmaceutically acceptable salt thereof, a hydrate thereof or a solvate thereof: 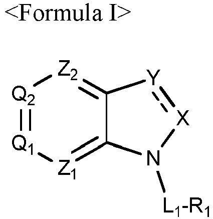 wherein in above formula I,
   L₁ is a single bond, -(C1-C6 alkylene)- or -(C=O)-;
   R₁ is H, C1-C3 alkyl, C1-C6 alkoxy, C3-C8 cycloalkyl, C3-C8 cycloalkenyl, 6- to 14-membered aryl, or 5- to 12-membered heteroaryl comprising in a ring 1 to 3 heteroatoms independently selected from the group consisting of N, O and S;
   in said R₁, at least one H of C1-C3 alkyl, C1-C6 alkoxy, C3-C8 cycloalkyl, C3-C8 cycloalkenyl, 6- to 14-membered aryl, or 5- to 12-membered heteroaryl comprising in a ring 1 to 3 heteroatoms independently selected from the group consisting of N, O and S may be each independently substituted or unsubstituted with C1-C6 alkyl, C1-C6 alkoxy, C3-C8 cycloalkyl, CF₂H, CF₃, -OCF₃, -OH, -(C1-C3 alkylene)-OH, -(C=O)Rp, -COORq, -CN, -(C1-C3 alkylene)-CN, -NRxRy, -(C1-C3 alkylene)-NRsRt or halogen, in which Rp, Rq, Rs, Rt, Rx and Ry are each independently H or C1-C6 alkyl;
   Z₁ and Z₂ are each independently N or CRa;
   Ra is H or C1-C6 alkoxy;
   Q₁ and Q₂ are each independently N, CH or CRb, in which at least one of Q₁ and Q₂ is CRb; 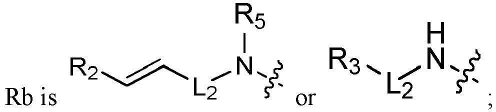
   L₂ is -(C=O)- or S(=O)₂;
   R₂, R₃ and R₅ are each independently H or C1-C6 alkyl;
   X-̅ -̅ -̅Y is X-Y or X=Y;
   when X-̅ -̅ -̅Y is X-Y, X and Y are each -CH₂-;
   when X-̅ -̅ -̅Y is X=Y, X is -CH- or N, and Y is -CR₄ or N;
   R₄ is H, C1-C6 alkyl or halogen;
   Z₁, Z₂, Q₁ and Q₂ are not all N, and when X-̅ -̅ -̅Y is X=Y, at least one of X and Y is N.
(2) In above (1), it may be provided that in above formula I,
   L₁ is a single bond, -(C1-C6 alkylene)- or -(C=O)-;
   R₁ is H, C1-C3 alkyl, C1-C6 alkoxy, C3-C8 cycloalkyl, C3-C8 cycloalkenyl, 6- to 14-membered aryl, or 5- to 12-membered heteroaryl comprising in a ring 1 to 3 heteroatoms independently selected from the group consisting of N, O and S;
   in said R₁, at least one H of C1-C6 alkoxy, C3-C8 cycloalkyl, C3-C8 cycloalkenyl, 6-to 14-membered aryl, or 5- to 12-membered heteroaryl comprising in a ring 1 to 3 heteroatoms independently selected from the group consisting of N, O and S may be each independently substituted or unsubstituted with C1-C6 alkyl, C1-C6 alkoxy, C3-C8 cycloalkyl, CF₂H, CF₃, - OCF₃, -OH, -CN, -NRxRy or halogen, in which Rx and Ry are each independently H or C1-C6 alkyl;
   Z₁ is Nor CH;
   Z₂ is N or CRa;
   Ra is H or C1-C6 alkoxy;
   Q₁ and Q₂ are each independently N, CH or CRb, in which at least one of Q₁ and Q₂ is CRb;
   Rb is 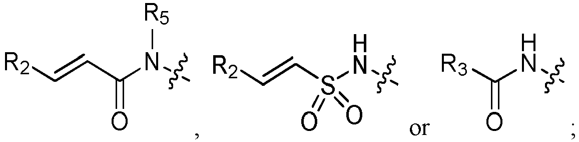
   R₂ and R₅ are each independently H or C1-C6 alkyl;
   R₃ is C1-C6 alkyl;
   X-̅ -̅ -̅Y is X-Y or X=Y;
   when X-̅ -̅ -̅Y is X-Y, X and Y are -CH₂-;
   when X-̅ -̅ -̅Y is X=Y, X is -CH- or N, and Y is -CR₄ or N;
   R₄ is H, C1-C6 alkyl or halogen; and
   Z₁, Z₂, Q₁ and Q₂ are not all N, and when X-̅ -̅ -̅Y is X=Y, either X or Y is N.
(3) In above (1) or (2), it may be provided that in above formula I,
   L₁ is a single bond, -(C1-C6 alkylene)- or -(C=O)-;
   R₁ is H, methoxy, cyclopentanyl, cyclohexanyl, cyclohexenyl, phenyl, pyridinyl, pyrazolyl, pyrimidinyl or thiophenyl;
   in above R₁, at least one H of methoxy, cyclohexanyl, cyclohexenyl, phenyl, pyridinyl, pyrazolyl, pyrimidinyl or thiophenyl may be each independently substituted or unsubstituted with methyl, isopropyl, tert-butyl, sec-butyl, methoxy, isobutoxy, cyclohexyl, CF₂H, CF₃, -OCF₃, -OH, -CN, NH₂ or halogen;
   Z₁ is Nor CH;
   Z₂ is N or CRa;
   Ra is H or methoxy;
   Q₁ is N, CH or CRb₁ and Q₂ is N, CH or CRb₂, in which at least one of Q₁ and Q₂ is CRb₁ or CRb₂;
   Rb₁ is 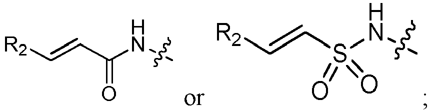
   Rb₂ is 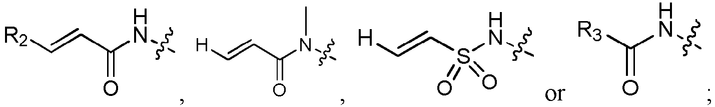
   R₂ is H or methyl;
   R₃ is isopropyl;
   X-̅ -̅ -̅Y is X-Y or X=Y;
   when X-̅ -̅ -̅Y is X-Y, X and Y are -CH₂-;
   when X-̅ -̅ -̅Y is X=Y, X is -CH- or N, and Y is -CR₄ or N;
   R₄ is H, methyl or halogen; and
   Z₁, Z₂, Q₁ and Q₂ are not all N, and when X-̅ -̅ -̅Y is X=Y, either X or Y is N.
   Specifically, it may be provided as above that Q₁ is N, CH or CRb₁, and Q₂ is N, CH or CRb₂, in which when Q₁ is N or CH, Q₂ is CRb₂; and when Q₂ is N or CH, Q₁ is CRb₁. Said Rb₁ and Rb₂ may be the same as described above, respectively.
**(4)** In any one of above **(1)** to **(3),** it may be provided that the compound represented by above formula I is a compound represented by formula II below: 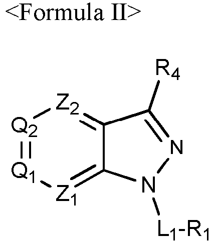 wherein in above formula II,
   L₁ is a single bond or -(C1-C6 alkylene)-;
   R₁ is H, C1-C6 alkoxy, C3-C8 cycloalkyl, C3-C8 cycloalkenyl, 6- to 14-membered aryl, or 5- to 12-membered heteroaryl comprising in a ring 1 to 3 heteroatoms independently selected from the group consisting of N, O and S;
   in said R₁, at least one H of C1-C6 alkoxy, C3-C8 cycloalkyl, C3-C8 cycloalkenyl, 6-to 14-membered aryl, or 5- to 12-membered heteroaryl comprising in a ring 1 to 3 heteroatoms independently selected from the group consisting of N, O and S may be each independently substituted or unsubstituted with C1-C6 alkyl, C1-C6 alkoxy, CF₂H, CF₃, -OCF₃, -OH, -CN, - NRxRy or halogen, in which Rx and Ry are each independently H or C1-C6 alkyl;
   Z₁ and Z₂ are each independently N or CH;
   Q₁ and Q₂ are each independently N, CH or CRb, in which at least one of Q₁ and Q₂ is CRb;
   Rb is 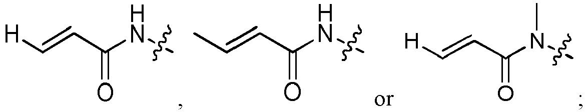 and
   R₄ is H, C1-C6 alkyl or halogen.
**(5)** In any one of above **(1)** to **(4),** it may be provided that the compound represented by above formula I is a compound represented by formula III below: 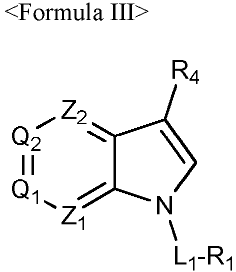 wherein in above formula III,
   L₁ is a single bond, -(C1-C6 alkylene)- or -(C=O)-;
   R₁ is H, C3-C8 cycloalkyl, 6- to 14-membered aryl, or 5- to 12-membered heteroaryl comprising in a ring 1 to 3 heteroatoms independently selected from the group consisting of N, O and S;
   in said R₁, at least one H of C3-C8 cycloalkyl, 6- to 14-membered aryl, or 5- to 12-membered heteroaryl comprising in a ring 1 to 3 heteroatoms independently selected from the group consisting of N, O and S may be each independently substituted or unsubstituted with C1-C6 alkyl, C1-C6 alkoxy, C3-C8 cycloalkyl, CF₂H, CF₃, -OCF₃, or halogen;
   Z₁ is Nor CH;
   Z₂ is N or CRa;
   Ra is H or C1-C6 alkoxy;
   Q₁ is N or CH;
   Q₂ is CRb;
   Rb is 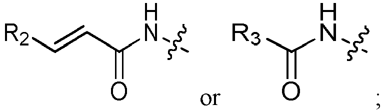
   R₂ is H or C1-C6 alkyl;
   R₃ is C1-C6 alkyl;
   R₄ is H, C1-C6 alkyl or halogen; and
   at least one of Z₁, Z₂, Q₁ and Q₂ is N.

In the present invention, the term "Cm-Cn" (in which m and n are each independently an integer of 1 or more) may mean the number of carbons, for example, "C1-C5 alkyl" may represent an alkyl having one to five carbon atoms.

In the present invention, "substitution" or "substituted with∼" may be defined to include implicit conditions, in which the substitution follows a permitted valency of a substituted atom and a substituent and induces a compound stabilized by substitution, for example, a compound which is not naturally modified by rearrangement, cyclization, removal, etc.

In the present invention, a "single bond" may mean a case in which adjacent atoms or groups of atoms are directly bonded to each other. As one example, in the compound represented by formula I of the present invention, when L₁ is a single bond, N and R₁ may be directly bonded, in which the formula may be represented by formula Ia below.

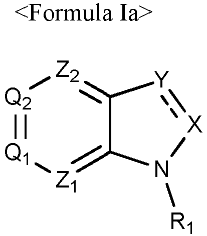

In the present invention, "-̅ -̅ -̅" indicated between two atoms may mean a single bond or a double bond between the two atoms. When -̅ -̅ -̅ is a single bond, the dotted line may be null, and when - - - is a double bond, the dotted line may represent a solid line.

In the present invention, "alkyl" may mean a linear (or straight-chain) saturated hydrocarbon group or a branched (or side-chain) saturated hydrocarbon group, unless otherwise stated. An example of alkyl may include at least one selected from methyl, ethyl, n-propyl, isopropyl, n-butyl, sec-butyl, isobutyl, tert-butyl, n-pentyl, n-hexyl, n-heptyl, and the like, but is not limited thereto.

In the present invention, "alkylene" may mean a divalent functional group which is induced from alkyl as defined above, unless otherwise stated.

In the present invention, "alkenyl" may mean an unsaturated hydrocarbon group including at least one carbon-carbon double bond in the alkyl, unless otherwise stated.

In the present invention, "alkoxy" may mean a monovalent group derived from a straight-chain or branched saturated hydrocarbon moiety represented by OCₙH₂ₙ₊₁, unless otherwise stated. An example of alkoxy may include at least one selected from methoxy, ethoxy, propoxy, isopropoxy, n-butoxy, sec-butoxy, tert-butoxy, pentoxy, hexoxy, and the like, but is not limited thereto.

In the present invention, "cycloalkyl" may mean a saturated hydrocarbon ring having three or more carbon atoms, and the saturated hydrocarbon ring may include both monocyclic and polycyclic structures, unless otherwise stated. An example of cycloalkyl may include at least one selected from cyclopropyl, cyclobutyl, cyclopentyl, cyclohexyl, cycloheptyl, cyclooctyl, and the like, but is not limited thereto.

In the present invention, "cycloalkenyl" may mean a cyclic group including at least one carbon-carbon double bond in the cycloalkyl, unless otherwise stated. An example of cycloalkenyl may include at least one selected from cyclopropenyl, cyclobutenyl, cyclopentenyl, cyclohexenyl, cycloheptenyl, cyclooctenyl, and the like, but is not limited thereto.

In the present invention, "heterocycloalkyl" may mean a cyclic group in which at least one carbon atom constituting a ring in the cycloalkyl is each independently substituted with a heteroatom or functional group selected from the group consisting of N, O, S, SO, and SO₂, unless otherwise stated. An example of heterocycloalkyl may include at least one selected from oxiranyl, oxetanyl, tetrahydrofuranyl, tetrahydropyranyl, tetrahydrothiopyranyl, tetrahydrothiophenyl, oxepanyl, piperidinyl, piperazinyl, morpholinyl, thiomorpholinyl, pyrrolidinyl, azetidinyl, aziridinyl, azepanyl and the like, but is not limited thereto.

In the present invention, "aryl" may mean a monocyclic or polycyclic cyclic group having at least one fused or non-fused aromatic ring, unless otherwise stated. An example of aryl may include at least one selected from phenyl, biphenyl, naphthalenyl, tetrahydronaphthyl, indenyl, anthracenyl, and the like, but is not limited thereto.

In the present invention, "heteroaryl" may mean a monocyclic or polycyclic heterocycle comprising in a ring at least one heteroatom selected from the group consisting of N, O and S and having at least one fused or non-fused aromatic ring, unless otherwise stated. An example of heteroaryl may include at least one selected from thiazolyl, oxazolyl, thiophenyl, furanyl, pyrrolyl, imidazolyl, isoxazolyl, pyrazolyl, triazolyl, thiadiazolyl, tetrazolyl, oxadiazolyl, pyridinyl, pyridazinyl, pyrimidinyl, pyrazinyl, indolyl, benzothiophenyl, benzofuranyl, benzothiazolyl, benzimidazolyl, benzoxazolyl, benzisoxazolyl, benzthiazolyl, benzthiadiazolyl, benztriazolyl, quinolinyl, isoquinolinyl, purinyl, furopyridinyl, and the like, but is not limited thereto.

In the present invention, "halogen" may be F, Cl, Br or I, unless otherwise stated.

**(6)** In any one of above **(1)** to **(5),** the compound represented by above formula I is at least one compound selected from the group consisting of compounds shown in table 1 below:

**[Table 1]**

| **Compound No.** | **Structure** | **Compound Name** |
|---|---|---|
| **1** | 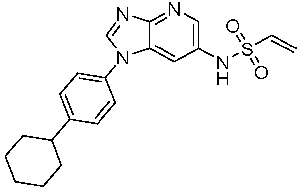 | N-(1-(4-cycloheaylphenyl)-1H-imidazo[4,5-b]pyridin-6-yl)ethenesulfonamide |
| **2** | 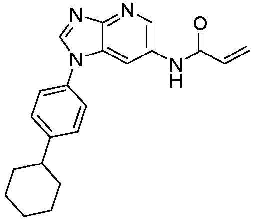 | N-(1-(4-cycloheaylphenyl)-1H-imidazo[4,5-b]pyridin-6-yl)acrylamide |
| **3** | 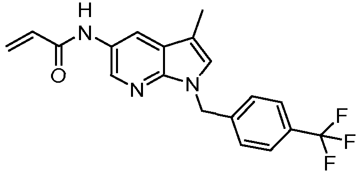 | N-(3-methyl-1-(4-(trifluoromethyl)benzyl)-1H-pyrrolo[2,3-b]pyridin-5-yl)acrylamide |
| **4** | 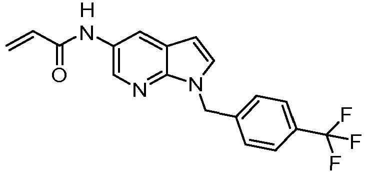 | N-(1-(4-(trifluoromethyl)benzyl)-1H-pyrrolo[2,3-b]pyridin-5-yl)acrylamide |
| **5** | 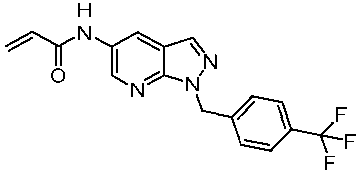 | N-(1-(4-(trifluoromethyl)benzyl)-1H-pyrazolo[3,4-b]pyridin-5-yl)acrylamide |
| **6** | 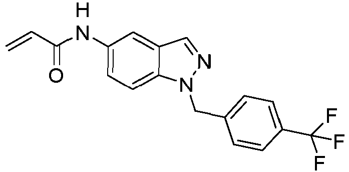 | N-(1-(4-(trifluoromethyl)benzyl)-1H-indazol-5-yl)acrylamide |
| **7** | 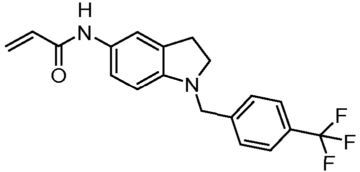 | N-(1-(4-(trifluoromethyl)benzyl)indolin-5-yl)acrylamide |
| **8** | 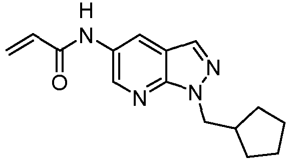 | N-(1-(cyclopentylmethyl)-1H-pyrazolo[3,4-b]pyridin-5-yl)acrylamide |
| **9** | 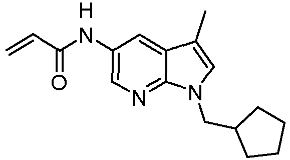 | N-(1-(cyclopentylmethyl)-3-methyl-1H-pyrrolo[2,3-b]pyridin-5-yl)acrylamide |
| **10** | 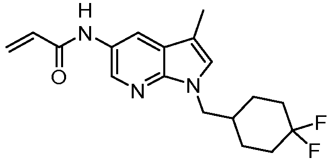 | N-(1-((4,4-difluorocyclohexyl)methyl)-3-methyl-1H-pyrrolo[2,3-b]pyridin-5-yl)acrylamide |
| **11** | 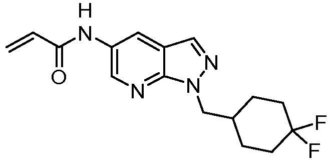 | N-(1-((4,4-difluorocyclohexyl)methyl)-1H-pyrazolo[3,4-b]pyridin-5-yl)acrylamide |
| **12** | 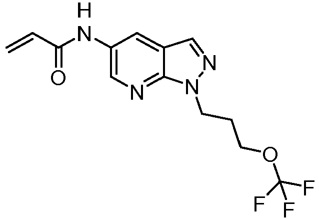 | N-(1-(3-(trifluoromethoxy)propyl)-1H-pyrazolo[3,4-b]pyridin-5-yl)acrylamide |
| **13** | 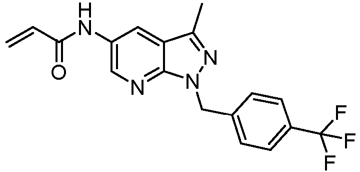 | N-(3-methyl-1-(4-(trifluoromethyl)benzyl)-1H-pyrazolo[3,4-b]pyridin-5-yl)acrylamide |
| **14** | 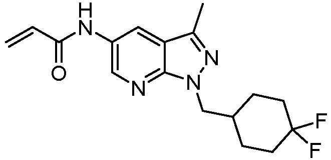 | N-(1-((4,4-difluorocyclohexyl)methyl)-3-methyl-1H-pyrazolo[3,4-b]pyridin-5-yl)acrylamide |
| **15** | 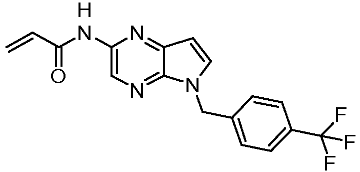 | N-(5-(4-(trifluoromethyl)benzyl)-5H-pyrrolo[2,3-b]pyrazin-2-yl)acrylamide |
| **16** | 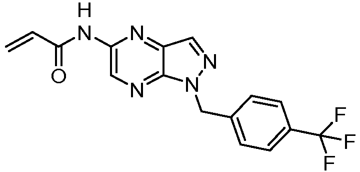 | N-(1-(4-(trifluoromethyl)benzyl)-1H-pyrazolo [3,4-b]pyrazin-5-yl)acrylamide |
| **17** | 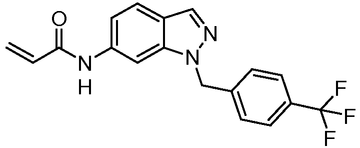 | N-(1-(4-(trifluoromethyl)benzyl)-1H-indazol-6-yl)acrylamide |
| **18** | 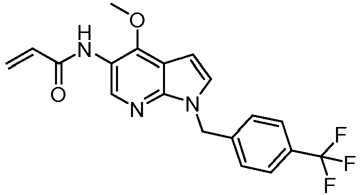 | N-(4-methoxy-1-(4-(trifluoromethyl)benzyl)-1H-pyrrolo[2,3-b]pyridin-5-yl)acrylamide |
| **19** | 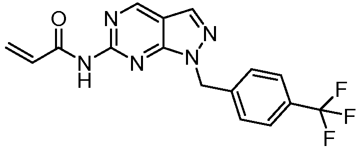 | N-(1-(4-(trifluoromethyl)benzyl)-1H-pyrazolo[3,4-d]pyrimidin-6-yl)acrylamide |
| **20** | 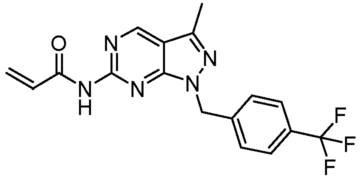 | N-(3-methyl-1-(4-(trifluoromethyl)benzyl)-1H-pyrazolo[3,4-d]pyrimidin-6-yl)acrylamide |
| **21** | 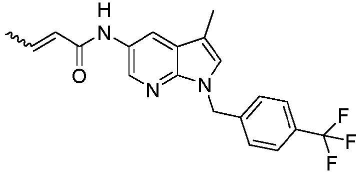 | N-(3-methyl-1-(4-(trifluoromethyl)benzyl)-1H-pyrrolo[2,3-b]pyridin-5-yl)but-2-enamide |
| **22** | 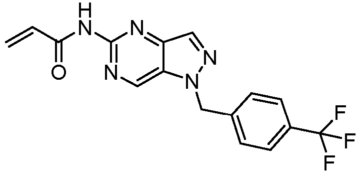 | N-(1-(4-(trifluoromethyl)benzyl)-1H-pyrazolo[4,3-d]pyrimidin-5-yl)acrylamide |
| **23** | 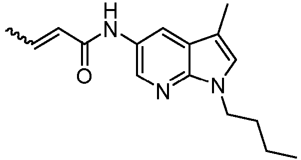 | N-(1-butyl-3-methyl-1H-pyrrolo[2,3-b]pyridin-5-yl)but-2-enamide |
| **24** | 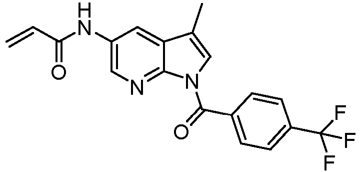 | N-(3-methyl-1-(4-(trifluoromethyl)benzoyl)-1H-pyrrolo [2,3-b]pyridin-5-yl)acrylamide |
| **25** | 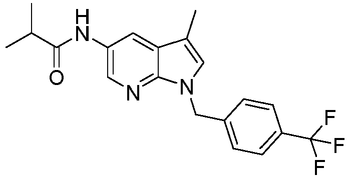 | N-(3-methyl-1-(4-(trifluoromethyl)benzyl)-1H-pyrrolo[2,3-b]pyridin-5-yl)isobutyramide |
| **26** | 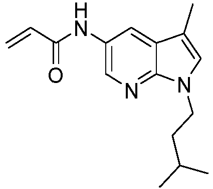 | N-(1-isopentyl-3-methyl-1H-pyrrolo[2,3-b]pyridin-5-yl)acrylamide |
| **27** | 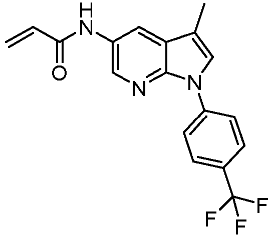 | N-(3-methyl-1-(4-(trifluoromethyl)phenyl)-1H-pyrrolo[2,3-b]pyridin-5-yl)acrylamide |
| **28** | 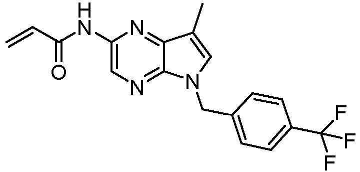 | N-(7-methyl-5-(4-(trifluoromethyl)benzyl)-5H-pyrrolo[2,3-b]pyrazin-2-yl)acrylamide |
| **29** | 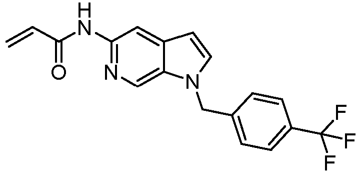 | N-(1-(4-(trifluoromethyl)benzyl)-1H-pyrrolo[2,3-c]pyridin-5-yl)acrylamide |
| **30** | 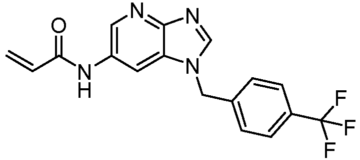 | N-(1-(4-(trifluoromethyl)benzyl)-1H-imidazo[4,5-b]pyridin-6-yl)acrylamide |
| **31** | 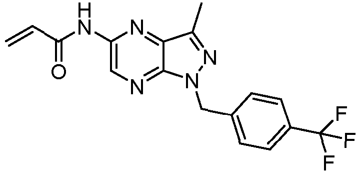 | N-(3-methyl-1-(4-(trifluoromethyl)benzyl)-1H-pyrazolo [3,4-b]pyrazin-5-yl)acrylamide |
| **32** | 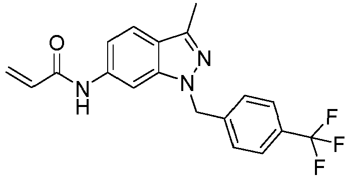 | N-(3-methyl-1-(4-(trifluoromethyl)benzyl)-1H-indazol-6-yl)acrylamide |
| **33** | 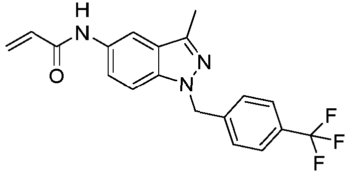 | N-(3-methyl-1-(4-(trifluoromethyl)benzyl)-1H-indazol-5-yl)acrylamide |
| **34** | 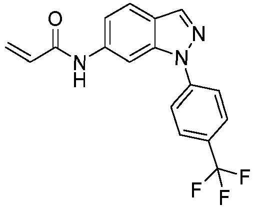 | N-(1-(4-(trifluoromethyl)phenyl)-1H-indazol-6-yl)acrylamide |
| **35** | 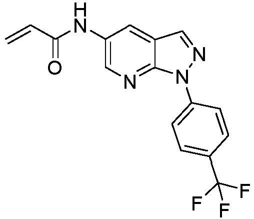 | N-(1-(4-(trifluoromethyl)phenyl)-1H-pyrazolo[3,4-b]pyridin-5-yl)acrylamide |
| **36** | 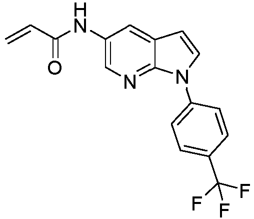 | N-(1-(4-(trifluoromethyl)phenyl)-1H-pyrrolo[2,3-b]pyridin-5-yl)acrylamide |
| **37** | 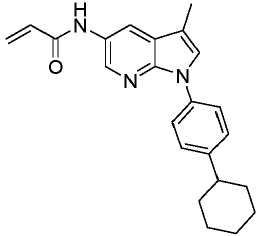 | N-(1-(4-cycloheaylphenyl)-3-methyl-1H-pyrrolo [2,3-b]pyridin-5-yl)acrylamide |
| **38** | 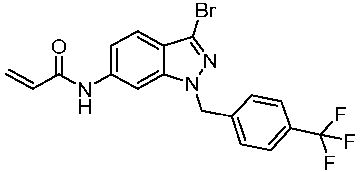 | N-(3-bromo-1-(4-(trifluoromethyl)benzyl)-1H-indazol-6-yl)acrylamide |
| **39** | 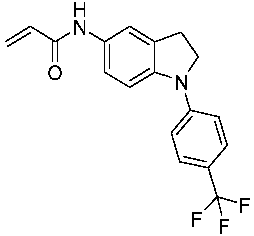 | N-(1-(4-(trifluoromethyl)phenyl)indofin-5-yl)acrylamide |
| **40** | 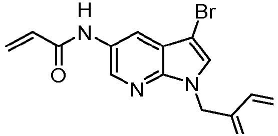 | N-(3-bromo-1-(4-(trifluoromethyl)benzyl)-1H-pyrrolo[2,3-b]pyridin-5-yl)acrylamide |
| **41** | 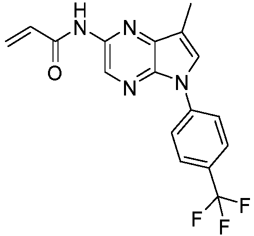 | N-(7-methyl-5-(4-(trifluoromethyl)phenyl)-5H-pyrrolo [2,3-b]pyrazin-2-yl)acrylamide |
| **42** | 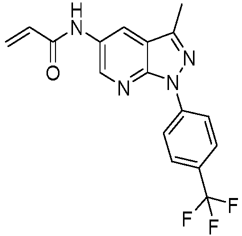 | N-(3-methyl-1-(4-(trifluoromethyl)phenyl)-1H-pyrazolo[3,4-b]pyridin-5-yl)acrylamide |
| **43** | 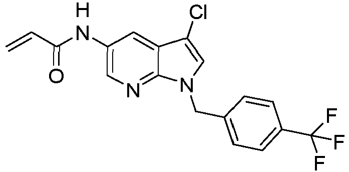 | N-(3-chloro-1-(4-(trifluoromethyl)benzyl)-1H-pyrrolo[2,3-b]pyridin-5-yl)acrylamide |
| **44** | 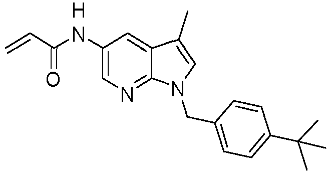 | N-(1-(4-(tert-butyl)benzyl)-3-methyl-1H-pyrrolo[2,3-b]pyridin-5-yl)acrylamide |
| **45** | 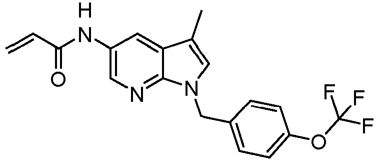 | N-(3-methyl-1-(4-(trifluoromethoxy)benzyl)-1H-pyrrolo[2,3-b]pyridin-5yl)acrylamide |
| **46** | 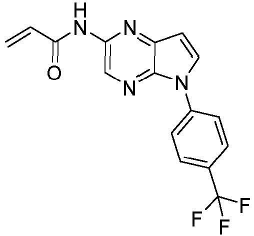 | N-(5-(4-(trifluoromethyl)phenyl)-5H-pyrrolo[2,3-b]pyrazin-2-yl)acrylamide |
| **47** | 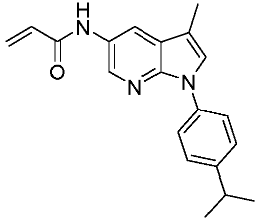 | N-(1-(4-isopropylphenyl)-3-methyl-1H-pyrrolo[2,3-b]pyridin-5-yl)acrylamide |
| **48** | 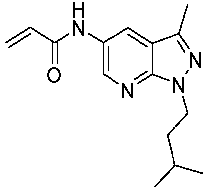 | N-(1-isopentyl-3-methyl-1H-pyrazolo[3,4-b]pyridin-5yl)acrylamide |
| **49** | 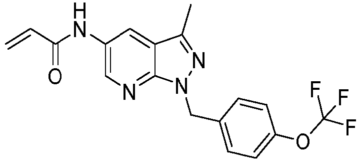 | N-(3-methyl-1-(4-(trifluoromethoxy)benzyl)-1H-pyrazolo [3,4-b]pyridin-5-yl)acrylamide |
| **50** | 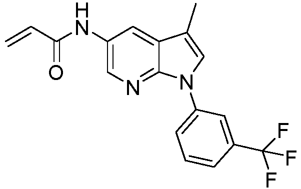 | N-(3-methyl-1-(3-(trifluoromethyl)phenyl)-1H-pyrrolo[2,3-b]pyridin-5-yl)acrylamide |
| **51** | 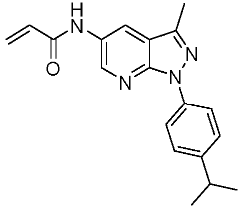 | N-(1-(4-isopropylphenyl)-3-methyl-1H-pyrazolo[3,4-b]pyridin-5-yl)acrylamide |
| **52** | 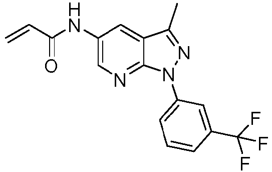 | N-(3-methyl-1-(3-(trifluoromethyl)phenyl)-1H-pyrazolo[3,4-b]pyridin-5-yl)acrylamide |
| **53** | 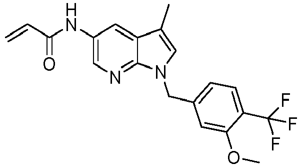 | N-(1-(3-methoxy-4-(trifluoromethyl)benzyl)-3-methyl-1H-pyrrolo[2,3-b]pyridin-5-yl)acrylamide |
| **54** | 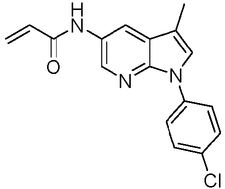 | N-(1-(4-chlorophenyl)-3-methyl-1H-pyrrolo[2,3-b]pyridin-5-yl)acrylamide |
| **55** | 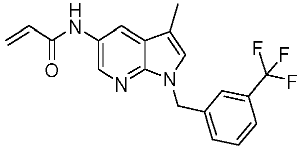 | N-(3-methyl-1-(3-(trifluoromethyl)benzyl)-1H-pyrrolo[2,3-b]pyridin-5-yl)acrylamide |
| **56** | 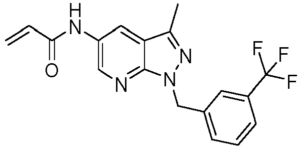 | N-(3-methyl-1-(3-(trifluoromethyl)benzyl)-1H-pyrazolo[3,4-b]pyridin-5-yl)acrylamide |
| **57** | 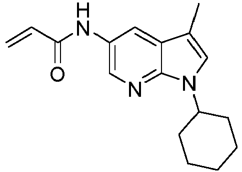 | N-(1-cyclohexyl-3-methyl-1H-pyrrolo[2,3-b]pyridin-5-yl)acrylamide |
| **58** | 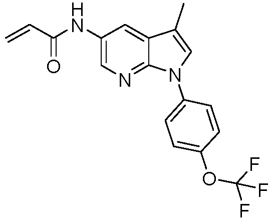 | N-(3-methyl-1-(4-(trifluoromethoxy)phenyl)-1H-pyrrolo[2,3-b]pyridin-5-yl)acrylamide |
| **59** | 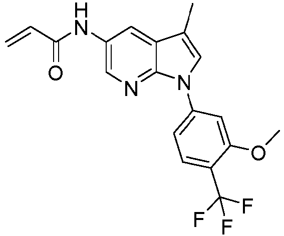 | N-(1-(3-methoxy-4-(trifluoromethyl)phenyl)-3-methyl-1H-pyrrolo[2,3-b]pyridin-5-yl)acrylamide |
| **60** | 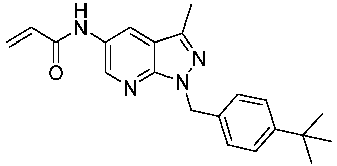 | N-(1-(4-(tert-butyl)benzyl)-3-methyl-1H-pyrazolo[3,4-b]pyridin-5-yl)acrylamide |
| **61** | 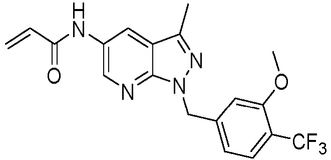 | N-(1-(3-methoxy-4-(trifluoromethyl)benzyl)-3-methyl-1H-pyrazolo[3,4-b]pyridin-5-yl)acrylamide |
| **62** | 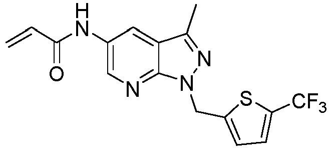 | N-(3-methyl-1-((5-(trifluoromethyl)thiophen-2-yl)methyl)-1H-pyrazolo[3,4-b]pyridin-5-yl)acrylamide |
| **63** | 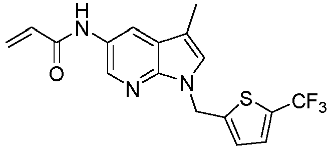 | N-(3-methyl-1-((5-(trifluoromethyl)thiophen-2-yl)methyl)-1H-pyrrolo[2,3-b]pyridin-5-yl)acrylamide |
| **64** | 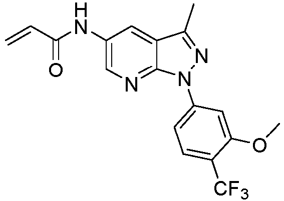 | N-(1-(3-methoxy-4-(trifluoromethyl)phenyl)-3-methyl-1H-pyrazolo[3,4-b]pyridin-5-yl)acrylamide |
| **65** | 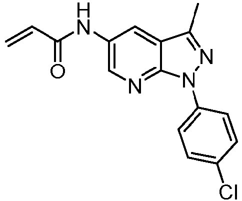 | N-(1-(4-chlorophenyl)-3-methyl-1H-pyrazolo[3,4-b]pyridin-5-yl)acrylamide |
| **66** | 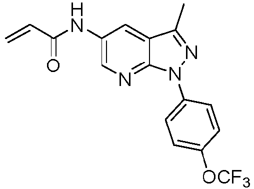 | N-(3-methyl-1-(4-(trifluoromethoxy)phenyl)-1H-pyrazolo[3,4-b]pyridin-5-yl)acrylamide |
| **67** | 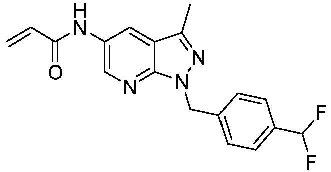 | N-(1-(4-(difluoromethyl)benzyl)-3-methyl-1H-pyrazolo[3,4-b]pyridin-5-yl)acrylamide |
| **68** | 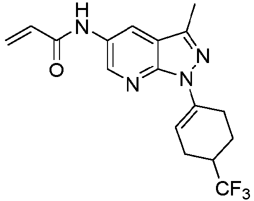 | N-(3-methyl-1-(4-(trifluoromethyl)cyclohex-1-en-1-yl)-1H-pyrazolo[3,4-b]pyridin-5-yl)acrylamide |
| **69** | 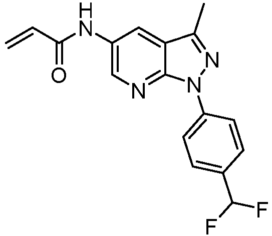 | N-(1-(4-(difluoromethyl)phenyl)-3-methyl-1H-pyrazolo[3,4-b]pyridin-5-yl)acrylamide |
| **70** | 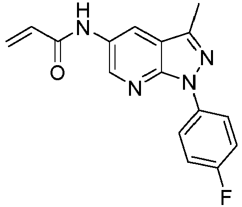 | N-(1-(4-fluorophenyl)-3-methyl-1H-pyrazolo[3,4-b]pyridin-5-yl)acrylamide |
| **71** | 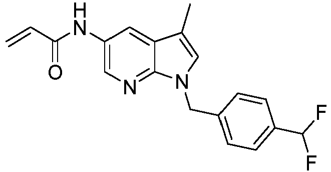 | N-(1-(4-(difluoromethyl)benzyl)-3-methyl-1H-pyrrolo[2,3-b]pyridin-5-yl)acrylamide |
| **72** | 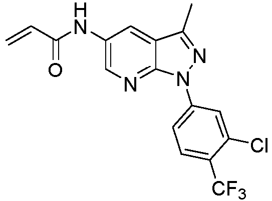 | N-(1-(3-chloro-4-(trifluoromethyl)phenyl)-3-methyl-1H-pyrazolo[3,4-b]pyridin-5-yl)acrylamide |
| **73** | 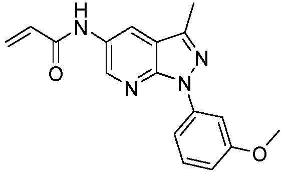 | N-(1-(3-methoxyphenyl)-3-methyl-1H-pyrazolo[3,4-b]pyridin-5-yl)acrylamide |
| **74** | 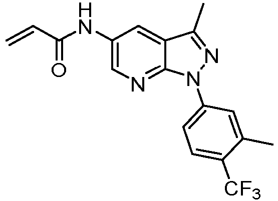 | N-(3-methyl-1-(3-methyl-4-(trifluoromethyl)phenyl)-1H-pyrazolo[3,4-b]pyridin-5-yl)acrylamide |
| **75** | 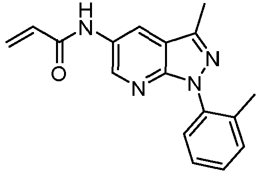 | N-(3-methyl-1-(o-tolyl)-1H-pyrazolo [3,4-b]pyridin-5-yl)acrylamide |
| **76** | 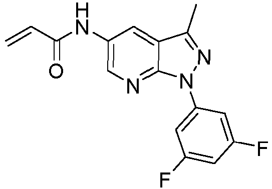 | N-(1-(3,5-difluorophenyl)-3-methyl-1H-pyrazolo[3,4-b]pyridin-5-yl)acrylamide |
| **77** | 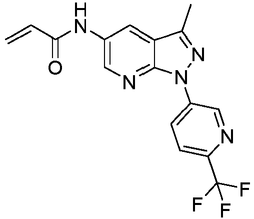 | N-(3-methyl-1-(6-(trifluoromethyl)pyridin-3-yl)-1H-pyrazolo[3,4-b]pyridin-5-yl)acrylamide |
| **78** | 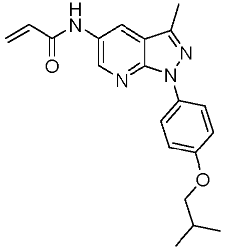 | N-(1-(4-isobutoxyphenyl)-3-methyl-1H-pyrazolo[3,4-b]pyridin-5-yl)acrylamide |
| **79** | 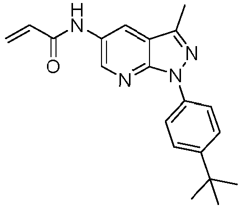 | N-(1-(4-(tert-butyl)phenyl)-3-methyl-1H-pyrazolo[3,4-b]pyridin-5-yl)acrylamide |
| **80** | 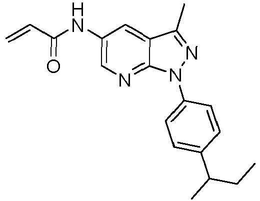 | N-(1-(4-(sec-butyl)phenyl)-3-methyl-1H-pyrazolo[3,4-b]pyridin-5-yl)acrylamide |
| **81** | 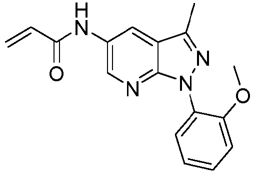 | N-(1-(2-methoxyphenyl)-3-methyl-1H-pyrazolo[3,4-b]pyridin-5-yl)acrylamide |
| **82** | 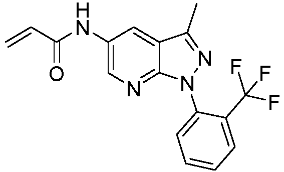 | N-(3-methyl-1-(2-(trifluoromethyl)phenyl)-1H-pyrazolo[3,4-b]pyridin-5-yl)acrylamide |
| **83** | 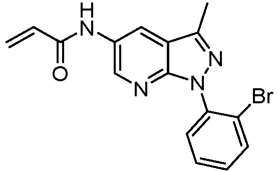 | N-(1-(2-bromophenyl)-3-methyl-1H-pyrazolo[3,4-b]pyridin-5-yl)acrylamide |
| **84** | 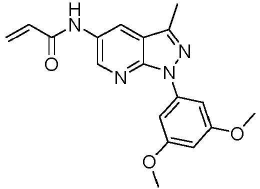 | N-(1-(3,5-dimethoxyphenyl)-3-methyl-1H-pyrazolo[3,4-b]pyridin-5-yl)acrylamide |
| **85** | 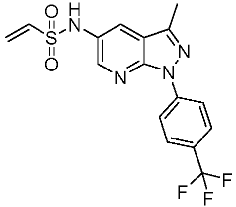 | N-(3-methyl-1-(4-(trifluoromethyl)phenyl)-1H-pyrazolo[3,4-b]pyridin-5-yl)ethenesulfonamide |
| **86** | 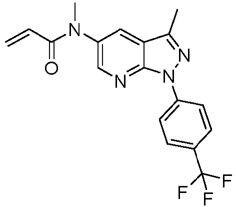 | N-methyl-N-(3-methyl-1-(4-(trifluoromethyl)phenyl)-1H-pyrazolo[3,4-b]pyridin-5-yl)acrylamide |
| **87** | 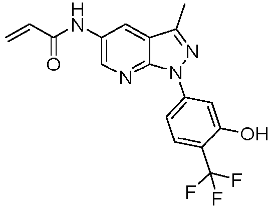 | N-(1-(3-hydroxy-4-(trifluoromethyl)phenyl)-3-methyl-1H-pyrazolo[3,4-b]pyridin-5-yl)acrylamide |
| **88** | 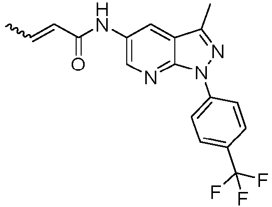 | N-(3-methyl-1-(4-(trifluoromethyl)phenyl)-1H-pyrazolo[3,4-b]pyridin-5-yl)but-2-enamide |
| **89** | 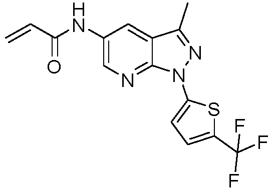 | N-(3-methyl-1-(5-(trifluoromethyl)thiophen-2-yl)-1H-pyrazolo[3,4-b]pyridin-5-yl)acrylamide |
| **90** | 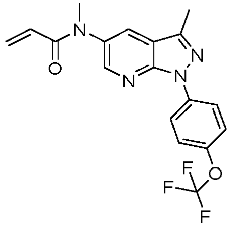 | N-methyl-N-(3-methyl-1-(4-(trifluoromethoxy)phenyl)-1H-pyrazolo[3,4-b]pyridin-5-yl)acrylamide |
| **91** | 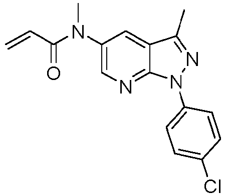 | N-(1-(4-chlorophenyl)-3-methyl-1H-pyrazolo[3,4-b]pyridin-5-yl)-N-methylacrylamide |
| **92** | 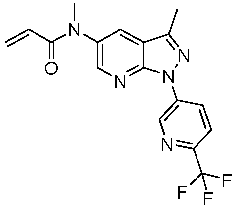 | N-methyl-N-(3-methyl-1-(6-(trifluoromethyl)pyridin-3-yl)-1H-pyrazolo[3,4-b]pyridin-5-yl)acrylamide |
| **93** | 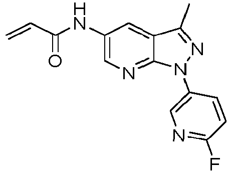 | N-(1-(6-fluoropyridin-3-yl)-3-methyl-1H-pyrazolo[3,4-b]pyridin-5-yl)acrylamide |
| **94** | 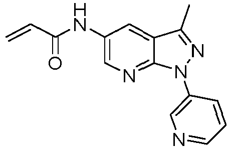 | N-(3-methyl-1-(pyridin-3-yl)-1H-pyrazolo[3,4-b]pyridin-5-yl)acrylamide |
| **95** | 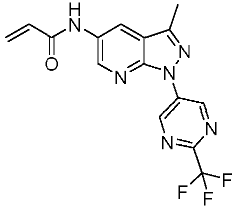 | N-(3-methyl-1-(2-(trifluoromethyl)pyrimidin-5-yl)-1H-pyrazolo[3,4-b]pyridin-5-yl)acrylamide |
| **96** | 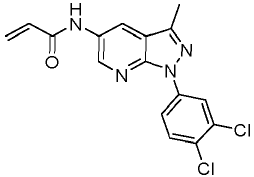 | N-(1-(3,4-dichlorophenyl)-3-methyl-1H-pyrazolo [3,4-b]pyridin-5-yl)acrylamide |
| **97** | 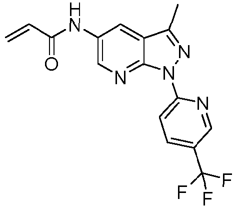 | N-(3-methyl-1-(5-(trifluoromethyl)pyridin-2-yl)-1H-pyrazolo[3,4-b]pyridin-5-yl)acrylamide |
| **98** | 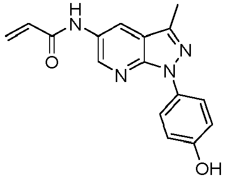 | N-(1-(4-hydroxyphenyl)-3-methyl-1H-pyrazolo[3,4-b]pyridin-5-yl)acrylamide |
| **99** | 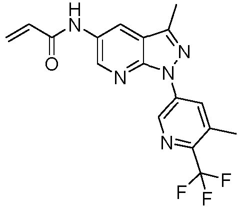 | N-(3-methyl-1-(5-methyl-6-(trifluoromethyl)pyridin-3-yl)-1H-pyrazolo[3,4-b]pyridin-5-yl)acrylamide |
| **100** | 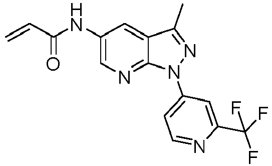 | N-(3-methyl-1-(2-(trifluoromethyl)pyridin-4-yl)-1H-pyrazolo[3,4-b]pyridin-5-yl)acrylamide |
| **101** | 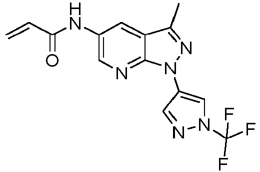 | N-(3-methyl-1-(1-(trifluoromethyl)-1H-pyrazol-4-yl)-1H-pyrazolo[3,4-b]pyridin-5-yl)acrylamide |
| **102** | 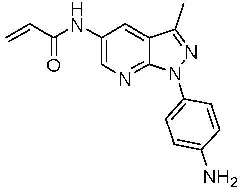 | N-(1-(4-aminophenyl)-3-methyl-1H-pyrazolo [3,4-b]pyridin-5-yl)acrylamide |
| **103** | 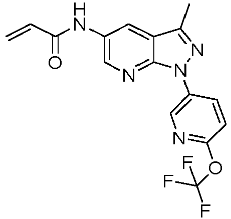 | N-(3-methyl-1-(6-(trifluoromethoxy)pyridin-3-yl)-1H-pyrazolo[3,4-b]pyridin-5-yl)acrylamide |
| **104** | 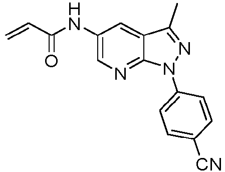 | N-(1-(4-cyanophenyl)-3-methyl-1H-pyrazolo[3,4-b]pyridin-5-yl)acrylamide |
| **105** | 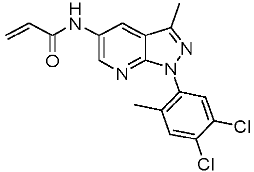 | N-(1-(4,5-dichloro-2-methylphenyl)-3-methyl-1H-pyrazolo[3,4-b]pyridin-5-yl)acrylamide |

In the present invention, "stereoisomer" may include a diastereomer and an optical isomer, in which the optical isomer may include not only an enantiomer, but also a mixture of the enantiomer and even a racemate.

In the present invention, "pharmaceutically acceptable" may refer to the one which is physiologically acceptable and does not conventionally cause an allergic response such as gastrointestinal disturbance and dizziness, or other responses similar thereto, when administered into an individual.

In the present invention, "pharmaceutically acceptable salts" may mean salts conventionally used in a pharmaceutical industry, for example, inorganic ion salts prepared from calcium, potassium, sodium, magnesium or the like; inorganic acid salts prepared from hydrochloric acid, nitric acid, phosphoric acid, bromic acid, iodic acid, perchloric acid, sulfuric acid or the like; organic acid salts prepared from acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbric acid, carbonic acid, vanillic acid or the like; sulfonic acid salts prepared from methanesulfonic acid, ethanesulfonic acid, benzenesulfonic acid, p-toluenesulfonic acid, naphthalenesulfonic acid or the like; amino acid salts prepared from glycine, arginine, lysine, or the like; amine salts prepared from trimethylamine, triethylamine, ammonia, pyridine, picoline, or the like; and the like, but types of salt meant in the present invention are not limited to those listed salts.

The pharmaceutically acceptable salt of the present invention may be prepared by a conventional method known to those skilled in the art.

The "hydrate" of the present invention may refer to the one in which the compound according to any one of above **(1)** to **(6)** or the pharmaceutically acceptable salt thereof is bound to water by a non-covalent intermolecular force, and may include a stoichiometric or non-stoichiometric amount of water. Specifically, the hydrate may include water at a ratio of about 0.25 mol to about 10 mol based on 1 mol of an active ingredient, more specifically about 0.5 mol, about 1 mol, about 1.5 mol, about 2 mol, about 2.5 mol, about 3 mol, about 5 mol, etc.

The "solvate" of the present invention may refer to the one in which the compound according to any one of above **(1)** to **(6),** the stereoisomer thereof or the pharmaceutically acceptable salt thereof is bound to a solvent other than water by a non-covalent intermolecular force, and may include a stoichiometric or non-stoichiometric amount of the solvent. Specifically, the solvate may include a solvent molecule at a ratio of about 0.25 mol to about 10 mol based on 1 mol of an active component, more specifically about 0.5 mol, about 1 mol, about 1.5 mol, about 2 mol, about 2.5 mol, about 3 mol, about 5 mol, etc.

The compound according to any one of above (1) to (6) of the present invention may be effectively used for preventing or treating cancer, which is caused by hyperproliferation of cells, by binding to a TEAD protein which is located at a lowest level of a signaling system of hippo signaling, which is one of the most important signaling systems for cell generation and growth, and functions as a switch for controlling the expression of genes involved in cell generation and growth, so as to inhibit the function of protein and inhibit the expression of genes.

### Pharmaceutical composition including compound represented by formula I, treatment method using same and use thereof

The present invention may provide a pharmaceutical composition including a compound according to any one of above (1) to (6), a stereoisomer thereof, a pharmaceutically acceptable salt thereof, a hydrate thereof or a solvate thereof as an active ingredient.

The present invention may provide a pharmaceutical composition including a compound according to any one of above (1) to (6), a stereoisomer thereof, a pharmaceutically acceptable salt thereof, a hydrate thereof or a solvate thereof as an active ingredient for preventing or treating cancer.

The cancer may be at least one selected from lung cancer, stomach cancer, ovarian cancer, prostate cancer, esophageal cancer, gastrointestinal cancer, pancreatic cancer, colorectal cancer, colon cancer, kidney cancer, testicular cancer, bladder cancer, breast cancer, uterine cancer, cervical cancer, head and neck cancer, blood cancer, bone cancer, liver cancer, thyroid cancer, parathyroid cancer, skin cancer, lymphoma, mesothelioma, leukemia, myeloma, sarcoma, virus-related cancer, and the like, but is not limited thereto. The virus-related cancer may be at least one selected from nasopharyngeal cancer, vaginal cancer, vulvar cancer, penile cancer, Kaposi's sarcoma, Burkitt's lymphoma, T-cell lymphoma, Merkel cell cancer, and the like, but is not limited thereto.

In the present invention, "prevention" may mean all the acts, which inhibit TEAD protein-related diseases, e.g., cancer or delay the occurrence thereof by administering a compound according to any one of above (1) to (6) of the present invention, a stereoisomer thereof, a pharmaceutically acceptable salt thereof, a hydrate thereof or a solvate thereof.

In the present invention, "treatment" may mean all the acts, by which a symptom of TEAD protein-related diseases, e.g., cancer gets better or takes a favorable turn by administering a compound according to any one of above (1) to (6) of the present invention, a stereoisomer thereof, a pharmaceutically acceptable salt thereof, a hydrate thereof or a solvate thereof.

The pharmaceutical composition of the present invention may further contain at least one pharmaceutically acceptable carrier, in addition to a compound according to any one of above (1) to (6), a stereoisomer thereof, a pharmaceutically acceptable salt thereof, a hydrate thereof or a solvate thereof.

In this case, the pharmaceutically acceptable carrier may be one which is conventionally used in the art for formulating a preparation, and may be at least one selected from lactose, dextrose, sucrose, sorbitol, mannitol, starch, acacia rubber, calcium phosphate, alginate, gelatin, calcium silicate, microcrystalline cellulose, polyvinyl pyrrolidone, cellulose, water, syrup, methyl cellulose, methyl hydroxybenzoate, propyl hydroxybenzoate, talc, magnesium stearate, mineral oil and the like, but is not limited thereto.

In addition, the pharmaceutical composition of the present invention may further include lubricant, humectant, a sweetening agent, a flavoring agent, emulsifier, a suspending agent, preservative, or the like, in addition to the above ingredients.

Furthermore, the pharmaceutical composition of the present invention may further include at least one medication selected from cell signal transduction inhibitors, mitosis inhibitors, alkylating agents, antimetabolites, antibiotics, growth factor inhibitors, cell cycle inhibitors, topoisomerase inhibitors, biological reaction modifiers, antihormonal agents, antiandrogen, cell differentiation/proliferation/survival inhibitors, apoptosis inducer, and the like, and may be used in combination with the medication further included above or may be formulated into a complex preparation when formulating the pharmaceutical composition of the present invention into a preparation.

The pharmaceutical composition of the present invention may be orally or parenterally administered (for example, applied intravenously, hypodermically, intraperitoneally or locally) according to an intended method, in which a dosage thereof varies depending on a patient's condition and weight, a degree of disease, a drug form, and an administration route and time, but may be appropriately selected by those skilled in the art.

In addition to a compound according to any one of above (1) to (6), a pharmaceutically acceptable salt thereof, a stereoisomer thereof, a hydrate thereof or a solvate thereof, the pharmaceutical composition of the present invention may further include at least one ingredient which may exhibit the same or similar medicinal effects or may bring synergy to medicinal effects in combination.

The present invention may provide a method for preventing or treating cancer, including administering a compound according to any one of above (1) to (6), a stereoisomer thereof, a pharmaceutically acceptable salt thereof, a hydrate thereof or a solvate thereof, or a pharmaceutical composition including the same into an individual.

The method for preventing or treating cancer according to the present invention may include administering a therapeutically effective amount of a compound according to any one of above **(1)** to **(6)**, a stereoisomer thereof, a pharmaceutically acceptable salt thereof, a hydrate thereof or a solvate thereof, or a pharmaceutical composition including the same.

In the present invention, "therapeutically effective amount" may mean an amount enough to treat diseases at a reasonable benefit/risk ratio applicable to medical treatment and not to cause a side effect. This may be determined by those skilled in the art according to factors including a patient's gender, age, weight and health condition, a type of disease, severity, activity of a drug, sensitivity to a drug, an administration method, an administration time, an administration route, an excretion rate, a treatment period, a drug combined or concurrently used, as well as other factors well known in a pharmaceutical field. The pharmaceutical composition according to the present invention may be administered as an individual therapeutic agent or in combination with other therapeutic agents, may be administered sequentially or simultaneously with a conventional therapeutic agent, and may be administered in a single or multiple manner. Considering all the factors described above, it may be important to carry out an administration by an amount, in which the maximum effect may be achieved by the minimum amount without a side effect, in which such amount may be easily determined by those skilled in the art.

Specifically, the effective amount of the pharmaceutical composition of the present invention may vary depending on a patient's age, gender, condition and weight, a degree of absorption of an active ingredient into the body, an inactivation rate and excretion rate, a disease type, and a concurrently used drug, and may be generally administered in an amount of about 0.01 to 300 mg per 1 kg of body weight, more specifically about 0.1 to 100 mg, which may be administered every day or every other day, or administered at one time to three times a day by dividing the daily dosage of the composition. However, the effective amount may increase or decrease depending on the route of administration, the severity of obesity, gender, weight, age, etc., and thus the dosage may not be intended to limit the scope of the present invention in any way.

In the present invention, "individual" may refer to a subject, whose disease needs to be prevented or treated, and more specifically to a mammal such as a human, monkey, mouse, dog, cat, horse, cow, etc., but is not limited thereto.

The present invention may provide a use of a compound according to any one of above (1) to (6), a stereoisomer thereof, a pharmaceutically acceptable salt thereof, a hydrate thereof or a solvate thereof, or a pharmaceutical composition including the same for preventing or treating cancer.

The present invention may provide a use of a compound according to any one of above (1) to (6), a stereoisomer thereof, a pharmaceutically acceptable salt thereof, a hydrate thereof or a solvate thereof, or a pharmaceutical composition including the same in preparation of a medicament for preventing or treating cancer.

Matters mentioned in each of the items of the present invention, that is, the compound, the pharmaceutical composition including the same, the treatment method using the same, and the use thereof may be applied the same, if not contradictory to each other. Besides, the terms and abbreviations used in the present specification may have their original meanings, unless defined otherwise.

### Advantageous Effects

The present invention may provide a compound represented by formula I, a stereoisomer thereof, a pharmaceutically acceptable salt thereof, a hydrate thereof or a solvate thereof, which has excellent intracellular permeability and in vivo stability as a compound having a novel structure.

A compound represented by formula I of the present invention, a stereoisomer thereof, a pharmaceutically acceptable salt thereof, a hydrate thereof or a solvate thereof may be advantageously used for preventing or treating cancer by inhibiting TEAD protein activity.

### Description of Drawings

FIGS. 1 and 2 show the results of evaluating a tumor growth inhibitory effect of the compound of the present invention using a xenograft mouse model according to Experimental Example 4, and FIG. 2 shows the results on day 24 of FIG. 1.
FIG. 3 shows the results of evaluating a change in mouse body weight according to the administration of the compound of the present invention in Experimental Example 4.

### Mode for Invention

Hereinafter, the present invention will be described in more detail through preparation examples and exemplary examples. However, the following preparation examples and exemplary examples are provided for the purpose of illustrating the present invention, and thus it will be apparent to those skilled in the art that the scope of the present invention is not limited thereto.

### Preparation of compound represented by formula I

The compound represented by formula I of the present invention may be prepared through the method described below. A starting material may be commercially available or may be prepared by known methods, unless otherwise described. All examples provided herein or the use of an exemplary language are merely intended to better illustrate the invention and do not limit the scope of the claimed invention.

### <Preparation Example>

Each of the compounds according to the present invention was synthesized by a method described below, and a conventional method derived by combining the following specific synthesis methods may be used by those skilled in the art. Each of the compounds used in the synthesis was purchased from a supplier outside or synthesized by using an organic synthesis method apparent to those skilled in the art or, and was used without a separate purification process. The compound of each example was identified through 400MHz ¹H-NMR (Agilent, 400-MR), LC-Mass (Waters, SQD2) analysis.

In the present specification, Cs₂CO₃ refers to cesium carbonate, CuI means copper(I) iodide, Cu₂O represents copper(I) oxide, DMSO refers to dimethylsulfoxide, NH₄Cl means ammonium chloride, NMP represents N-methyl-2-pyrrolidone, and Zn refers to zinc.

### Preparation Example 1. Synthesis of N-(3-methyl-1-(4-(trifluoromethyl)benzyl)-1H-pyrrolo[2,3-b]pyridin-5-yl)acrylamide (compound 3)

### [Step a-1] Synthesis of 3-methyl-5-nitro-1-(4-(trifluoromethyl)benzyl)-1H-pyrrolo[2,3-b]pyridine: Alkylation reaction of amine with alkyl halide

4-(trifluoromethyl)benzyl bromide (8.26 g, 34.54 mmol) was slowly added dropwise to a mixture of 3-methyl-5-nitro-1H-pyrrolo[2,3-b]pyridine (5.10 g, 28.79 mmol), Cs₂CO₃ (14.07 g, 43.18 mmol) and dimethylformamide (144 mL), and then stirred at 70°C for four hours. After that, a temperature of the reaction mixture was lowered to room temperature (15 to 25°C), distilled water (300 mL) was added to terminate the reaction, and the obtained solid was filtered. The filtered solid was washed four times with distilled water (50 mL) and twice with diethyl ether (50 mL), and then dried in a vacuum oven at 60°C for two hours. After that, the target compound 3-methyl-5-nitro-1-(4-(trifluoromethyl)benzyl)-1H-pyrrolo[2,3-b]pyridine (8.34 g, 86.43%) was obtained as a white solid without a separate purification process.

### [Step b-1] Synthesis of 3-methyl-1-(4-(trifluoromethyl)benzyl)-1H-pyrrolo[2,3-b]pyridin-5-amine: Synthesis of primary amine through selective amination reaction of nitro compound

NH₄Cl (9.98 g, 186.56 mmol) and Zn dust (12.20 g, 186.56 mmol) were added dropwise to a mixture of 1,4-dioxane/distilled water (3:1 v/v, 120 mL) and 3-methyl-5-nitro-1-(4-(trifluoromethyl)benzyl)-1H-pyrrolo[2,3-b]pyridine (8.34 g, 24.87 mmol) synthesized in above [step a-1] at 0°C, and then stirred at room temperature for three hours. After that, the reaction was terminated by slowly adding distilled water (100 mL) while a container including the reaction mixture was put into cooling water (0 to 5°C). Then, Celite^{™} was laid on, and the reaction mixture was filtered while washing with distilled water (200 mL) and ethyl acetate (200 mL). The remaining filtrate was washed three times with brine (50 mL), and then an organic layer was separated, dried with magnesium sulfate (MgSO₄), and concentrated by drying under reduced pressure. The concentrated mixture was recrystallized with hexane and diethyl ether and filtered. The obtained solid compound was washed three times with hexane (20 mL), and then dried in a vacuum oven at 60°C for two hours. After that, the target compound 3-methyl-1-(4-(trifluoromethyl)benzyl)-1H-pyrrolo[2,3-b]pyridin-5-amine (4.81 g, 63.34%) was obtained as a somewhat yellowish white solid without a separate purification process.

### [Step c-1] Synthesis of N-(3-methyl-1-(4-(trifluoromethyl)benzyl)-1H-pyrrolo[2,3-b]pyridin-5-yl)acrylamide (compound 3): Synthesis of amide through reaction of acyl chloride and amine

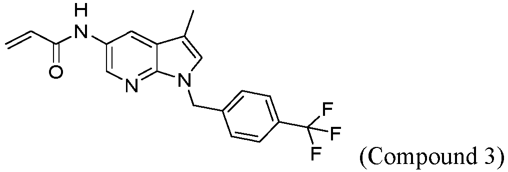

Triethylamine (2.64 mL, 18.91 mmol) and acryloyl chloride (1.34 mL, 16.54 mmol) were sequentially added dropwise while 3-methyl-1-(4-(trifluoromethyl)benzyl)-1H-pyrrolo[2,3-b]pyridin-5-amine (4.81 g, 15.75 mmol) synthesized in above [step b-1] and dichloromethane (80 mL) were stirred at 0°C in the presence of nitrogen (N₂), and the reaction mixture was stirred at room temperature for two hours. After that, the resulting mixture was concentrated by drying under reduced pressure, and the concentrated mixture was separated by column chromatography (hexane : ethyl acetate = 3:1) and then purified by recrystallization (solvent is dichloromethane : hexane=1:1, v/v) to obtain the target compound N-(3-methyl-1-(4-(trifluoromethyl)benzyl)-1H-pyrrolo[2,3-b]pyridin-5-yl)acrylamide (compound 3) (4.97 g, 87.79%) as a white solid.

The target compounds of table 2 below were synthesized through substantially the same reaction as in Preparation Example 1, except that the starting compounds of table 2 below corresponding to each target compound were used instead of 3-methyl-5-nitro-1H-pyrrolo[2,3-b]pyridine used in [Step a-1] of above Preparation Example 1.

However, in the case of compounds 21 and 25, instead of acryloyl chloride, but-2-enoylchloride was used for compound 21 and isobutyryl chloride was used for compound 25.

**[Table 2]**

| **Compound No.** | **Starting compound** | **Target compound** |
|---|---|---|
| **4** | 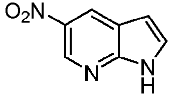 | 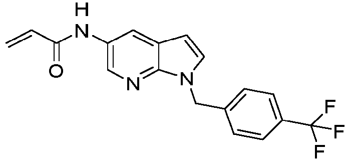 |
| **5** | 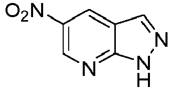 | 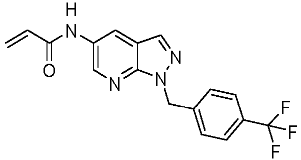 |
| **6** | 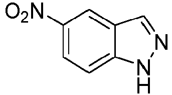 | 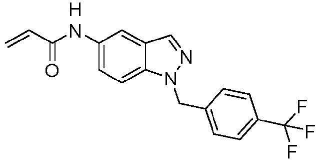 |
| **7** | 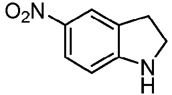 | 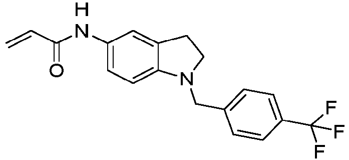 |
| **13** | 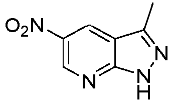 | 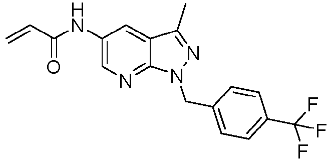 |
| **17** | 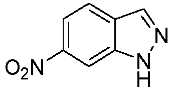 | 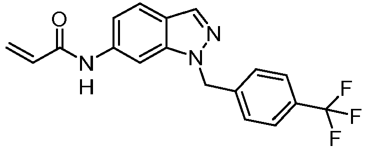 |
| **18** | 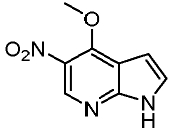 | 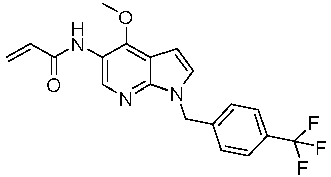 |
| **21** | 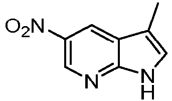 | 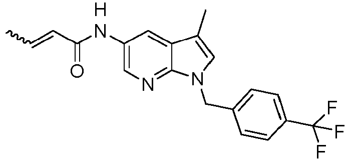 |
| **25** | 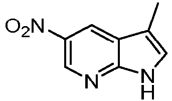 | 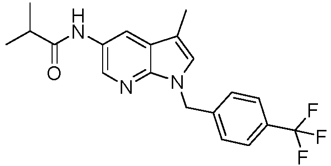 |
| **32** | 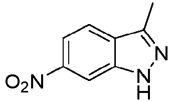 | 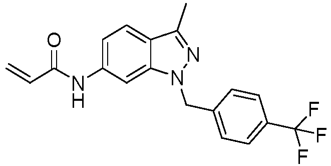 |
| **33** | 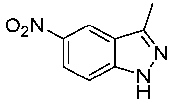 | 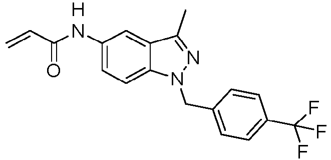 |
| **38** | 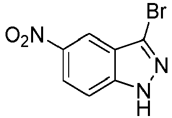 | 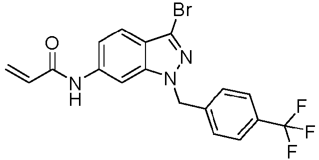 |
| **40** | 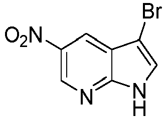 | 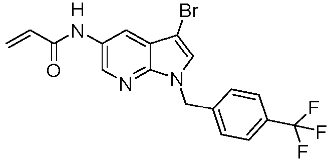 |
| **43** | 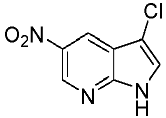 | 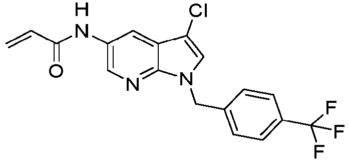 |

The target compounds of table 3 below were synthesized through substantially the same reaction as in above Preparation Example 1, except that the starting compounds of table 3 below and R_{A}-X were used, respectively, as compounds corresponding to target compounds instead of 3-methyl-5-nitro-1H-pyrrolo[2,3-b]pyridine and 4-(trifluoromethyl)benzyl bromide in above Preparation Example 1.

However, but-2-enoyl chloride was used instead of acryloyl chloride for compound 23.

**[Table 3]**

| **Compound No.** | **Starting compound** | **R_{A}-X (X is Br)** | **Target compound** |
|---|---|---|---|
| **8** | 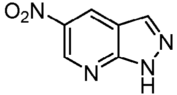 | 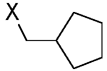 | 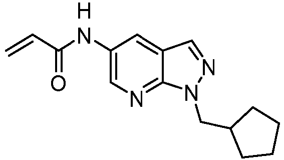 |
| **9** | 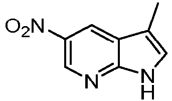 | 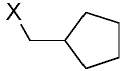 | 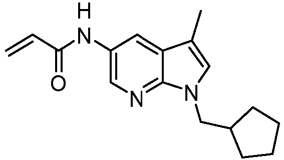 |
| **10** | 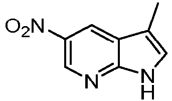 | 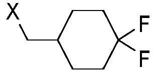 | 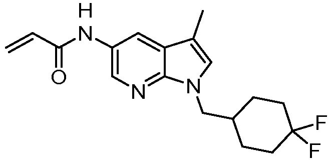 |
| **11** | 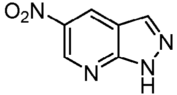 | 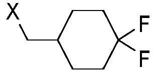 | 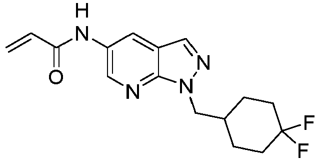 |
| **12** | 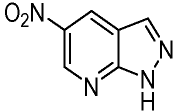 | 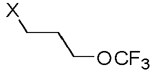 | 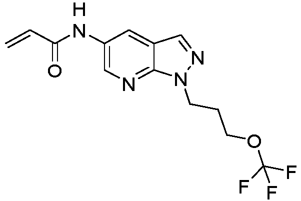 |
| **14** | 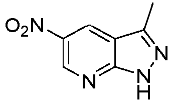 | 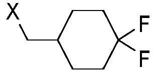 | 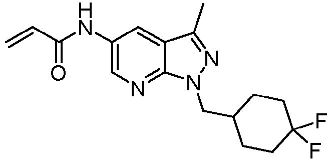 |
| **23** | 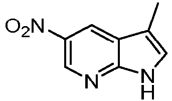 | 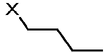 | 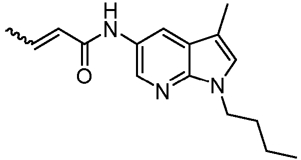 |
| **26** | 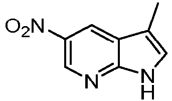 | 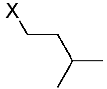 | 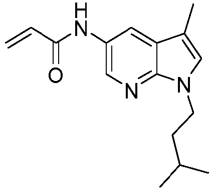 |
| **44** | 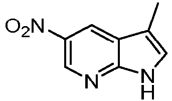 | 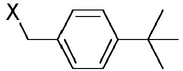 | 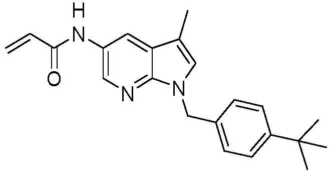 |
| **45** | 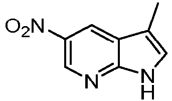 | 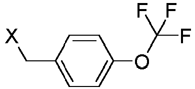 | 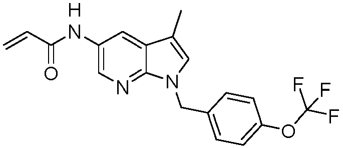 |
| **48** | 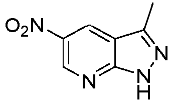 | 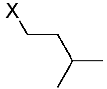 | 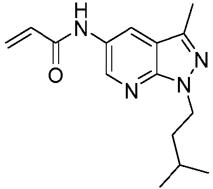 |
| **49** | 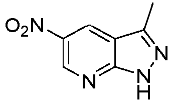 | 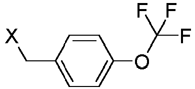 | 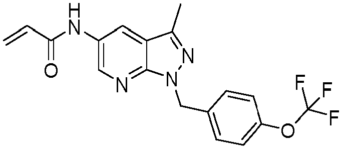 |
| **53** | 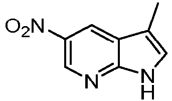 | 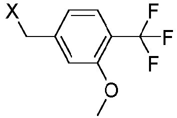 | 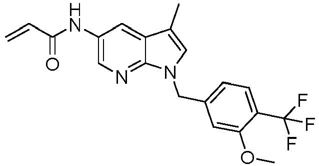 |
| **55** | 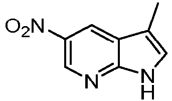 | 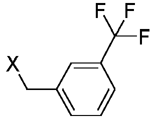 | 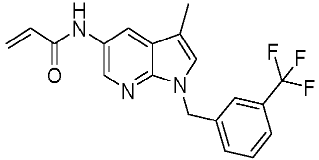 |
| **56** | 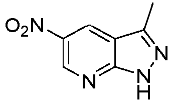 | 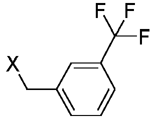 | 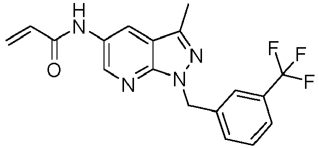 |
| **60** | 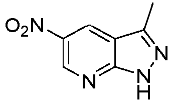 | 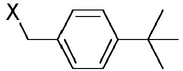 | 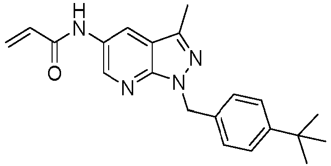 |
| **61** | 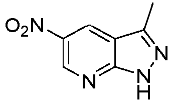 | 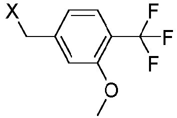 | 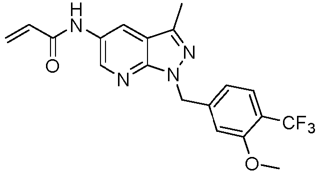 |
| **62** | 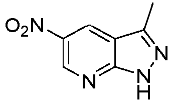 | 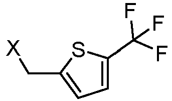 | 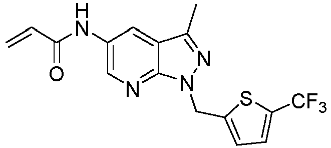 |
| **63** | 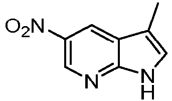 | 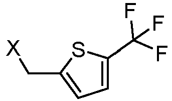 | 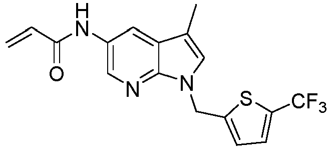 |
| **67** | 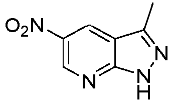 | 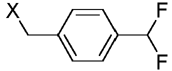 | 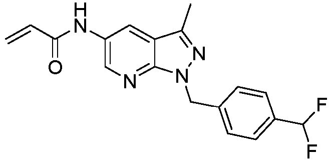 |
| **71** | 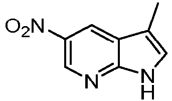 | 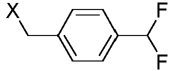 | 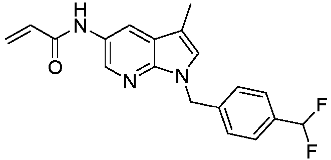 |

### Preparation Example 2. Synthesis of N-(3-methyl-1-(4-(trifluoromethyl)phenyl)-1H-pyrrolo[2,3-b]pyridin-5-yl)acrylamide (compound 27)

### [Step a-2] Synthesis of 3-methyl-5-nitro-1-(4-(trifluoromethyl)phenyl)-1H-pyrrolo[2,3-b]pyridine: Alkylation reaction through Suzuki coupling

A mixture of 3-methyl-5-nitro-1H-pyrrolo[2,3-b]pyridine (5.03 g, 28.40 mmol), 4-(trifluoromethyl)phenylboronic acid (13.18 g, 69.38 mmol), copper acetate (II) (Cu(OAc)₂)(7.74 g, 42.60 mmol), triethylamine (11.88 mL, 85.21 mmol) and dichloromethane (120 mL) was refluxed at 110°C for two hours in the presence of nitrogen (N₂). After that, Celite^{™} was laid on, and the reaction mixture was filtered while washing with methanol (50 mL), dichloromethane (100 mL) and ethyl acetate (100 mL). The remaining filtrate was concentrated in vacuum, dissolved again in ethyl acetate (100 mL), and then washed twice with distilled water (50 mL) and saturated brine. The organic layer was separated, dried over magnesium sulfate (MgSO₄), concentrated by drying under reduced pressure, and purified by column chromatography (hexane : ethyl acetate=2:1, v/v) to obtain the target compound 3-methyl-5-nitro-1-(4-(trifluoromethyl)phenyl)-1H-pyrrolo[2,3-b]pyridine (2.95 g, 32.34%) as a white solid.

### [Step b-1] and [Step c-1] Synthesis of N-(3-methyl-1-(4-(trifluoromethyl)phenyl)-1H-pyrrolo[2,3-b]pyridin-5-yl)acrylamide (compound 27)

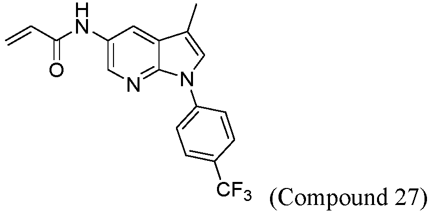

The target compound N-(3-methyl-1-(4-(trifluoromethyl)phenyl)-1H-pyrrolo[2,3-b]pyridin-5-yl)acrylamide (compound 27) was synthesized through substantially the same reaction as in [Step b-1] and [Step c-1] of above Preparation Example 1, except that 3-methyl-5-nitro-1-(4-(trifluoromethyl)phenyl)-1H-pyrrolo[2,3-b]pyridine, an intermediate compound synthesized through an alkylation reaction using Suzuki coupling of [Step a-2] of above Preparation Example 2, was used instead of an alkylation reaction of [Step a-1] of above Preparation Example 1.

The target compounds of table 4 below were synthesized through substantially the same reaction as in above Preparation Example 2, except that the starting compounds of table 4 below and R_{A}-B(OH)₂ were used, respectively, instead of 3-methyl-5-nitro-1H-pyrrolo[2,3-b]pyridine and 4-(trifluoromethyl)phenylboronic acid in [Step a-2] of above Preparation Example 2.

However, in case of compound 85, ethenesulfonyl chloride was used instead of acryloyl chloride. In case of compound 88, but-2-enoylchloride was used instead of acryloyl chloride.

**[Table 4]**

| **Compound No.** | **Starting compound** | **R_{A}-B(OH)₂** | **Target compound** |
|---|---|---|---|
| **34** | 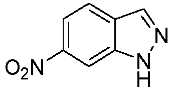 | 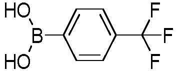 | 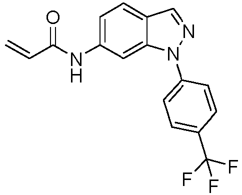 |
| **35** | 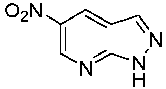 | 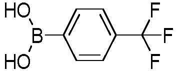 | 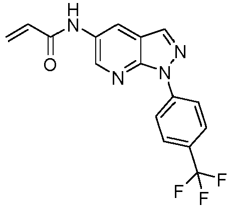 |
| **36** | 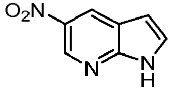 | 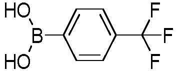 | 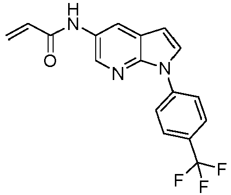 |
| **37** | 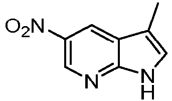 | 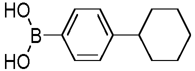 | 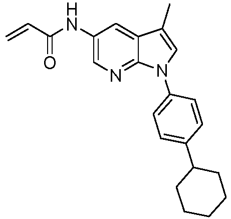 |
| **39** | 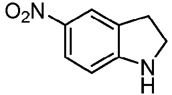 | 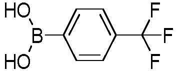 | 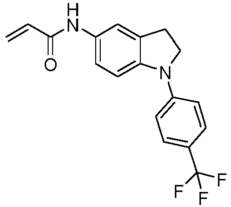 |
| **42** | 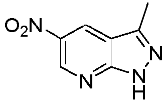 | 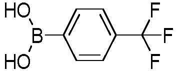 | 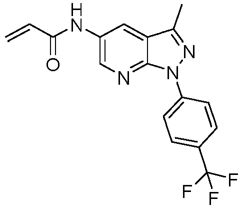 |
| **47** | 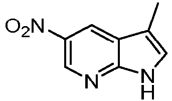 | 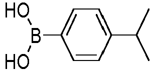 | 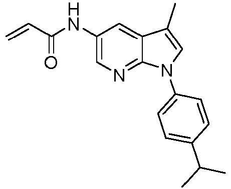 |
| **50** | 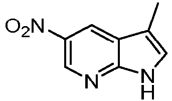 | 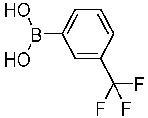 | 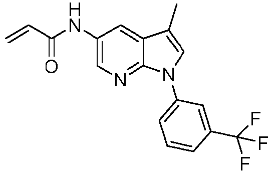 |
| **51** | 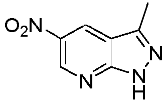 | 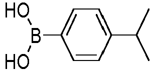 | 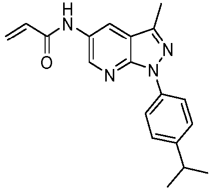 |
| **52** | 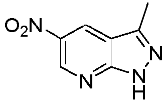 | 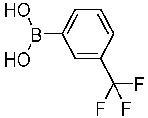 | 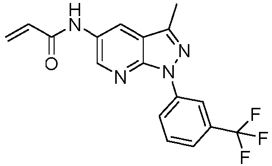 |
| **54** | 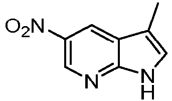 | 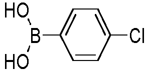 | 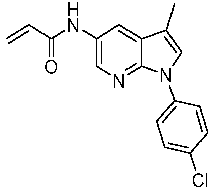 |
| **58** | 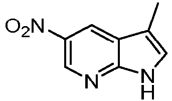 | 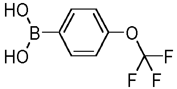 | 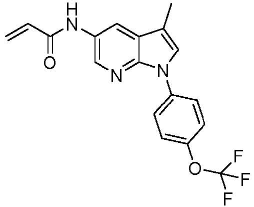 |
| **59** | 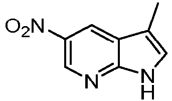 | 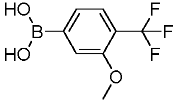 | 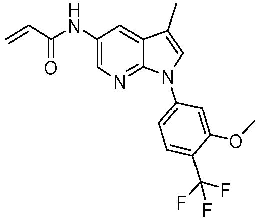 |
| **64** | 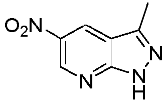 | 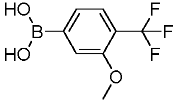 | 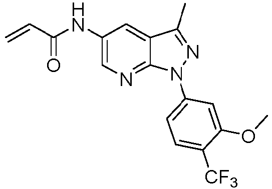 |
| **65** | 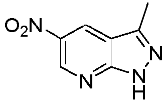 | 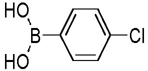 | 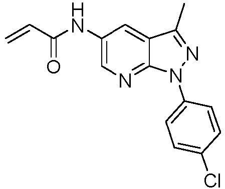 |
| **66** | 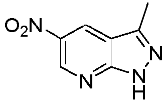 | 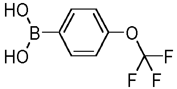 | 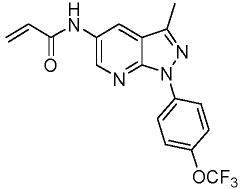 |
| **68** | 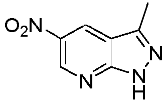 | 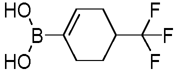 | 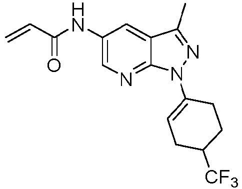 |
| **69** | 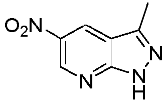 | 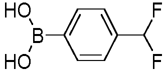 | 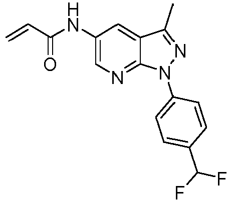 |
| **70** | 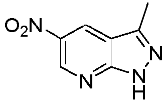 | 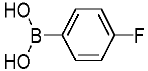 | 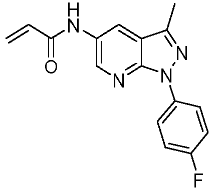 |
| **72** | 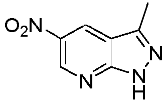 | 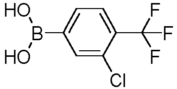 | 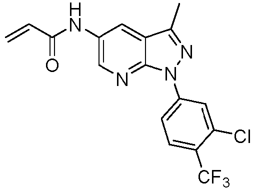 |
| **73** | 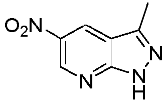 | 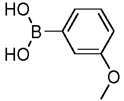 | 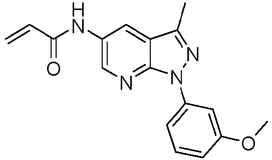 |
| **74** | 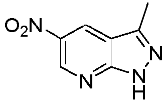 | 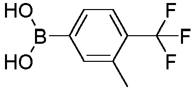 | 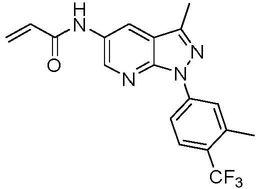 |
| **75** | 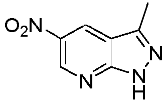 | 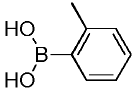 | 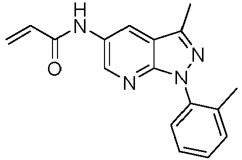 |
| **76** | 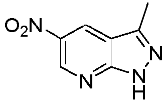 | 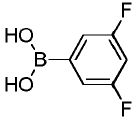 | 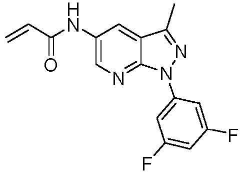 |
| **77** | 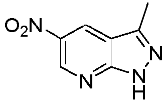 | 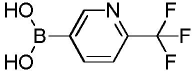 | 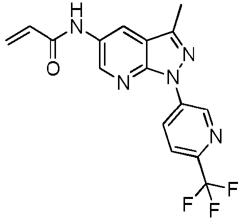 |
| **78** | 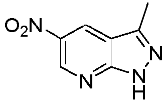 | 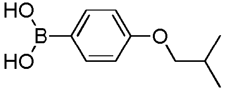 | 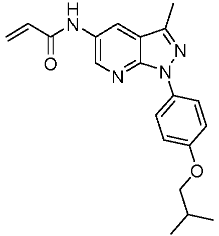 |
| **79** | 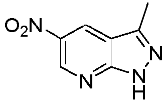 | 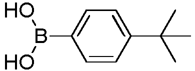 | 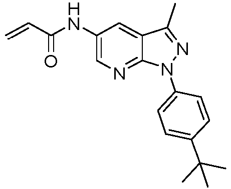 |
| **80** | 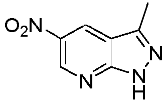 | 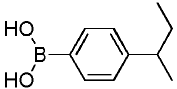 | 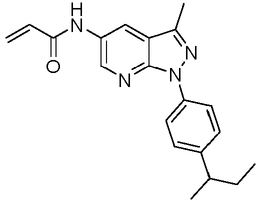 |
| **81** | 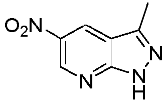 | 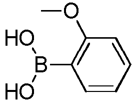 | 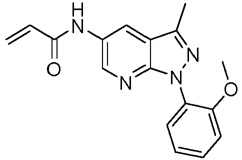 |
| **82** | 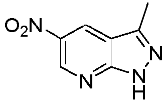 | 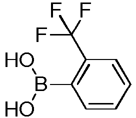 | 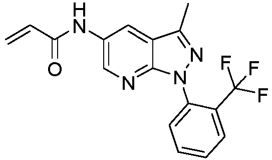 |
| **83** | 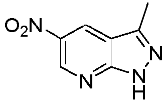 | 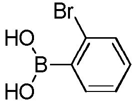 | 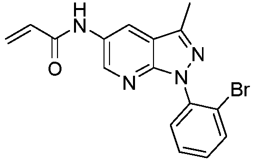 |
| **84** | 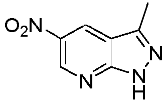 | 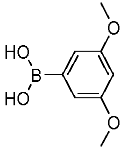 | 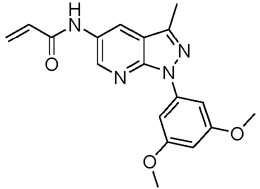 |
| **85** | 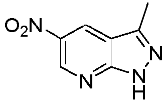 | 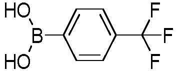 | 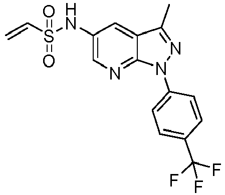 |
| **87** | 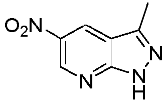 | 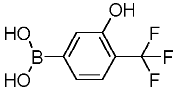 | 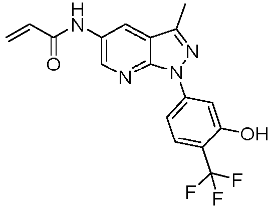 |
| **88** | 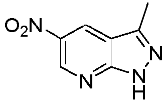 | 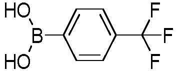 | 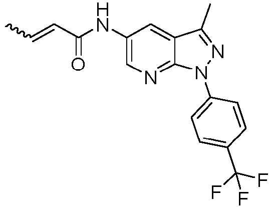 |
| **89** | 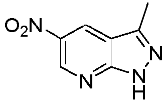 | 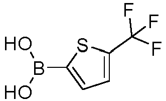 | 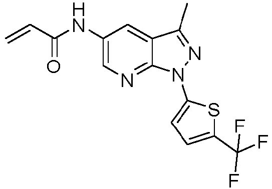 |
| **93** | 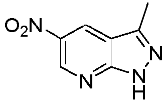 | 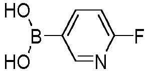 | 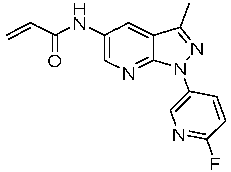 |
| **94** | 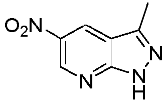 | 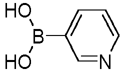 | 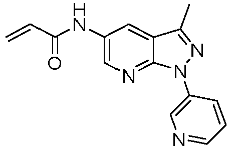 |
| **95** | 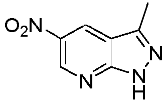 | 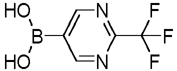 | 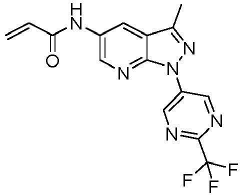 |
| **96** | 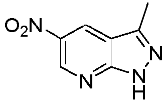 | 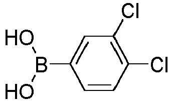 | 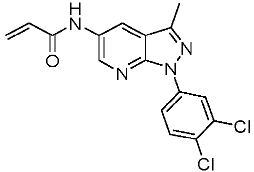 |
| **97** | 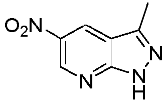 | 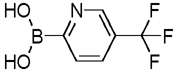 | 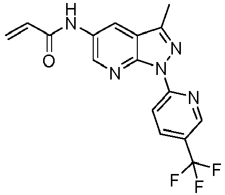 |
| **98** | 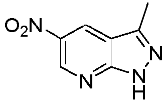 | 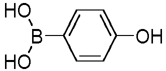 | 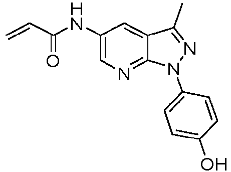 |
| **99** | 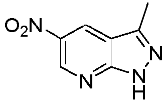 | 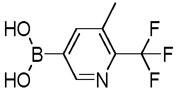 | 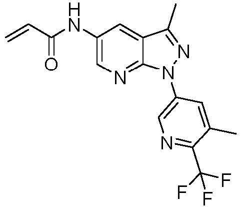 |
| **100** | 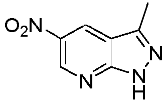 | 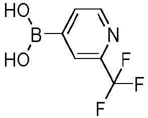 | 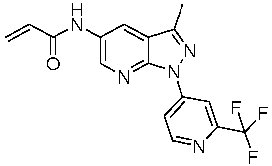 |
| **101** | 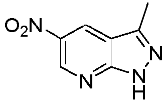 | 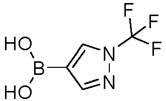 | 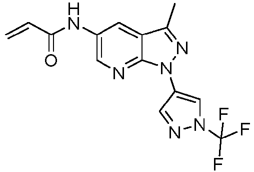 |
| **102** | 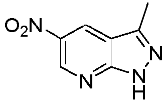 | 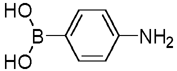 | 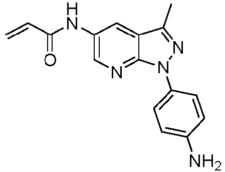 |
| **103** | 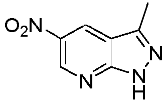 | 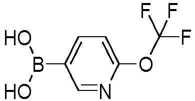 | 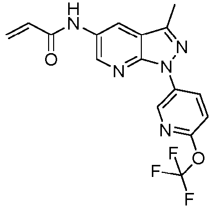 |
| **104** | 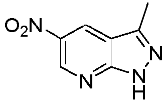 | 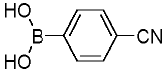 | 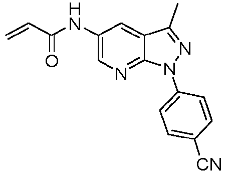 |
| **105** | 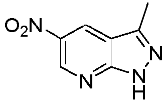 | 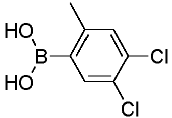 | 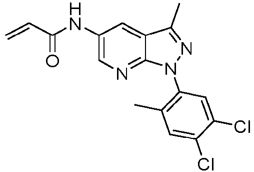 |

### Preparation Example 3: Synthesis of N-methyl-N-(3-methyl-1-(4-(trifluoromethyl)phenyl)-1H-pyrazolo[3,4-b]pyridin-5-yl)acrylamide (compound 86)

While compound 42 (N-(3-methyl-1-(4-(trifluoromethyl)phenyl)-1H-pyrazolo[3,4-b]pyridin-5-yl)acrylamide (122.2 mg, 350 µmol) synthesized through [Step c-1] of above Preparation Example 2 and N,N-dimethylformamide (1 mL) were stirred at room temperature, cesium carbonate (172.7 mg, 530 µmol) and iodomethyl (32.9 µmL, 530 µmol) were sequentially added thereto. The reaction mixture was stirred at room temperature for three hours, after which distilled water (10 mL) was added to the reaction mixture to terminate the reaction. The resulting mixture was extracted twice with ethyl acetate (20 mL), and washed with distilled water (10 mL) and saturated brine (10 mL). The organic layer was separated, dried over magnesium sulfate, concentrated by drying under reduced pressure, and purified by column chromatography (hexane : ethyl acetate=2:1, v/v) to obtain the target compound N-methyl-N-(3-methyl-1-(4-(trifluoromethyl)phenyl)-1H-pyrazolo[3,4-b]pyridin-5-yl)acrylamide (91.3 mg, 72.4%) as a white solid.

The target compounds 90 to 92 of table 5 below were synthesized through substantially the same reaction as in above Preparation Example 3, except that compounds 66, 65 and 77 were used, respectively, instead of compound 42 (N-(3-methyl-1-(4-(trifluoromethyl)phenyl)-1H-pyrazolo[3,4-b]pyridin-5-yl)acrylamide in above Preparation Example 3.

**[Table 5]**

| **Compound No.** | **Target compound** | **Compound No.** | **Target compound** |
|---|---|---|---|
| **86** | 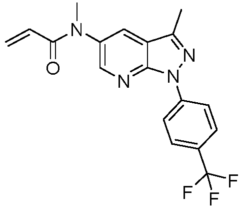 | **90** | 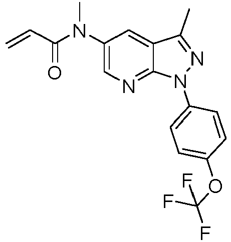 |
| **91** | 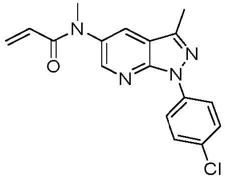 | **92** | 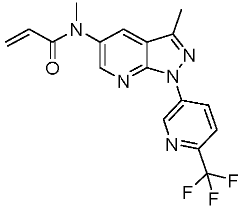 |

### Preparation Example 4. Synthesis of N-(3-methyl-1-(4-(trifluoromethyl)benzoyl)-1H-pvrrolo[2,3-b]pyridin-5-yl)acrylamide (compound 24)

### [Step a-3] Synthesis of (3-methyl-5-nitro-1H-pyrrolo[2,3-b]pyridin-1-yl)(4-(trifluoromethyl)phenyl)methanone: Amide synthesis via acylation reaction

A mixture of 3-methyl-5-nitro-1H-pyrrolo[2,3-b]pyridine (50 mg, 282.23 µmol), 4-(trifluoromethyl)benzoylchloride (125.82 µL, 846.68 µmol), triethylamine (51.14 µL, 366.89 µmol) and dichloromethane (2 mL) was stirred at room temperature for two hours in the presence of nitrogen (N₂). After that, the resulting mixture was treated with distilled water (5 mL), extracted twice with ethyl acetate (5 mL), and washed with distilled water (5 mL) and saturated brine (5 mL). The organic layer was separated, dried over magnesium sulfate (MgSO₄), concentrated by drying under reduced pressure, and purified by column chromatography (hexane : ethyl acetate=2:1, v/v) to obtain the target compound (3-methyl-5-nitro-1H-pyrrolo[2,3-b]pyridin-1-yl)(4-(trifluoromethyl)phenyl)methanone (61 mg, 61.88%) as a white solid.

### [Step b-1] and [Step c-1] Synthesis of N-(3-methyl-1-(4-(trifluoromethyl)benzoyl)-1H-pyrrolo[2,3-b]pyridin-5-yl)acrylamide (compound 24)

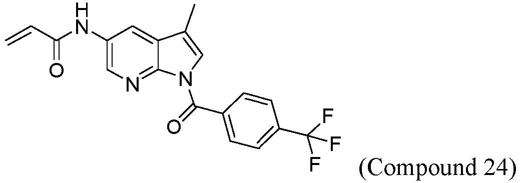

Target compound N-(3-methyl-1-(4-(trifluoromethyl)benzoyl)-1H-pyrrolo[2,3-b]pyridin-5-yl)acrylamide (compound 24) was synthesized through substantially the same reaction as in [Step b-1] and [Step c-1] of above Preparation Example 1, except for using the compound (3-methyl-5-nitro-1H-pyrrolo[2,3-b]pyridin-1-yl)(4-(trifluoromethyl)phenyl)methanone obtained from [Step a-3] of above Preparation Example 4.

### Preparation Example 5. Synthesis of N-(1-cyclohexyl-3-methyl-1H-pyrrolo[2,3-b]pyridin-5-yl)acrylamide (compound 57)

### [Step a-4] Synthesis of 1-cyclohexyl-3-methyl-5-nitro-1H-pyrrolo[2,3-b]pyridine: Use of microwaves

Compound 3-methyl-5-nitro-1H-pyrrolo[2,3-b]pyridine (200 mg, 112 µmol), which was a starting material of [Step a-1] of above Preparation Example 1, potassium carbonate (K₂CO₃) (460 mg, 338 µmol), LiCl (47 mg, 112 µmol), and CuI (21 mg, 11 µmol) were added to a microwave vial and mixed. Argon (Ar) gas was purged into said vial, dimethylformamide (2 mL) was added thereto, and then cyclohexane bromide (170 µL, 1.35 mmol) was added dropwise thereto. The reaction mixture was allowed to react in a 150°C microwave reactor (Biotage initiator+) for 30 minutes. After that, the reaction mixture was treated with an aqueous 1 N NaOH solution and extracted twice with ethyl acetate (10 mL). The organic layer was dried over magnesium sulfate (MgSO₄) and concentrated by drying under reduced pressure, and then the resulting mixture was purified by column chromatography (hexane : ethyl acetate=3:1, v/v) to obtain the target compound 1-cyclohexyl-3-methyl-5-nitro-1H-pyrrolo[2,3-b]pyridine (20 mg, 6.9%) as a white solid.

### [Step b-1] and [Step c-1] Synthesis of N-(1-cyclohexyl-3-methyl-1H-pyrrolo[2,3-b]pyridin-5-yl)acrylamide (compound 57)

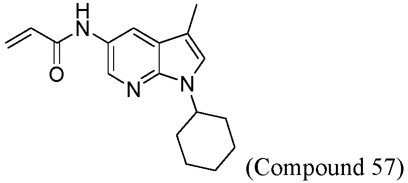

Target compound N-(1-cyclohexyl-3-methyl-1H-pyrrolo[2,3-b]pyridin-5-yl)acrylamide (compound 57) (yield of 34.4%) was synthesized through substantially the same reaction as in [Step b-1] and [Step c-1] of above Preparation Example 1, except for using the compound 1-cyclohexyl-3-methyl-5-nitro-1H-pyrrolo[2,3-b]pyridine obtained from [Step a-4] of above Preparation Example 5.

### Preparation Example 6. Synthesis of N-(1-(4-(trifluoromethyl)benzyl)-1H-pyrazolo[3,4-d]pyrimidin-6-yl)acrylamide (compound 19)

### [Step e-1] Synthesis of 1-(4-(trifluoromethyl)benzyl)-1H-pyrazolo[3,4-d]pyrimidine-6-amine: Copper-catalyzed amination reaction of halide compound

A mixture of the compound 6-chloro-1-(4-(trifluoromethyl)benzyl)-1H-pyrazolo[3,4-d]pyrimidine (31 mg, 99.14 µmol) synthesized in substantially the same manner as in [Step a-1] of above Preparation Example 1 except for using 6-chloro-1H-pyrazolo[3,4-d]pyrimidine instead of 3-methyl-5-nitro-1H-pyrrolo[2,3-b]pyridine, Cu₂O (70.93 mg, 495.71 µmol), and a reaction solvent (1 mL, NH₄OH : NMP = 1:1, v/v) was added to a pressure vessel in the presence of nitrogen (N₂) and the reaction was carried out at 180°C for four hours. After that, Celite^{™} was laid on, and the reaction mixture was filtered while washing three times with distilled water (3 mL) and ethyl acetate (3 mL), respectively. The organic layer was separated from the remaining filtrate, dried over magnesium sulfate (MgSO₄) and concentrated by drying under reduced pressure, and then the resulting mixture was purified by column chromatography (ethyl acetate : hexane = 1: 1, v/v) to obtain the target compound 1-(4-(trifluoromethyl)benzyl)-1H-pyrazolo[3,4-d]pyrimidin-6-amine (27 mg, 92.87%) as a white solid.

### [Step c-1] Synthesis of N-(1-(4-(trifluoromethyl)benzyl)-1H-pyrazolo[3,4-d] pyrimidin-6-yl)acrylamide

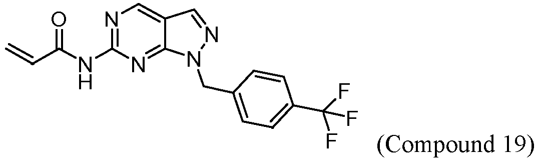

Target compound N-(1-(4-(trifluoromethyl)benzyl)-1H-pyrazolo[3,4-d]pyrimidin-6-yl)acrylamide was synthesized through substantially the same reaction as in [Step c-1] of above Preparation Example 1, except for using the compound 1-(4-(trifluoromethyl)benzyl)-1H-pyrazolo[3,4-d]pyrimidin-6-amine obtained from [Step e-1] of above Preparation Example 6.

The target compounds of table 6 below were synthesized through substantially the same reaction as in above Preparation Example 6, except that the starting compounds of table 6 below and R_{A}-X or R_{A}-B(OH)₂ were used, respectively, as compounds corresponding to target compounds instead of 6-chloro-1H-pyrazolo[3,4-d]pyrimidine and 4-(trifluoromethyl)benzyl bromide in above Preparation Example 6.

However, in case of compound 1, ethenesulfonyl chloride was used instead of acryloyl chloride.

**[Table 6]**

| **Compound No.** | **Starting compound (X is Br)** | **R_{A}-X (X is Br) or R_{A}-B(OH)₂** | **Target compound** |
|---|---|---|---|
| **1** | 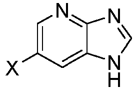 | 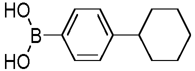 | 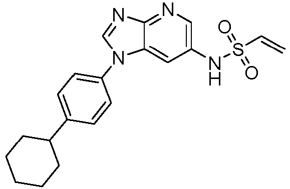 |
| **2** | 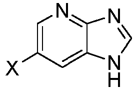 | 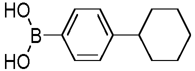 | 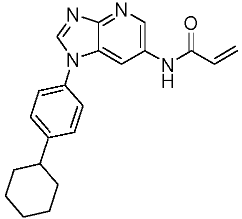 |
| **15** | 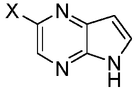 | 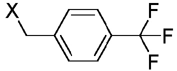 | 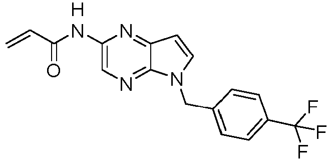 |
| **16** | 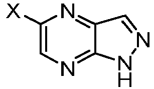 | 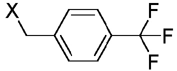 | 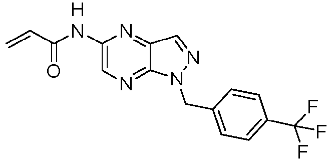 |
| **20** | 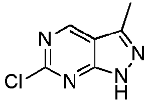 | 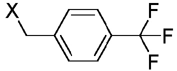 | 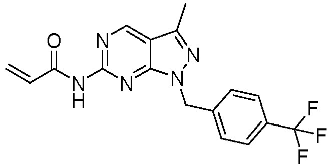 |
| **22** | 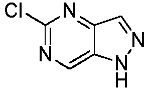 | 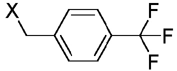 | 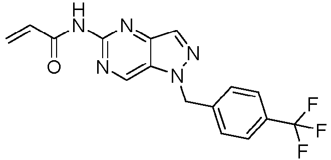 |
| **28** | 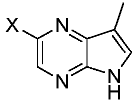 | 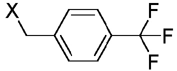 | 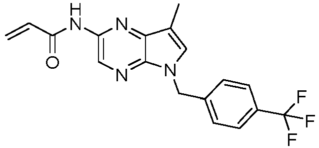 |
| **29** | 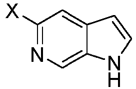 | 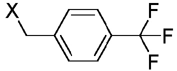 | 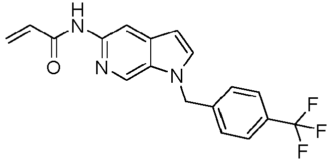 |
| **30** | 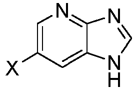 | 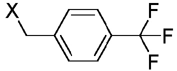 | 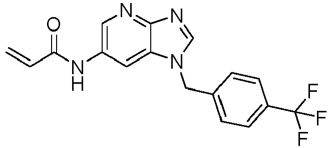 |
| **31** | 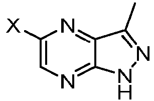 | 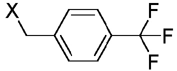 | 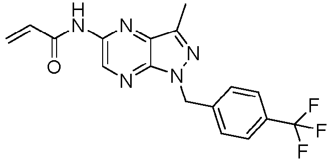 |
| **41** | 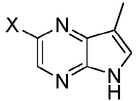 | 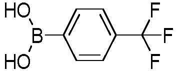 | 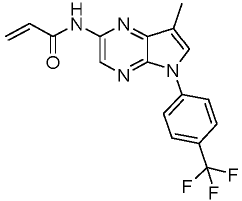 |
| **46** | 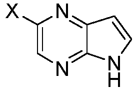 | 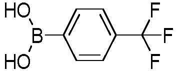 | 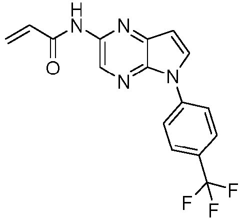 |

The analysis results of compound of each example herein are as shown in table 7 below.

**[Table 7]**

| **Compound No.** | **¹H NMR(400MHz)6 ppm** | **LC/MS ESI m/z (M+H)⁺** |
|---|---|---|
| **1** | ¹H NMR (400 MHz, DMSO-*d₆*) δ 10.16 (s, 1H), 8.75 (s, 1H), 8.30 (d, *J* = 2.3 Hz, 1H), 7.74 (d, *J* = 2.4 Hz, 1H), 7.55 (d, *J* = 8.6 Hz, 2H), 7.49 (d, *J* = 8.5 Hz, 2H), 6.81 (dd, *J* = 16.4, 10.0 Hz, 1H), 6.00 (d, *J* = 10.2 Hz, 1H), 5.97 (d, *J* = 3.8 Hz, 1H), 2.72 - 2.55 (m, 2H), 1.89 - 1.79 (m, 4H), 1.75 - 1.66 (m, 1H), 1.49 - 1.33 (m, 4H). | 383.4 |
| **2** | ¹H NMR (400 MHz, DMSO-*d₆*) δ 10.59 (s, 1H), 8.74 (s, 1H), 8.61 (d, *J* = 22.4 Hz, 2H), 7.59 (d, *J* = 7.5 Hz, 2H), 7.52 (d, *J* = 7.3 Hz, 2H), 6.47 (dd, *J* = 17.0, 10.1 Hz, 1H), 6.29 (d, *J* = 16.8 Hz, 1H), 5.82 (d, *J* = 9.8 Hz, 1H), 2.71 - 2.60 (m, 1H), 1.85 (t, *J* = 11.3 Hz, 4H), 1.74 (d, *J* = 11.5 Hz, 1H), 1.57 - 1.36 (m, 4H), 1.34 - 1.20 (m, 1H). | 347.5 |
| **3** | ¹H NMR (400 MHz, DMSO-*d₆*) δ 10.22 (s, 1H), 8.37 - 8.30 (m, 2H), 7.65 (d, *J =* 8.1 Hz, 2H), 7.40 - 7.31 (m, 3H), 6.45 (dd, *J* = 17.0, 10.1 Hz, 1H), 6.25 (dd, *J* = 17.0, 2.0 Hz, 1H), 5.74 (dd, *J* = 10.1, 2.0 Hz, 1H), 5.48 (s, 2H), 2.23 (s, 3H). | 360.3 |
| **4** | ¹H NMR (400 MHz, DMSO-*d₆*) δ 10.22 (s, 1H), 8.37 (dd, *J* = 6.4, 2.3 Hz, 2H), 7.71 - 7.57 (m, 3H), 7.36 (d, *J* = 8.1 Hz, 2H), 6.52 (d, *J* = 3.5 Hz, 1H), 6.49 - 6.37 (m, 1H), 6.25 (dd, *J =* 17.0, 2.0 Hz, 1H), 5.74 (dd, *J =* 10.1, 2.0 Hz, 1H), 5.55 (s, 2H). | 346.3 |
| **5** | ¹H NMR (400 MHz, DMSO-*d₆*) δ 9.81 (s, 1H), 7.70 (d, *J* = 8.2 Hz, 2H), 7.56 (d, *J* = 8.1 Hz, 2H), 7.46- 7.39 (m, 1H), 7.23 (dd, *J* = 8.4, 2.0 Hz, 1H), 6.50 (d, *J =* 8.4 Hz, 1H), 6.41 - 6.32 (m, 1H), 6.16 (dd, *J* = 17.0, 2.1 Hz, 1H), 5.65 (dd, *J =* 10.1, 2.2 Hz, 1H), 4.31 (s, 2H). | 347.2 |
| **6** | ¹H NMR (400 MHz, DMSO-*d₆*) δ 10.17 (s, 1H), 8.24 (d, *J* = 1.5 Hz, 1H), 8.10 (d, *J* = 0.8 Hz, 1H), 7.71 - 7.63 (m, 3H), 7.48 (dd, *J* = 9.0, 1.9 Hz, 1H), 7.35 (d,*J* = 8.1 Hz, 2H), 6.43 (dd, *J* = 17.0, 10.1 Hz, 1H), 6.24 (dd, *J* = 17.0, 2.1 Hz, 1H), 5.75 - 5.67 (m, 3H). | 346.2 |
| **7** | ¹H NMR (400 MHz, DMSO-*d₆*) δ 9.83 (s, 1H), 7.68 (d, *J* = 8.0 Hz, 2H), 7.54 (d, *J* = 8.0 Hz, 2H), 7.45 (d, *J* = 1.7 Hz, 1H), 7.25 (dd, *J* = 8.4, 2.0 Hz, 1H), 6.50 (d, *J* = 8.4 Hz, 1H), 6.38 (dd, *J* = 17.0, 10.1 Hz, 1H), 6.18 (dd, *J* = 17.0, 2.2 Hz, 1H), 5.64 (dd, *J* = 10.1, 2.1 Hz, 1H), 4.29 (s, 2H), 3.22 (t, *J* = 8.3 Hz, 2H), 2.87 (t, *J* = 8.2 Hz, 2H). | 347.2 |
| **8** | ¹H NMR (400 MHz, DMSO-*d₆*) δ 10.41 (s, 1H), 8.63 - 8.57 (m, 2H), 8.10 (s, 1H), 6.45 (dd, *J* = 17.0, 10.1 Hz, 1H), 6.28 (dd, *J* = 17.0, 2.0 Hz, 1H), 5.78 (dd, *J =* 10.1, 2.0 Hz, 1H), 4.33 (d, *J* = 7.5 Hz, 2H), 1.63 - 1.41 (m, 6H), 1.33 - 1.21 (m, 3H). | 271.3 |
| **9** | ¹H NMR (400 MHz, DMSO-*d₆*) δ 10.17 (s, 1H), 8.34 - 8.26 (m, 2H), 7.30 (s, 1H), 6.44 (dd, *J* = 17.0, 10.1 Hz, 1H), 6.24 (dd, *J* = 17.0, 2.0 Hz, 1H), 5.77 - 5.68 (m, 1H), 4.05 (d, *J* = 7.6 Hz, 2H), 2.44 - 2.33 (m, 1H), 2.21 (s, 3H), 1.65 - 1.42 (m, 6H), 1.28 - 1.16 (m, 2H). | 284.3 |
| **10** | ¹H NMR (400 MHz, DMSO-*d₆*) δ 10.20 (s, 1H), 8.38 - 8.21 (m, 2H), 7.26 (s, 1H), 6.50 - 6.39 (m, 1H), 6.25 (dd, *J* = 17.0, 1.9 Hz, 1H), 5.73 (dd, *J* = 10.1, 1.8 Hz, 1H), 4.06 (d, *J* = 7.2 Hz, 2H), 2.21 (s, 3H), 2.04 - 1.89 (m, 3H), 1.81 - 1.60 (m, 2H), 1.58 - 1.47 (m, 2H), 1.29 - 1.11 (m, 2H). | 334.3 |
| **11** | ¹H NMR (400 MHz, DMSO-*d₆*) δ 10.42 (s, 1H), 8.66 - 8.57 (m, 2H), 8.13 (s, 1H), 6.49 - 6.37 (m, 1H), 6.28 (dd, *J* = 17.0, 2.0 Hz, 1H), 5.78 (dd, *J* = 10.1, 2.0 Hz, 1H), 4.34 (d, *J* = 7.2 Hz, 2H), 2.16 - 2.05 (m, 1H), 2.03 - 1.89 (m, 2H), 1.83 - 1.63 (m, 2H), 1.61 - 1.51 (m, 2H), 1.33 - 1.17 (m, 2H). | 321.3 |
| **12** | ¹H NMR (400 MHz, DMSO-*d₆*) δ 10.44 (s, 1H), 8.63 (s, 2H), 8.15 (s, 1H), 6.45 (dd, *J* = 16.9, 10.0 Hz, 1H), 6.33 - 6.24 (m, 1H), 5.83 - 5.74 (m, 1H), 4.53 (t, *J =* 6.9 Hz, 2H), 4.08 (t, *J* = 6.1 Hz, 2H), 2.28 - 2.18 (m, 2H). | 315.2 |
| **13** | ¹H NMR (400 MHz, DMSO-*d₆*) δ 10.44 (s, 1H), 8.63 - 8.57 (m, 2H), 7.66 (d, *J* = 8.0 Hz, 2H), 7.39 (d, *J* = 8.0 Hz, 2H), 6.45 (dd, *J* = 17.0, 10.1 Hz, 1H), 6.28 (dd, *J* = 17.0, 2.0 Hz, 1H), 5.79 (dd, *J* = 10.1, 2.0 Hz, 1H), 5.66 (s, 2H), 2.47 (s, 3H). | 361.2 |
| **14** | ¹H NMR (400 MHz, DMSO-*d₆*) δ 10.40 (s, 1H), 8.56 (s, 2H), 6.45 (dd, *J* = 17.0, 10.1 Hz, 1H), 6.27 (dd, *J* = 17.0, 2.0 Hz, 1H), 5.78 (dd, *J* = 10.1, 2.0 Hz, 1H), 4.25 (d, *J* = 7.2 Hz, 2H), 2.46 (s, 3H), 2.12 - 2.02 (m, 1H), 2.01 - 1.90 (m, 2H), 1.82 - 1.64 (m, 2H), 1.61 - 1.51 (m, 2H), 1.31 - 1.17 (m, 2H). | 335.3 |
| **15** | ¹H NMR (400 MHz, DMSO-*d₆*) δ 10.84 (s, 1H), 9.11 (s, 1H), 8.02 (d, *J* = 3.5 Hz, 1H), 7.67 (d, *J* = 8.0 Hz, 2H), 7.41 (d, *J* = 8.0 Hz, 2H), 6.67 - 6.51 (m, 2H), 6.31 (dd, *J* = 17.0, 1.9 Hz, 1H), 5.78 (dd, *J* = 10.2, 1.9 Hz, 1H), 5.58 (s, 2H). | 347.3 |
| **16** | ¹H NMR (400 MHz, DMSO-*d₆*) δ 11.15 (s, 1H), 9.42 (s, 1H), 8.37 (s, 1H), 7.68 (d, *J* = 8.0 Hz, 2H), 7.44 (d, *J* = 8.0 Hz, 2H), 6.62 (dd, *J* = 17.0, 10.2 Hz, 1H), 6.37 (dd, *J* = 17.0, 1.9 Hz, 1H), 5.85 (dd, *J* = 10.2, 1.8 Hz, 1H), 5.80 (s, 2H). | 348.2 |
| **17** | ¹H NMR (400 MHz, DMSO-*d₆*) δ 10.31 (s, 1H), 8.17 (s, 1H), 8.06 (s, 1H), 7.69 (dd, *J =* 16.4, 8.4 Hz, 3H), 7.31 (d, *J* = 8.0 Hz, 2H), 7.20 (d, *J* = 8.8 Hz, 1H), 6.44 (dd, *J* = 17.0, 10.0 Hz, 1H), 6.25 (d, *J* = 16.9 Hz, 1H), 5.75 (d, *J* = 10.1 Hz, 1H), 5.70 (s, 2H). | 346.2 |
| **18** | ¹H NMR (400 MHz, DMSO-*d₆*) δ 9.44 (s, 1H), 8.31 (s, 1H), 7.66 (d, *J* = 8.2 Hz, 2H), 7.56 (d, *J* = 3.5 Hz, 1H), 7.38 (d, *J* = 7.9 Hz, 2H), 6.85 (d, *J* = 3.5 Hz, 1H), 6.55 (dd, *J* = 17.3, 10.0 Hz, 1H), 6.19 (d, *J* = 15.5 Hz, 1H), 5.70 (d, *J* = 10.4 Hz, 1H), 5.53 (s, 2H), 4.24 (s, 3H). | 376.3 |
| **19** | ¹H NMR (400 MHz, Chloroform-*d*) δ 9.22 (s, 1H), 8.23 (s, 1H), 7.57 (d, *J* = 8.0 Hz, 2H), 7.47 (d, *J* = 8.1 Hz, 2H), 6.54 (d, *J =* 16.2 Hz, 1H), 6.37 (dd, *J* = 16.8, 10.3 Hz, 2H), 5.79 (d, *J* = 9.8 Hz, 1H), 5.66 (s, 2H). | 348.2 |
| **20** | ¹H NMR (400 MHz, Chloroform-*d*) δ 9.23 (s, 1H), 7.58 (d, *J* = 8.0 Hz, 2H), 7.47 (d, *J* = 8.1 Hz, 2H), 6.54 (d, *J* = 16.2 Hz, 1H), 6.37 (dd, *J* = 16.8, 10.3 Hz, 2H), 5.79 (d, *J* = 9.8 Hz, 1H), 5.66 (s, 2H), 1.96 (s, 3H). | 362.3 |
| **21** | ¹H NMR (400 MHz, CD₃OH) δ 8.71 (d, *J* = 1.8 Hz, 1H), 8.51 (d, *J* = 2.4 Hz, 1H), 7.64 (d, *J* = 8.2 Hz, 2H), 7.39 (s, 1H), 7.34 (d, *J* = 7.9 Hz, 2H), 7.13 - 6.89 (m, 2H), 6.16 (d, *J* = 15.0 Hz, 1H), 5.59 (s, 2H), 2.36 (s, 3H), 1.95 (d, *J* = 6.7 Hz, 3H). | 374.3 |
| **22** | ¹H NMR (400 MHz, Chloroform-*d*) δ 9.23 (s, 1H), 8.68 (s, 1H), 7.52 (d, *J =* 8.0 Hz, 2H), 7.46 (d, *J* = 8.1 Hz, 2H), 6.54 (d, *J* = 16.2 Hz, 1H), 6.37 (dd, *J* = 16.8, 10.3 Hz, 2H), 5.79 (d, *J* = 9.8 Hz, 1H), 5.66 (s, 2H). | 348.3 |
| **23** | ¹H NMR (400 MHz, Chlorofonn-*d*) δ 10.56 (s, 1H), 9.34 (s, 1H), 8.89 (s, 1H), 7.05 (s, 2H), 6.41 (t, *J* = 14.8 Hz, 1H), 4.47 (s, 2H), 2.30 (s, 3H), 1.92 (d, *J* = 5.8 Hz, 3H), 1.87 - 1.78 (m, 2H), 1.45 - 1.30 (m, 2H), 0.92 (t, *J* = 6.9 Hz, 3H). | 272.3 |
| **24** | ¹H NMR (400 MHz, DMSO-*d₆*) δ 10.38 (s, 1H), 8.42 (d, *J* = 2.3 Hz, 1H), 8.23 (d, *J* = 2.3 Hz, 1H), 7.90 (q, *J* = 8.8 Hz, 4H), 7.73 (s, 1H), 6.43 (dd, *J* = 17.0, 10.0 Hz, 1H), 6.31 - 6.22 (m, 1H), 5.78 (d, *J* = 11.9 Hz, 1H), 2.26 (s, 3H). | 374.3 |
| **25** | ¹H NMR (400 MHz, DMSO-*d₆*) δ 9.86 (s, 1H), 8.26 (d, *J* = 1.7 Hz, 2H), 7.64 (d, *J* = 8.2 Hz, 2H), 7.38 - 7.27 (m, 3H), 5.47 (s, 2H), 2.64 - 2.54 (m, 1H), 2.21 (s, 3H), 1.10 (d, *J* = 6.8 Hz, 6H). | 376.3 |
| **26** | ¹H NMR (400 MHz, CD₃OD) δ 8.34 (d*, J* = 2.0 Hz, 1H), 8.28 (d, *J* = 2.1 Hz, 1H), 7.17 (s, 1H), 6.52 - 6.33 (m, 3H), 5.82 - 5.74 (m, 1H), 4.22 (t, *J* = 7.4 Hz, 2H), 2.28 (s, 3H), 1.70 (q, *J* = 7.2 Hz, 2H), 1.57 - 1.47 (m, 1H), 0.94 (d, *J* = 6.6 Hz, 6H). | 272.4 |
| **27** | ¹H NMR (400 MHz, DMSO-*d₆*) δ 10.38 (s, 1H), 8.46 (d, *J* = 3.4 Hz, 2H), 8.25 (d, *J* = 8.5 Hz, 2H), 7.91 (s, 1H), 7.86 (d, *J* = 8.6 Hz, 2H), 6.47 (dd, *J* = 17.0, 10.1 Hz, 1H), 6.28 (d, *J* = 17.0 Hz, 1H), 5.78 (d, *J* = 10.0 Hz, 1H), 2.30 (s, 3H). | 346.3 |
| **28** | ¹H NMR (400 MHz, DMSO-*d₆*) δ 10.89 (s, 1H), 9.11 (s, 1H), 7.77 (s, 1H), 7.67 (d, *J* = 8.3 Hz, 2H), 7.40 (d, *J* = 8.3 Hz, 2H), 6.63 (dd, *J* = 17.0, 10.1 Hz, 1H), 6.31 (d, *J* = 16.8 Hz, 1H), 5.82 - 5.74 (m, 1H), 5.51 (s, 2H), 2.25 (s, 3H). | 361.3 |
| **29** | ¹H NMR (400 MHz, DMSO-*d₆*) δ 10.42 (s, 1H), 8.57 (s, 1H), 8.35 (s, 1H), 7.75 (d, *J* = 2.8 Hz, 1H), 7.68 (d, *J =* 8.0 Hz, 2H), 7.38 (d, *J* = 8.3 Hz, 2H), 6.65 - 6.53 (m, 2H), 6.24 (d, *J* = 16.9 Hz, 1H), 5.69 (d, *J* = 10.6 Hz, 1H), 5.62 (s, 2H). | 346.4 |
| **30** | ¹H NMR (400 MHz, DMSO-*d₆*) δ 10.37 (s, 1H), 8.60 (s, 1H), 8.48 (s, 2H), 7.69 (d, *J =* 8.4 Hz, 2H), 7.48 (d, *J* = 7.7 Hz, 2H), 6.48 - 6.39 (m, 1H), 6.28 (d, *J* = 17.0 Hz, 1H), 5.78 (d, *J* = 12.1 Hz, 1H), 5.58 (s, 2H). | 347.3 |
| **31** | ¹H NMR (400 MHz, DMSO-*d₆*) δ 11.24 (s, 1H), 9.45 (s, 1H), 7.70 (d, *J* = 8.1 Hz, 2H), 7.46 (d, *J* = 8.2 Hz, 2H), 6.65 (dd, *J* = 17.1, 10.2 Hz, 1H), 6.39 (d, *J* = 15.7 Hz, 1H), 5.87 (d, *J* = 11.5 Hz, 1H), 5.74 (s, 2H), 2.53 (s, 3H). | 362.3 |
| **32** | ¹H NMR (400 MHz, Chloroform-*d*) δ 8.20 (s, 1H), 8.14 (s, 1H), 7.52 (d, *J* = 8.6 Hz, 1H), 7.47 (d, *J* = 8.1 Hz, 2H), 7.23 (d, *J* = 8.0 Hz, 2H), 6.91 (d, *J* = 9.8 Hz, 1H), 6.43 (d, *J* = 16.8 Hz, 1H), 6.28 (dd, *J* = 16.8, 10.1 Hz, 1H), 5.73 (d, *J* = 10.2 Hz, 1H), 5.48 (s, 2H), 2.53 (s, 3H). | 360.4 |
| **33** | ¹H NMR (400 MHz, DMSO-*d₆*) δ 10.15 (s, 1H), 8.17 (s, 1H), 7.66 (d, *J* = 8.1 Hz, 2H), 7.59 (d, *J* = 9.1 Hz, 1H), 7.45 (d, *J* = 9.2 Hz, 1H), 7.35 (d, *J* = 8.1 Hz, 2H), 6.43 (dd, *J* = 17.0, 10.3 Hz, 1H), 6.24 (d, *J* = 16.9 Hz, 1H), 5.73 (d, *J* = 9.8 Hz, 1H), 5.64 (s, 2H), 2.44 (s, 3H). | 360.3 |
| **34** | ¹H NMR (400 MHz, Chloroform-*d*) δ 8.68 (s, 1H), 8.17 (s, 1H), 7.91 (d, *J* = 8.8 Hz, 2H), 7.80 (d, *J* = 8.4 Hz, 2H), 7.73 (d, *J* = 8.8 Hz, 1H), 7.46 (s, 1H), 7.04 (d, *J =* 8.4 Hz, 1H), 6.49 (d, *J =* 16.4 Hz, 1H), 6.28 (dd, *J =* 16.7, 10.0 Hz, 1H), 5.84 (d, *J* = 10.0 Hz, 1H), | 332.3 |
| **35** | ¹H NMR (400 MHz, Chloroform-*d*) δ 8.79 (s, 1H), 8.52 (d, *J* = 10.0 Hz, 3H), 8.22 (s, 1H), 7.78 (d, *J* = 8.4 Hz, 2H), 7.50 (s, 1H), 6.53 (d, *J* = 17.2 Hz, 1H), 6.32 (dd, *J =* 16.8, 10.0 Hz, 1H), 5.88 (d, *J* = 10.4 Hz, 1H). | 333.3 |
| **36** | ¹H NMR (400 MHz, Chloroform-*d*) δ 8.58 (s, 1H), 8.29 (s, 1H), 7.95 (d, *J* = 8.4 Hz, 2H), 7.78 (d, *J* = 8.8 Hz, 2H), 7.57 (d, *J* = 4.0 Hz, 1H), 7.42 (s, 1H), 6.68 (d, *J* = 3.6 Hz, 1H), 6.50 (d, *J* = 16.4 Hz, 1H), 6.31 (dd, *J =* 16.4, 10.4 Hz, 1H), 5.83 (d, *J* = 10.4 Hz, 1H). | 332.4 |
| **37** | ¹H NMR (400 MHz, Chloroform-*d*) δ 9.42 (s, 1H), 8.46 (s, 1H), 8.17 (s, 1H), 8.10 (s, 1H), 7.55 (d, *J =* 8.2 Hz, 2H), 7.28 (d, *J =* 8.1 Hz, 2H), 6.44 (d, *J =* 16.8 Hz, 1H), 6.27 (dd, *J =* 16.8, 10.2 Hz, 1H), 5.71 (d, *J* = 10.1 Hz, 1H), 2.57 - 2.46 (m, 1H), 2.29 (s, 3H), 1.92 - 1.79 (m, 4H), 1.75 (d, J = 12.2 Hz, 2H), 1.40 (p, J = 13.1 Hz, 4H). | 360.5 |
| **38** | ¹H NMR (400 MHz, DMSO-*d₆*) δ 10.43 (s, 1H), 8.26 (s, 1H), 7.69 (d, *J* = 8.1 Hz, 2H), 7.55 (d, *J* = 8.8 Hz, 1H), 7.36 (d, *J* = 8.0 Hz, 2H), 7.29 (d, *J* = 8.8 Hz, 1H), 6.44 (dd, *J* = 16.9, 10.0 Hz, 1H), 6.27 (d, *J* = 16.9 Hz, 1H), 5.78 (d, *J* = 10.0 Hz, 1H), 5.70 (s, 2H). | 424.3 |
| **39** | ¹H NMR (400 MHz, DMSO-*d₆*) δ 10.00 (s, 1H), 7.61 (d, *J =* 8.3 Hz, 3H), 7.34 (dd, *J* = 16.6, 8.2 Hz, 3H), 7.23 (d, *J =* 8.5 Hz, 1H), 6.45 - 6.34 (m, 1H), 6.20 (d, *J* = 16.9 Hz, 1H), 5.69 (d, *J* = 10.0 Hz, 1H), 3.98 (t, *J* = 8.5 Hz, 2H), 3.11 (t, *J* = 8.5 Hz, 2H). | 333.4 |
| **40** | ¹H NMR (400 MHz, DMSO-*d₆*) δ 10.38 (s, 1H), 8.40 (d, *J* = 17.9 Hz, 2H), 7.93 (s, 1H), 7.67 (d, *J* = 7.9 Hz, 2H), 7.40 (d, *J* = 7.8 Hz, 2H), 6.48 - 6.40 (m, 1H), 6.27 (d, *J* = 16.8 Hz, 1H), 5.78 (d, *J* = 10.0 Hz, 1H), 5.55 (s, 2H). | 424.3 |
| **41** | ¹H NMR (400 MHz, Chloroform-*d*) δ 9.42 (s, 1H), 8.33 (s, 1H), 7.96 (d, *J* = 8.4 Hz, 2H), 7.78 (d, *J =* 8.8 Hz, 2H), 7.67 (s, 1H), 6.54 (d, *J =* 17.2 Hz, 1H), 6.34 (dd, *J* = 17.0, 10.2 Hz, 1H), 5.87 (d, *J* = 10.4 Hz, 1H), 2.41 (s, 3H). | 347.4 |
| **42** | ¹H NMR (400 MHz, Chloroform-*d*) *δ* 9.45 (s, 1H), 8.49 (s, 1H), 7.96 (d, *J* = 8.8 Hz, 2H), 7.79 (d, *J* = 8.4 Hz, 2H), 7.69 (s, 1H), 6.55 (d, *J* = 16.4 Hz, 1H), 6.35 (dd, *J* = 17.0, 10.2 Hz, 1H), 5.89 (d, *J* = 10.4 Hz, 1H), 2.42 (s, 3H). | 347.4 |
| **43** | ¹H NMR (400 MHz, Chloroform-*d*) δ 8.46 (s, 1H), 8.38 (s, 1H), 7.57 (d, *J* = 8.2 Hz, 2H), 7.41 (s, 1H), 7.30 (d, *J* = 8.0 Hz, 2H), 7.17 (s, 1H), 6.50 (d, *J* = 16.9 Hz, 1H), 6.31 (dd, *J* = 16.6, 10.5 Hz, 1H), 5.84 (d, *J* = 10.2 Hz, 1H), 5.51 (s, 2H). | 380.4 |
| **44** | ¹H NMR (400 MHz, Chloroform-*d*) δ 8.39 (s, 1H), 8.20 (s, 1H), 7.92 (s, 1H), 7.29 (d, *J* = 8.2 Hz, 2H), 7.10 (d, *J =* 8.1 Hz, 2H), 6.93 (s, 1H), 6.44 (d, *J =* 16.7 Hz, 1H), 6.29 (dd, *J* = 16.8, 10.1 Hz, 1H), 5.72 (d, *J* = 10.2 Hz, 1H), 5.35 (s, 2H), 2.23 (s, 3H), 1.27 (s, 9H). | 348.5 |
| **45** | ¹H NMR (400 MHz, Chloroform-*d*) δ 8.40 (s, 2H), 8.20 (s, 1H), 7.13 (d, *J* = 8.5 Hz, 2H), 7.07 (d, *J* = 8.3 Hz, 2H), 6.90 (s, 1H), 6.42 (d, *J* = 16.8 Hz, 1H), 6.31 (dd, *J* = 16.9, 10.0 Hz, 1H), 5.69 (d, *J* = 11.1 Hz, 1H), 5.34 (s, 2H), 2.21 (s, 3H). | 376.4 |
| **46** | ¹H NMR (400 MHz, Chloroform-*d*) δ 9.45 (s, 1H), 8.32 (s, 1H), 7.97 (d, J = 8.4 Hz, 2H), 7.87 (d, J = 3.6 Hz, 1H), 7.81 (d, J = 8.4 Hz, 2H), 6.76 (d, J = 3.6 Hz, 1H), 6.54 (d, J = 17.2 Hz, 1H), 6.35 (dd, J = 17.0, 10.2 Hz, 1H), 5.88 (d, J = 10.4 Hz, 1H). | 333.3 |
| **47** | ¹H NMR (400 MHz, Chloroform-*d*) *δ* 8.63 (s, 1H), 8.29 (s, 1H), 7.89 (s, 1H), 7.58 (d, *J* = 8.4 Hz, 2H), 7.35 (d, *J* = 8.0 Hz, 2H), 7.27 - 7.26 (m, 1H), 6.48 (d, *J* = 15.6 Hz, 1H), 6.35 (dd, *J* = 16.6, 10.2 Hz, 1H), 5.80 (d, *J* = 10.0 Hz, 1H), 2.96 (q, *J* = 7.0 Hz, 1H), 2.34 (s, 3H), 1.27 (t, *J* = 8.2 Hz, 6H). | 320.4 |
| **48** | ¹H NMR (400 MHz, DMSO-*d₆*) δ 10.37 (s, 1H), 8.53 (s, 2H), 6.43 (dd, *J* = 16.8, 9.6 Hz, 1H), 6.26 (d, *J =* 15.3 Hz, 1H), 5.76 (d, *J* = 9.8 Hz, 1H), 4.33 (t, *J* = 7.2 Hz, 2H), 2.44 (s, 3H), 1.69 (dd, *J* = 14.1, 6.8 Hz, 2H), 1.49 - 1.37 (m, 1H), 0.87 (d, *J* = 6.6 Hz, 6H). | 273.4 |
| **49** | ¹H NMR (400 MHz, Chloroform-*d*) δ 8.60 (s, 1H), 8.39 (s, 1H), 7.49 (s, 1H), 7.32 (d, *J* = 8.5 Hz, 2H), 7.12 (d, *J* = 8.0 Hz, 2H), 6.50 (d, *J* = 16.7 Hz, 1H), 6.30 (dd, *J* = 16.8, 10.2 Hz, 1H), 5.84 (d, *J* = 10.0 Hz, 1H), 5.61 (s, 2H), 2.55 (s, 3H). | 377.4 |
| **50** | ¹H NMR (400 MHz, Chloroform-*d*) δ 8.69 (s, 1H), 8.33 (s, 1H), 8.03 - 7.99 (m, 2H), 7.90 (s, 1H), 7.63 (t, *J =* 8.0 Hz, 1H), 7.56 (d, *J =* 7.2 Hz, 1H), 7.34 (s, 1H), 6.50 (d, *J* = 16.4 Hz, 1H), 6.36 (dd, *J* = 17.0, 10.2 Hz, 1H), 5.83 (d, *J* = 10.4 Hz, 1H), 2.37 (s, 3H). | 346.4 |
| **51** | ¹H NMR (400 MHz, Chloroform-*d*) δ 8.73 (s, 1H), 8.44 (d, *J* = 2.0 Hz, 1H), 8.02 (d, *J* = 8.4 Hz, 2H), 7.59 (s, 1H), 7.35 (d, *J* = 8.4 Hz, 2H), 6.51 (d, *J* = 16.7 Hz, 1H), 6.32 (dd, *J =* 16.8, 10.4 Hz, 1H), 5.85 (d, *J =* 10.4 Hz, 1H), 2.97 - 2.92 (m, 1H), 2.62 (s, 3H), 1.28 (d, *J* = 7.2 Hz, 6H). | 321.4 |
| **52** | ¹H NMR (400 MHz, Chloroform-*d*) δ 8.76 (s, 1H), 8.63 (s, 1H), 8.56 (d, *J* = 8.0 Hz, 1H), 8.45 (d, *J* = 2.0 Hz, 1H), 7.60 (t, *J* = 7.5 Hz, 1H), 7.54 - 7.48 (m, 2H), 6.52 (d, *J* = 17.2 Hz, 1H), 6.32 (dd, *J* = 16.8, 10.0 Hz, 1H), 5.87 (d, *J* = 10.8 Hz, 1H), 2.64 (s, 3H). | 347.4 |
| **53** | ¹H NMR (400 MHz, Chloroform-*d*) δ ¹H NMR (400 MHz, Chloroform-J) δ 8.62 (s, 1H), 8.37 (s, 1H), 8.21 (s, 1H), 7.38 (d, *J* = 7.9 Hz, 1H), 6.90 (s, 1H), 6.76 (s, 1H), 6.65 (d, *J* = 7.9 Hz, 1H), 6.41 (d, *J* = 16.3 Hz, 1H), 6.29 (dd, *J* = 16.9, 10.0 Hz, 1H), 5.65 (d, *J* = 10.7 Hz, 1H), 5.34 (s, 2H), 3.72 (s, 3H), 2.19 (s, 3H). | 390.5 |
| **54** | ¹H NMR (400 MHz, Chloroform-*d*) *δ* 8.65 (s, 1H), 8.30 (s, 1H), 7.86 (s, 1H), 7.67 (d, *J* = 8.8 Hz, 2H), 7.46 (d, *J* = 8.8 Hz, 2H), 7.27 - 7.25 (m, 1H), 6.49 (d, *J* = 16.4 Hz, 1H), 6.38 - 6.32 (m, 1H), 5.82 (d, *J* = 9.6 Hz, 1H), 2.35 (s, 3H). | 312.4 |
| **55** | ¹H NMR (400 MHz, Chloroform-J) *δ* 8.57 (s, 1H), 8.30 (s, 1H), 7.83 (s, 1H), 7.51 (d, *J* = 8.8 Hz, 2H), 7.40 (t, *J* = 7.4 Hz, 1H), 7.32 (d, *J* = 7.6 Hz, 1H), 6.96 (s, 1H), 6.48 (d, *J* = 16.8 Hz, 1H), 6.35 (dd, *J* = 16.8, 10.0 Hz, 1H), 5.80 (d, *J* = 10.4 Hz, 1H), 5.49 (s, 2H), 2.29 (s, 3H). | 360.4 |
| **56** | ¹H NMR (400 MHz, Chloroform-*d*) *δ* 8.63 (s, 1H), 8.41 (d, *J* = 2.0 Hz, 1H), 7.57 (d, *J* = 14.4 Hz, 2H), 7.50 (d, *J* = 7.8 Hz, 1H), 7.45 (d, *J* = 7.6 Hz, 1H), 7.39 (t, *J* = 7.8 Hz, 1H), 6.49 (d, *J* = 16.8 Hz, 1H), 6.30 (dd, *J* = 16.8, 10.0 Hz, 1H), 5.83 (d, *J* = 10.0 Hz, 1H), 5.66 (s, 2H), 2.55 (s, 3H). | 361.4 |
| **57** | ¹H NMR (400 MHz, Chloroform-*d*) *δ* 8.50 (s, 1H), 8.26 (s, 1H), 7.81 (s, 1H), 7.10 (s, 1H), 6.47 (d, *J* = 17.2 Hz, 1H), 6.35 (dd, *J* = 16.8, 10.0 Hz, 1H), 5.79 (d, *J* = 10.5 Hz, 1H), 4.72 (t, *J* = 11.6 Hz, 1H), 2.29 (s, 3H), 2.06 (d, *J* = 16.0 Hz, 2H), 1.77 (d, *J* = 13.6 Hz, 2H), 1.70 - 1.53 (m, 4H), 1.25 (s, 2H). | 284.4 |
| **58** | ¹H NMR (400 MHz, DMSO-*d₆*) δ 10.35 (s, 1H), 8.42 (d, *J* = 16.0 Hz, 2H), 8.03 (d, *J* = 9.0 Hz, 2H), 7.78 (s, 1H), 7.51 (d, *J* = 8.6 Hz, 2H), 6.45 (dd, *J* = 17.0, 10.0 Hz, 1H), 6.26 (d, *J =* 16.9 Hz, 1H), 5.76 (d, *J =* 10.3 Hz, 1H), 2.28 (s, 3H). | 362.3 |
| **59** | ¹H NMR (400 MHz, DMSO-*d₆*) δ 10.37 (s, 1H), 8.45 (s, 2H), 7.96 (s, 1H), 7.83 (d, *J* = 9.1 Hz, 2H), 7.71 (d, *J* = 8.2 Hz, 1H), 6.53 - 6.36 (m, 1H), 6.27 (d, *J* = 16.7 Hz, 1H), 5.77 (d, *J* = 10.3 Hz, 1H), 3.96 (s, 3H), 2.30 (s, 3H). | 376.4 |
| **60** | ¹H NMR (400 MHz, Chloroform-*d*) *δ* 8.60 (s, 1H), 8.39 (s, 1H), 7.65 (s, 1H), 7.28 (d, *J* = 8.0 Hz, 1H), 7.28 - 7.19 (m, 3H), 6.47 (d, *J* = 16.4 Hz, 1H), 6.29 (dd, *J* = 17.0, 10.6 Hz, 1H), 5.80 (d, *J* = 10.4 Hz, 1H), 5.57 (s, 2H), 2.52 (s, 3H), 1.24 (s, 9H). | 349.4 |
| **61** | ¹H NMR (400 MHz, Chloroform-*d*) *δ* 8.66 (s, 1H), 8.43 (s, 1H), 7.63 (s, 1H), 7.44 (d, *J* = 8.0 Hz, 1H), 6.99 (s, 1H), 6.88 (d, *J* = 7.6 Hz, 1H), 6.49 (d, *J* = 16.8 Hz, 1H), 6.30 (dd, *J* = 16.8, 10.0 Hz, 1H), 5.83 (d, *J* = 10.0 Hz, 1H), 5.62 (s, 2H), 3.81 (s, 3H), 2.54 (s, 3H). | 391.4 |
| **62** | ¹H NMR (400 MHz, DMSO-*d₆*) *δ* 10.45 (s, 1H), 8.61 (d, *J* = 9.6 Hz, 2H), 7.54 (s, 1H), 7.19 (s, 1H), 6.45 (dd, *J* = 16.8, 10.0 Hz, 1H), 6.28 (d, *J* = 17.2 Hz, 1H), 5.82 - 5.78 (m, 3H), 2.48 (d, *J* = 1.2 Hz, 3H). | 367.4 |
| **63** | ¹H NMR (400 MHz, DMSO-*d₆*) *δ* 10.24 (s, 1H), 8.35 (d, *J* = 18.0 Hz, 2H), 7.52 (s, 1H), 7.40 (s, 1H), 7.15 (s, 1H), 6.45 (dd, *J =* 17.2, 10.0 Hz, 1H), 6.26 (d, *J =* 16.8 Hz, 1H), 5.75 (d, *J* = 10.4 Hz, 1H), 5.62 (s, 2H), 2.21 (s, 3H). | 366.3 |
| **64** | ¹H NMR (400 MHz, Chloroform-J) *δ* 8.73 (s, 1H), 8.47 (d, *J* = 2.4 Hz, 1H), 8.11 - 8.07 (m, 2H), 7.66 (d, *J* = 8.8 Hz, 1H), 7.50 (s, 1H), 6.53 (d, *J* = 16.8 Hz, 1H), 6.33 (dd, *J* = 17.0, 10.2 Hz, 1H), 5.88 (d, *J* = 10.4 Hz, 1H), 4.03 (s, 3H), 2.64 (s, 3H). | 377.3 |
| **65** | ¹H NMR (400 MHz, Chloroform-*d*) δ 8.74 (s, 1H), 8.44 (s, 1H), 8.23 (d, *J =* 8.4 Hz, 2H), 7.46 (d, *J* = 9.2 Hz, 3H), 6.52 (d, *J* = 16.8 Hz, 1H), 6.35 - 6.29 (m, 1H), 5.87 (d, *J* = 10.4 Hz, 1H), 2.63 (s, 3H). | 313.3 |
| **66** | ¹H NMR (400 MHz, Chloroform-*d*) δ 8.74 (s, 1H), 8.44 (d, *J* = 2.0 Hz, 1H), 8.30 (d, *J* = 8.8 Hz, 2H), 7.51 (s, 1H), 7.35 (d, *J* = 8.8 Hz, 2H), 6.52 (d, *J* = 16.8 Hz, 1H), 6.32 (dd, *J* = 16.7, 10.4 Hz, 1H), 5.87 (d, *J* = 10.4 Hz, 1H), 2.63 (s, 3H). | 363.3 |
| **67** | ¹H NMR (400 MHz, Chloroform-*d*) δ 8.64 (s, 1H), 8.41 (d, *J*= 2.4 Hz, 1H), 7.57 (s, 1H), 7.42 (d, *J* = 8.0 Hz, 2H), 7.37 (d, *J* = 8.0 Hz, 2H), 6.73 - 6.44 (m, 2H), 6.31 (dd, *J* = 16.8, 10.0 Hz, 1H), 5.84 (d, *J* = 10.8 Hz, 1H), 5.66 (s, 2H), 2.55 (s, 3H). | 343.4 |
| **68** | ¹H NMR (400 MHz, Chloroform-*d*) δ 8.66 (s, 1H), 8.37 (s, 1H), 7.52 (s, 1H), 6.55 (d, *J* = 5.5 Hz, 1H), 6.48 (s, 1H), 6.31 (dd, *J* = 16.8, 10.2 Hz, 1H), 5.85 (d, *J =* 10.3 Hz, 1H), 3.14 (dd, *J* = 18.9, 4.7 Hz, 1H), 2.81 - 2.67 (m, 1H), 2.55 (s, 3H), 2.46-2.42 (m, 1H), 2.23 (d, *J* = 6.6 Hz, 1H), 1.88 - 1.77 (m, 1H), 1.26 (t, *J* = 7.1 Hz, 2H). | 351.4 |
| **69** | ¹H NMR (400 MHz, DMSO-*d₆*) δ 10.59 (s, 1H), 8.73 (d, *J* = 2.8 Hz, 2H), 8.45 (d, *J* = 8.4 Hz, 2H), 7.73 (d, *J* = 8.0 Hz, 2H), 7.07 (s, 1H), 6.48 (dd, *J* = 17.2, 10.0 Hz, 1H), 6.32 (d, *J* = 16.8 Hz, 1H), 5.83 (d, *J* = 10.4 Hz, 1H), 2.59 (s, 3H). | 329.3 |
| **70** | ¹H NMR (400 MHz, DMSO-*d₆*) δ 10.57 (s, 1H), 8.72 (dd, *J* = 11.4, 2.2 Hz, 2H), 8.26 (dd, *J =* 9.1, 4.9 Hz, 2H), 7.39 (t, *J* = 8.9 Hz, 2H), 6.49 (dd, *J* = 17.0, 10.1 Hz, 1H), 6.32 (dd,*J* = 17.0, 1.8 Hz, 1H), 5.87 - 5.79 (m, 1H), 2.59 (s, 3H). | 297.3 |
| **71** | ¹H NMR (400 MHz, Chloroform-*d*) δ 8.49 (s, 1H), 8.24 (s, 1H), 7.66 (s, 1H), 7.42 (d, *J* = 8.0 Hz, 2H), 7.26 - 7.23 (m, 2H), 6.95 (s, 1H), 6.74 - 6.46 (m, 2H), 6.33 (dd, *J* = 16.8, 10.0 Hz, 1H), 5.80 (d, *J* = 10.4 Hz, 1H), 5.46 (s, 2H), 2.28 (s, 3H). | 342.4 |
| **72** | ¹H NMR (400 MHz, DMSO-*d₆*) δ 10.65 (s, 1H), 8.77 (s, 2H), 8.70 (s, 1H), 8.50 (d, *J* = 8.8 Hz, 1H), 8.02 (d, *J* = 8.8 Hz, 1H), 6.48 (dd, *J* = 17.2, 10.0 Hz, 1H), 6.32 (d, *J* = 16.8 Hz, 1H), 5.84 (d, *J* = 10.0 Hz, 1H), 2.60 (s, 3H). | 381.3 |
| **73** | ¹H NMR (400 MHz, Chloroform-*d*) δ 8.76 (s, 1H), 8.63 (s, 1H), 8.56 (d, *J* = 8.0 Hz, 1H), 8.45 (d, *J* = 2.0 Hz, 1H), 7.60 (t, *J* = 7.5 Hz, 1H), 7.54 - 7.48 (m, 2H), 6.52 (d, *J* = 17.2 Hz, 1H), 6.32 (dd, *J* = 16.8, 10.0 Hz, 1H), 5.87 (d, *J* = 10.8 Hz, 1H), 3.87(s, 3H), 2.06 (s, 3H). | 309.3 |
| **74** | ¹H NMR (400 MHz, DMSO-*d₆*) δ 10.61 (s, 1H), 8.74 (s, 2H), 8.40 - 8.35 (m, 2H), 7.82 (d, *J* = 8.8 Hz, 1H), 6.49 - 6.44 (m, 1H), 6.32 (d, *J* = 16.8 Hz, 1H), 5.83 (d, *J* = 10.0 Hz, 1H), 2.59 (s, 3H), 2.53 (s, 3H). | 361.3 |
| **75** | ¹H NMR (400 MHz, DMSO-*d₆*) δ 10.52 (s, 1H), 8.72 (s, 1H), 8.58 (s, 1H), 7.46 - 7.37 (m, 4H), 6.49 (dd, *J* = 16.8, 10.0 Hz, 1H), 6.32 (d, *J* = 16.8 Hz, 1H), 5.83 (d, *J* = 10.0 Hz, 1H), 2.59 (s, 3H), 2.10 (s, 3H). | 293.1 |
| **76** | ¹H NMR (400 MHz, Chloroform-*d*) δ 8.79 (s, 1H), 8.46 (s, 1H), 8.05 (d, *J =* 8.8 Hz, 2H), 7.46 (s, 1H), 6.69 (t, *J* = 9.5 Hz, 1H), 6.54 (d, *J* = 17.2 Hz, 1H), 6.33 (dd, *J* = 16.8, 10.3 Hz, 1H), 5.89 (d, *J* = 9.7 Hz, 1H), 2.64 (s, 3H). | 315.2 |
| **77** | ¹H NMR (400 MHz, Chloroform-*d*) δ 9.81 (s, 1H), 8.92 (d, *J* = 8.8 Hz, 1H), 8.81 (s, 1H), 8.49 (s, 1H), 7.81 (d, *J* = 8.8 Hz, 1H), 7.49 (s, 1H), 6.55 (d, *J =* 16.6 Hz, 1H), 6.34 (dd, *J* = 16.9, 9.8 Hz, 1H), 5.90 (d, *J* = 10.4 Hz, 1H), 2.66 (s, 3H) | 348.3 |
| **78** | ¹H NMR (400 MHz, DMSO-*d₆*) δ 10.55 (s, 1H), 8.71 (s, 1H), 8.67 (s, 1H), 8.07 (d, *J* = 7.3 Hz, 2H), 7.09 (d, *J* = 7.2 Hz, 2H), 6.48 (dd, *J* = 17.0, 9.2 Hz, 1H), 6.32 (d, *J* = 16.9 Hz, 1H), 5.83 (d, *J* = 9.9 Hz, 1H), 3.79 (d, *J* = 5.3 Hz, 2H), 2.57 (s, 3H), 2.03 (dd, *J* = 16.6, 10.1 Hz, 1H), 1.00 (d, *J* = 5.2 Hz, 6H). | 351.1 |
| **79** | ¹H NMR (400 MHz, DMSO-*d₆*) δ 10.56 (s, 1H), 8.71 (s, 1H), 8.69 (s, 1H), 8.12 (d, *J* = 7.7 Hz, 2H), 7.55 (d, *J* = 7.8 Hz, 2H), 6.49 (dd, *J* = 16.8, 10.2 Hz, 1H), 6.32 (d, *J* = 16.9 Hz, 1H), 5.83 (d, *J* = 9.9 Hz, 1H), 2.58 (s, 3H), 1.33 (s, 9H). | 335.1 |
| **80** | ¹H NMR (400 MHz, DMSO-*d₆*) δ 10.56 (s, 1H), 8.72 (s, 1H), 8.68 (s, 1H), 8.11 (d, *J* = 7.2 Hz, 2H), 7.36 (d, *J* = 7.3 Hz, 2H), 6.48 (dd, *J* = 16.9, 10.2 Hz, 1H), 6.32 (d, *J* = 16.9 Hz, 1H), 5.83 (d, *J* = 9.9 Hz, 1H), 2.70 - 2.61 (m, 1H), 2.58 (s, 3H), 1.65 - 1.52 (m, 2H), 1.23 (d, *J* = 5.4 Hz, 3H), 0.80 (t, *J* = 6.5 Hz, 3H). | 335.1 |
| **81** | ¹H NMR (400 MHz, Chloroform-*d*) *δ* 8.74 (s, 1H), 8.38 (s, 1H), 7.62 (s, 1H), 7.48 - 7.44 (m, 2H), 7.12 - 7.10 (m, 2H), 6.53 (d, *J* = 16.8 Hz, 1H), 6.35 - 6.28 (m, 1H), 5.85 (d, *J* = 10.0 Hz, 1H), 3.78 (s, 3H), 2.66 (s, 3H). | 309.1 |
| **82** | ¹H NMR (400 MHz, Chloroform-*d*) *δ* 8.72 (s, 1H), 8.32 (s, 1H), 7.84 (d, *J* = 7.6 Hz, 1H), 7.68 - 7.66 (m, 1H), 7.60 - 7.59 (m, 1H), 7.52 (d, *J* = 7.2 Hz, 1H), 7.42 (s, 1H), 6.48 (d, *J* = 16.8 Hz, 1H), 6.30 - 6.24 (m, 1H), 5.82 (d, *J* = 10.4 Hz, 1H), 2.60 (s, 3H). | 347.1 |
| **83** | ¹H NMR (400 MHz, Chloroform-*d*) *δ* 8.71 (s, 1H), 8.32 (s, 1H), 7.70 (d, *J =* 6.9 Hz, 1H), 7.43 (d, *J =* 12.9 Hz, 3H), 7.30 (d, *J =* 7.4 Hz, 1H), 6.45 (d, *J =* 16.7 Hz, 1H), 6.29 - 6.18 (m, 1H), 5.79 (d, *J* = 9.7 Hz, 1H), 2.59 (s, 3H). | 359.0 |
| **84** | 1H NMR (400 MHz, Chloroform-*d*) δ 8.75 (d, J = 2.3 Hz, 1H), 8.42 (d, J = 2.4 Hz, 1H), 7.81 (s, 1H), 7.50 (d, J = 2.2 Hz, 2H), 6.51 (d, J = 16.8 Hz, 1H), 6.38 (t, J = 2.2 Hz, 1H), 6.32 (dd, J = 16.9, 10.2 Hz, 1H), 5.85 (d, J = 10.3 Hz, 1H), 3.88 (s, 6H), 2.63 (s, 3H). | 339.07 |
| **85** | ¹H NMR (400 MHz, Chloroform-*d*) δ 8.48 (d, J = 8.5 Hz, 2H), 8.42 (d, J = 2.4 Hz, 1H), 8.05 (d, J = 2.4 Hz, 1H), 7.75 (d, J = 8.7 Hz, 2H), 6.61 (dd, J = 16.5, 9.9 Hz, 1H), 6.45 (s, 1H), 6.24 (d, J = 16.5 Hz, 1H), 6.01 (d, J = 9.9 Hz, 1H), 2.66 (s, 3H). | 383.0 |
| **86** | ¹H NMR (400 MHz, Chloroform-*d*) δ 8.51 (d, *J* = 8.5 Hz, 2H), 8.48 (d, *J* = 2.3 Hz, 1H), 7.89 (d, *J* = 2.1 Hz, 1H), 7.77 (d, *J* = 8.6 Hz, 2H), 6.45 (dd, *J* = 16.7, 1.8 Hz, 1H), 6.04 (d, *J* = 9.8 Hz, 1H), 5.59 (d, *J* = 9.8 Hz, 1H), 3.45 (s, 3H), 2.67 (s, 3H). | 361.0 |
| **87** | ¹H NMR (400 MHz, Chloroform-*d*) δ 8.69 (d, *J* = 2.0 Hz, 1H), 8.37 (d, *J* = 2.4 Hz, 1H), 8.04 (s, 1H), 7.95 (d, *J* = 8.8 Hz, 1H), 7.61 (d, *J* = 92 Hz, 2H), 6.57 - 6.53 (m, 2H), 6.34 (dd, *J* = 16.6, 10.2 Hz, 1H), 5.90 (d, *J* = 10.0 Hz, 1H), 2.55 (s, 3H). | 363.0 |
| **88** | ¹H NMR (400 MHz, Chloroform-*d*) δ 8.77 (s, 1H), 8.65 (d, *J* = 2.3 Hz, 1H), 8.43 (t, *J* = 2.6 Hz, 3H), 8.40 (d, *J* = 2.3 Hz, 2H), 6.39-6.34 (m, 1H), 6.02 (d, *J =* 16.8 Hz, 1H), 2.64 (s, 3H), 1.96 (d, *J* = 1.4 Hz, 3H). | 361.0 |
| **89** | ¹H NMR (400 MHz, DMSO-*d₆*) δ 10.69 (s, 1H), 8.80 - 8.78 (m, 2H), 7.71 (s, 2H), 6.50 (dd, *J =* 17.2, 10.0 Hz, 1H), 6.34 (d, *J =* 17.2 Hz, 1H), 5.86 (d, *J* = 10.4 Hz, 1H), 2.60 (s, 3H). | 353.0 |
| **90** | ¹H NMR (400 MHz, Chloroform-*d*) δ 8.46 (d, *J =* 2.3 Hz, 1H), 8.37 - 8.30 (m, 2H), 7.89 (d, *J* = 2.0 Hz, 1H), 7.38 (d, *J* = 8.8 Hz, 2H), 6.44 (d, *J* = 18.3 Hz, 1H), 6.03 (s, 1H), 5.58 (d, *J* = 10.6 Hz, 1H), 3.45 (s, 3H), 2.66 (s, 3H). | 377.0 |
| **91** | ¹H NMR (400 MHz, Chloroform-*d*) δ 8.38 (s, 1H), 8.18 (d, *J* = 8.5 Hz, 2H), 7.81 (s, 1H), 7.42 (d, *J* = 8.5 Hz, 2H), 6.37 (d, *J* = 16.7 Hz, 1H), 5.94 (dd, *J* = 16.7, 10.3 Hz, 1H), 5.51 (d, *J* = 10.3 Hz, 1H), 3.38 (s, 3H), 2.59 (s, 3H). | 327.0 |
| **92** | ¹H NMR (400 MHz, Chloroform-*d*) δ 9.83 (d, *J* = 2.3 Hz, 1H), 8.98 (dd, *J* = 8.6, 2.3 Hz, 1H), 8.53 (d, *J* = 2.2 Hz, 1H), 7.94 (d, *J* = 1.8 Hz, 1H), 7.85 (d, *J* = 8.6 Hz, 1H), 6.48 (d, *J* = 15.2 Hz, 1H), 6.11- 5.96 (m, 1H), 5.63 (d, *J* = 9.6 Hz, 1H), 3.48 (s, 3H), 2.71 (s, 3H). | 362.0 |
| **93** | ¹H NMR (400 MHz, DMSO-*d₆*) δ 10.61 (s, 1H), 9.07 (s, 1H), 8.83 - 8.74 (m, 3H), 7.42 (dd, *J* = 8.8, 3.2 Hz, 1H), 6.50 (dd, *J* = 17.0, 10.2 Hz, 1H), 6.34 (dd, *J* = 16.8, 2.0 Hz, 1H), 5.85 (dd, *J* = 10.0, 1.6 Hz, 1H), 2.62 (s, 3H). | 298.1 |
| **94** | ¹H NMR (400 MHz, DMSO-*d₆*) δ 10.61 (s, 1H), 9.49 (s, 1H), 8.77 (dd, *J* = 9.2, 2.0 Hz, 2H), 8.64 - 8.61 (m, 1H), 8.51 (d, *J* = 4.4 Hz, 1H), 7.60 (dd, *J* = 8.4, 4.8 Hz, 1H), 6.50 (dd, *J* = 16.8, 10.0 Hz, 1H), 6.34 (dd, *J* = 17.0, 1.8 Hz, 1H), 5.85 (dd, *J* = 10.0, 1.6 Hz, 1H), 2.62 (s, 3H). | 280.1 |
| **95** | ¹H NMR (400 MHz, Chloroform-*d*) δ 9.91 (s, 2H), 8.77 (s, 1H), 8.43 (d, *J* = 2.3 Hz, 1H), 7.39 (s, 1H), 6.47 (d, *J* = 16.8 Hz, 1H), 6.26 (dd, *J* = 16.8, 10.2 Hz, 1H), 5.83 (d, *J* = 10.3 Hz, 1H), 2.60 (s, 3H). | 349.0 |
| **96** | ¹H NMR (400 MHz, DMSO-*d₆*) δ 10.62 (s, 1H), 8.76 (dd, *J* = 6.2, 2.2 Hz, 2H), 8.60 (d, *J* = 2.4 Hz, 1H), 8.34 (dd, *J* = 9.0, 2.6 Hz, 1H), 7.82 (d, *J* = 8.8 Hz, 1H), 6.50 (dd, *J* = 17.0, 10.2 Hz, 1H), 6.34 (dd, *J* = 17.0, 1.8 Hz, 1H), 5.85 (dd, *J =* 10.0, 2.0 Hz, 1H), 2.61 (s, 3H). | 347.0 |
| **97** | ¹H NMR (400 MHz, Chloroform-*d*) δ 8.83 (s, 1H), 8.77 (s, 1H), 8.56 (d, *J =* 8.8 Hz, 1H), 8.48 (d, *J* = 2.4 Hz, 1H), 8.01 (dd, *J =* 8.7, 2.2 Hz, 1H), 7.56 (s, 1H), 6.47 (d, *J* = 16.8 Hz, 1H), 6.26 (dd, *J* = 16.9, 10.2 Hz, 1H), 5.81 (d, *J* = 10.4 Hz, 1H), 2.62 (s, 3H). | 348.0 |
| **98** | ¹H NMR (400 MHz, Chloroform-*d*) δ 8.71 (s, 1H), 8.42 (s, 1H), 7.92 (d, *J* = 8.8 Hz, 2H), 7.66 (s, 1H), 6.94 (d, *J* = 8.9 Hz, 2H), 6.52 (d, *J* = 16.8 Hz, 1H), 6.39 - 6.27 (m, 1H), 5.86 (d, *J* = 10.4 Hz, 1H), 2.63 (s, 3H). | 295.07 |
| **99** | ¹H NMR (400 MHz, DMSO-*d₆*) δ 10.67 (s, 1H), 9.50 (d, *J* = 2.1 Hz, 1H), 8.79 (s, 2H), 8.77 (s, 1H), 6.53 (d, *J* = 10.0 Hz, 1H), 6.35 (dd, *J* = 17.0, 1.8 Hz, 1H), 5.90 - 5.81 (m, 1H), 2.63 (s, 3H), 2.58 (s, 3H). | 362.0 |
| **100** | ¹H NMR (400 MHz, DMSO-*d₆*) δ 10.70 (s, 1H), 8.85 (d, *J*= 5.6 Hz, 1H), 8.81 (dd, *J* = 5.8, 2.3 Hz, 2H), 8.80 (d, *J* = 2.0 Hz, 1H), 6.51 (dd, *J* = 17.0, 10.1 Hz, 1H), 6.35 (dd, *J* = 17.0, 1.9 Hz, 1H), 5.87 (dd, *J =* 10.1, 1.9 Hz, 1H), 2.64 (s, 3H). | 348.0 |
| **101** | ¹H NMR (400 MHz, Chloroform-*d*) δ 8.74 (s, 1H), 8.61 (s, 1H), 8.50 (s, 1H), 8.46 (d, *J* = 2.4 Hz, 1H), 7.41 (s, 1H), 6.53 (d, *J* = 16.4 Hz, 1H), 6.32 (dd, *J* = 16.8, 10.0 Hz, 1H), 5.88 (d, *J* = 10.4 Hz, 1H), 2.62 (s, 3H). | 337.1 |
| **102** | ¹H NMR (400 MHz, DMSO-*d₆*) δ 10.50 (s, 1H), 8.65 (dd, *J* = 18.8, 2.4 Hz, 2H), 7.70 (d, *J* = 8.8 Hz, 2H), 6.70 (d, *J* = 8.8 Hz, 2H), 6.49 (dd, *J* = 17.0, 10.2 Hz, 1H), 6.32 (dd, *J* = 16.8, 2.0 Hz, 1H), 5.83 (dd, *J* = 10.0, 2.0 Hz, 1H), 5.21 (s, 2H), 2.56 (s, 3H). | 294.1 |
| **103** | ¹H NMR (400 MHz, Chloroform-*d*) δ 9.34 (d, *J* = 2.6 Hz, 1H), 8.75 (dd, *J* = 8.6, 2.8 Hz, 2H), 8.44 (d, *J* = 2.3 Hz, 1H), 7.62 (s, 1H), 7.16 (d, *J* = 8.9 Hz, 1H), 6.54 (d, *J* = 16.8 Hz, 1H), 6.34 (dd, *J* = 16.8, 10.2 Hz, 1H), 5.88 (d, *J* = 10.3 Hz, 1H), 2.63 (s, 3H). | 364.08 |
| **104** | ¹H NMR (400 MHz, Chloroform-J) δ 8.78 (s, 1H), 8.59 (d, *J* = 8.9 Hz, 2H), 8.48 (s, 1H), 7.78 (d, *J* = 8.9 Hz, 2H), 7.47 (s, 1H), 6.54 (d, *J* = 16.8 Hz, 1H), 6.34 (dd, *J* = 16.8, 10.2 Hz, 1H), 5.90 (d, *J* = 11.2 Hz, 1H), 2.65 (s, 3H). | 304.09 |
| **105** | ¹H NMR (400 MHz, DMSO-*d₆*) δ 10.53 (s, 1H), 8.73 (d, *J* = 2.3 Hz, 1H), 8.61 (d, *J* = 2.3 Hz, 1H), 7.80 (s, 1H), 7.76 (s, 1H), 6.49 (dd, *J* = 16.9, 10.1 Hz, 1H), 6.33 (dd, *J =* 17.0, 2.0 Hz, 1H), 5.83 (dd, *J* = 10.0, 2.0 Hz, 1H), 2.59 (s, 3H), 2.15 (s, 3H). | 361.0 |

### <Experimental Example>

### Experimental Example 1. TEAD response element (TRE) reporter assay

MCF-7 cell lines injected with TEAD reporter genes expressing firefly luciferase upon activation of TEAD response element (TRE) were obtained from BPS bioscience (#60618). The TEAD reporter genes were injected to stable expression in the MCF-7 cell lines.

TEAD reporter genes expressing firefly luciferase upon activation of TEAD response element (TRE) were injected with TEAD reporter genes to ensure stable expression of the TEAD reporter genes,. Here, the TEAD reporter genes expresses firefly luciferase upon activation of TEAD response element (TRE). The cells were cultured in a cell-only culture medium (BPS bioscience) to which 10 µg/mL insulin (Sigma) was added at 37°C and 5% CO₂.

In 24 hours after dispensing said cells into a 96-well plate, each of the compounds of the examples or a vehicle solvent (final 1% DMSO) was treated by being diluted with a serum-free culture medium so that the final concentration became 0.2 µM. In 24 hours after said treatment, a luciferase assay working solution (ONE-Step Luciferase assay reagent; BPS bioscience) containing a luciferase substrate was treated in each well at 100 µL, and then left in a shaker for 15 minutes to be well mixed. Then, a degree of luminescence generated by luciferase expressed according to TEAD activation was measured by a microplate reader (TECAN). A relative activity of luciferase (%) was calculated with a luminescence level of the vehicle group (solvent-treated control group) as 100%, and the results are shown in table 8 below.

**[Table 8]**

| **Compound No.** | **% of inhibition of TRE luciferase at 0.2 µM** | **Compound No.** | **% of inhibition of TRE luciferase at 0.2 µM** |
|---|---|---|---|
| **1** | C | **2** | C |
| **3** | A | **4** | B |
| **5** | A | **6** | B |
| **7** | A | **8** | B |
| **9** | A | **10** | A |
| **11** | B | **12** | C |
| **13** | B | **14** | A |
| **15** | A | **16** | A |
| **17** | B | **18** | B |
| **19** | C | **20** | C |
| **21** | C | **22** | C |
| **23** | C | **24** | C |
| **25** | C | **26** | B |
| **27** | A | **28** | A |
| **29** | C | **30** | C |
| **31** | A | **32** | A |
| **33** | A | **34** | B |
| **35** | A | **36** | A |
| **37** | C | **38** | B |
| **39** | B | **40** | A |
| **41** | A | **42** | A |
| **43** | A | **44** | C |
| **45** | A | **46** | C |
| **47** | B | **48** | A |
| **49** | C | **50** | A |
| **51** | C | **52** | A |
| **53** | A | **54** | A |
| **55** | A | **56** | A |
| **57** | C | **58** | C |
| **59** | B | **60** | C |
| **61** | A | **62** | B |
| **63** | B | **64** | A |
| **65** | A | **66** | A |
| **67** | B | **68** | A |
| **69** | B | **70** | B |
| **71** | B | **72** | B |
| **73** | C | **74** | A |
| **75** | C | **76** | B |
| **77** | A | **78** | C |
| **79** | B | **80** | B |
| **81** | C | **82** | C |
| **83** | C | **84** | C |
| **85** | A | **86** | A |
| **87** | C | **88** | B |
| **89** | A | **90** | A |
| **91** | A | **92** | A |
| **93** | B | **94** | C |
| **95** | A | **96** | A |
| **97** | A | **98** | C |
| **99** | A | **100** | A |
| **101** | A | **102** | C |
| **103** | A | **104** | A |
| **105** | A | | |

| | | | |
|---|---|---|---|
| Note) A: > 50% inhibition B: 30 to 50% inhibition C: < 30% inhibition | | | |

Referring to above table 8, it has been confirmed that the compounds according to the present invention effectively inhibit the transcription of luciferase by TEAD, and thus it has been confirmed that the expression of genes dependent on TEAD activity may be effectively inhibited. In other words, it has been confirmed that the compounds of the present invention may be advantageously used in preventing or treating cancer through the inhibition of the expression of these genes.

### Experimental Example 2. Evaluation of palmitoylation of TEAD protein

Each compound corresponding to a concentration of 5 µM was added to recombinant GST-TEAD1 proteins (209 to 426 a.a.) and cultured for 15 minutes. For an autopalmitoylation reaction of proteins after culturing, 3 µM alkyne palmitoyl-CoA (Cayman chemical) and 50 mM MES buffer (pH 6.4) were added and cultured for 30 minutes, after which 1% of sodium dodecyl sulfate (SDS) was added to stop the reaction. After that, biotinylation was performed through a CLICK (Click chemistry tools) reaction, and then a protein solution was concentrated to prepare a sample.

Analysis of the sample was performed using SDS-PAGE and streptavidin HRP (Thermo scientific). The resulting values were quantified using an Image J (NIH). For the intensity of the band obtained from Streptavidin blot, a palmitoylation inhibitory activity was analyzed based on the control group (DMSO). The normalization of each sample was performed using an anti-GST (91G1) (cell signaling) antibody.

**[Table 9]**

| **Compound No.** | **% of inhibition of TEAD1 palmitoylation at 5 µM** | **Compound No.** | **% of inhibition of TEAD1 palmitoylation at 5 µM** |
|---|---|---|---|
| **3** | A | **4** | B |
| **5** | A | **6** | A |
| **7** | A | **8** | B |
| **9** | A | **10** | A |
| **11** | B | **12** | C |
| **13** | A | **14** | B |
| **15** | A | **16** | C |
| **17** | B | **18** | A |
| **19** | C | **20** | C |
| **21** | C | **22** | C |
| **23** | C | **24** | A |
| **25** | B | **26** | A |
| **27** | A | **28** | A |
| **29** | B | **30** | B |
| **31** | B | **32** | B |
| **33** | B | **34** | B |
| **35** | B | **36** | A |
| **37** | A | **38** | A |
| **39** | B | **40** | A |
| **41** | B | **42** | A |
| **43** | A | **44** | B |
| **45** | B | **46** | B |
| **47** | A | **48** | B |
| **49** | B | **50** | A |
| **51** | A | **52** | B |
| **53** | C | **54** | B |
| **55** | B | **56** | C |
| **57** | B | **58** | A |
| **59** | A | **60** | B |
| **61** | C | **62** | B |
| **63** | B | **64** | B |
| **65** | A | **66** | A |
| **67** | C | **68** | B |
| **69** | B | **70** | C |
| **71** | C | **72** | B |
| **73** | C | **74** | B |
| **75** | B | **76** | B |
| **77** | B | **78** | B |
| **79** | B | **80** | B |
| **81** | C | **82** | C |
| **83** | C | **84** | C |
| **85** | A | **86** | A |
| **87** | B | **88** | B |
| **89** | B | **90** | A |
| **91** | A | **92** | B |
| **93** | B | **94** | C |
| **95** | B | **96** | B |
| **97** | C | **98** | C |
| **99** | B | **100** | C |
| **101** | B | **102** | C |
| **103** | B | **104** | C |
| **105** | B | | |

| | | | |
|---|---|---|---|
| Note) A: > 80% inhibition B: 40 to 80% inhibition C: < 40% inhibition | | | |

As can be seen from above table 9, it has been confirmed that the compounds according to the present invention may be advantageously used for preventing or treating cancer by inhibiting an autopalmitoylation of TEAD proteins and inhibiting an interaction between YAP/TAZ and TEAD.

### Experimental Example 3. Evaluation of inhibition of cancer cell proliferation

The NF2-deficient mesothelioma cell line NCI-H226 was purchased from ATCC (#CRL-5826). NCI-H226 cells were cultured in RRPMI medium (ATCC) containing penicillin (100 units/mL), streptomycin (100 µg/mL) and 10% fetal bovine serum (FBS) at 37°C and 5% CO₂.

In 24 hours after dispensing said NCI-H226 cells into a 96-well plate, the cell culture medium was removed all, and then each compound corresponding to a certain concentration section (0.01, 0.05, 0.1, 0.5, 1, 5, 10 µM) or a vehicle solvent (final 1% DMSO) was diluted with a culture medium containing 2% FBS, treated at 100 µL for each well, and then cultured at 37°C and 5% CO₂. In five days after said treatment, CellTiter-Glo^{®} 2.0 Reagent (Promega) was treated in the same amount as the culture medium to measure cell growth, and placed in a shaker for two minutes so as to be well mixed, after which the plate was cultured at room temperature for ten minutes to stabilize a luminescence signal. After that, for ATP measurement through an intracellular luciferase reaction, the luminescence signal was measured using a microplate reader (TECAN). The value of the maximum concentration (GI₅₀) at the moment when the cell proliferation was reduced by half was derived using the SoftMax Pro 5.4.1 program, and the results are shown in table 10 below.

**[Table 10]**

| **Compound No.** | **Cancer cell growth inhibition NCI-H226 (GI₅₀, µM)** |
|---|---|
| **3** | 0.07 |
| **27** | 0.08 |
| **40** | 0.22 |
| **42** | 0.01 |
| **43** | 0.22 |
| **50** | 0.28 |
| **54** | 0.17 |
| **65** | 0.05 |
| **66** | 0.05 |
| **77** | 0.03 |
| **86** | 0.03 |
| **90** | 0.03 |
| **92** | 0.05 |
| **95** | 0.3 |
| **105** | 0.26 |

As can be seen from above table 10, it has been found that the compounds of the present invention reduce the proliferation of cancer cells to 50% at a very low concentration. In other words, it has been confirmed that the compounds of the present invention significantly inhibit the proliferation of cancer cells, and thus may be effectively used in preventing or treating cancer.

### Experimental Example 4. Evaluation of in vivo anticancer efficacy using NF2-deficient mesothelioma xenograft mouse model

In order to confirm the anticancer efficacy of the compounds of the present invention in animals, a tumor growth inhibitory activity was evaluated by using an NF2-deficient mesothelioma xenograft mouse model. NCI-H226 cells for inoculation were cultured in RRPMI medium (ATCC) containing penicillin (100 units/mL), streptomycin (100 µg/mL) and 10% FBS at 37°C and 5% CO₂. The cultured cells were prepared to become 10⁷ cells per animal in five-week-old BALB/c nude mice (Charles river Japan) by using PBS (Gibco) buffer before inoculation, and cell transplantation was completed through subcutaneous inoculation.

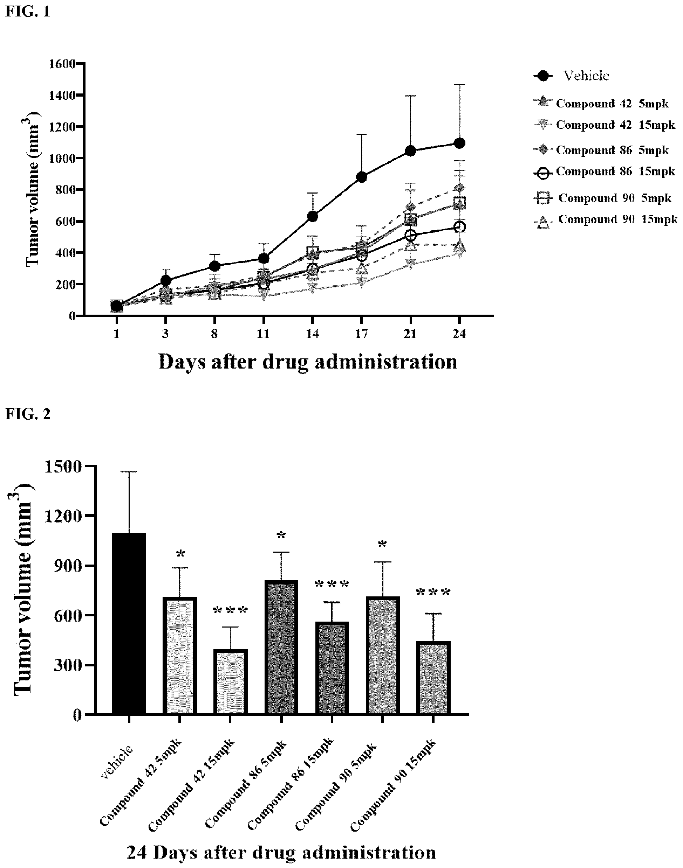

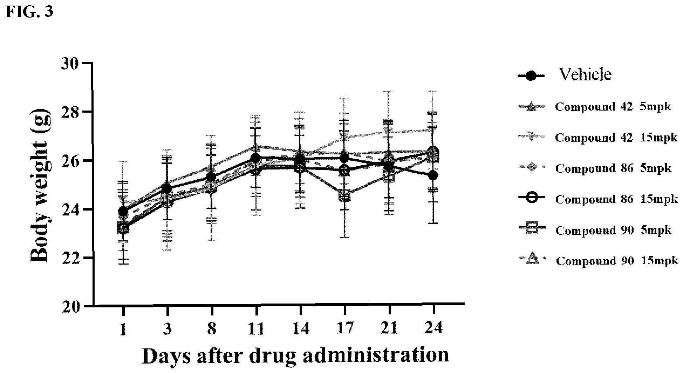

When the size of engrafted tumors reached 60 to 80 mm³, the animals were divided into a vehicle group (seven animals) and each drug administration group (seven animals) as an experimental group. The drug administration group was orally dosed with each of compounds (42, 86 and 90) corresponding to a predetermined dose (5 or 15 mpk (mg per kg)) once a day for 24 days. During the administration, a tumor size was measured to evaluate the anticancer efficacy, and a weight was also measured to confirm a toxicity reaction according to the drug. The statistical processing of results for evaluating the tumor growth inhibitory activity was performed using the Graphpad Prism 9 program, and the results are shown in FIGS. 1 to 3.

As can be seen from FIGS. 1 to 3, it has been found that the compound of the present invention not only exhibits an excellent tumor inhibitory activity, but also does not cause a weight loss or death of test animals, and thus it has been confirmed that the compound is safe without drug toxicity and may be advantageously used for preventing or treating cancer.

The present invention has been described with reference to one exemplary embodiment of the present invention, but it will be understood by those skilled in the art that the present invention may be variously changed and modified without departing from the spirit and field of the present invention, as described in the following scope of patent claims. Thus, it should be understood that the substantial scope of the present invention is defined by the accompanying claims and equivalents thereto.

## Claims

1. A compound represented by formula I below, a stereoisomer thereof, a pharmaceutically acceptable salt thereof, a hydrate thereof, or a solvate thereof: 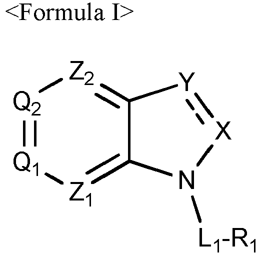 wherein in the formula I,
L₁ is a single bond, -(C1-C6 alkylene)- or -(C=O)-;
R₁ is H, C1-C3 alkyl, C1-C6 alkoxy, C3-C8 cycloalkyl, C3-C8 cycloalkenyl, 6- to 14-membered aryl, or 5- to 12-membered heteroaryl comprising in a ring 1 to 3 heteroatoms independently selected from the group consisting of N, O and S;
in the Ri, at least one H of C1-C3 alkyl, C1-C6 alkoxy, C3-C8 cycloalkyl, C3-C8 cycloalkenyl, 6- to 14-membered aryl, or 5- to 12-membered heteroaryl comprising in a ring 1 to 3 heteroatoms independently selected from the group consisting of N, O and S may be each independently substituted or unsubstituted with C1-C6 alkyl, C1-C6 alkoxy, C3-C8 cycloalkyl, CF₂H, CF₃, -OCF₃, -OH, -(C1-C3 alkylene)-OH, -(C=O)Rp, -COORq, -CN, -(C1-C3 alkylene)-CN, -NRxRy, -(C1-C3 alkylene)-NRsRt or halogen, in which Rp, Rq, Rs, Rt, Rx and Ry are each independently H or C1-C6 alkyl;
Z₁ and Z₂ are each independently N or CRa;
Ra is H or C1-C6 alkoxy;
Q₁ and Q₂ are each independently N, CH or CRb, in which at least one of Q₁ and Q₂ is CRb;
Rb is 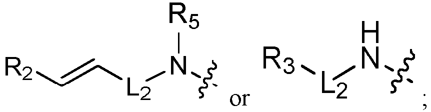
L₂ is -(C=O)- or S(=O)₂;
R₂, R₃ and R₅ are each independently H or C1-C6 alkyl;
X-̅ -̅ -̅Y is X-Y or X=Y;
when X-̅ -̅ -̅Y is X-Y, X and Y are each -CH₂-;
when X-̅ -̅ -̅Y is X=Y, X is -CH- or N, and Y is -CR₄ or N;
R₄ is H, C1-C6 alkyl or halogen; and
Z₁, Z₂, Q₁ and Q₂ are not all N, and when X-̅ -̅ -̅ Y is X=Y, at least one of X and Y is N.

2. The compound represented by formula I, the stereoisomer thereof, the pharmaceutically acceptable salt thereof, the hydrate thereof or the solvate thereof of claim 1,
wherein in the formula I,
L₁ is a single bond, -(C1-C6 alkylene)- or -(C=O)-;
R₁ is H, C1-C3 alkyl, C1-C6 alkoxy, C3-C8 cycloalkyl, C3-C8 cycloalkenyl, 6- to 14-membered aryl, or 5- to 12-membered heteroaryl comprising in a ring 1 to 3 heteroatoms independently selected from the group consisting of N, O and S;
in the Ri, at least one H of C1-C6 alkoxy, C3-C8 cycloalkyl, C3-C8 cycloalkenyl, 6-to 14-membered aryl, or 5- to 12-membered heteroaryl comprising in a ring 1 to 3 heteroatoms independently selected from the group consisting of N, O and S may be each independently substituted or unsubstituted with C1-C6 alkyl, C1-C6 alkoxy, C3-C8 cycloalkyl, CF₂H, CF₃, - OCF₃, -OH, -CN, -NRxRy or halogen, in which Rx and Ry are each independently H or C1-C6 alkyl;
Z₁ is Nor CH;
Z₂ is N or CRa;
Ra is H or C1-C6 alkoxy;
Q₁ and Q₂ are each independently N, CH or CRb, in which at least one of Q₁ and Q₂ is CRb;
Rb is 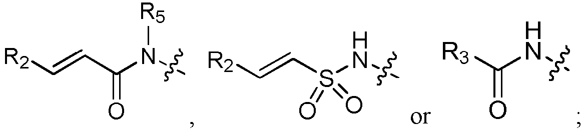
R₂ and R₅ are each independently H or C1-C6 alkyl;
R₃ is C1-C6 alkyl;
X_̅ _̅ _̅Y is X-Y or X=Y;
when X_̅ _̅ _̅Y is X-Y, X and Y are -CH₂-;
when X_̅ _̅ _̅Y is X=Y, X is -CH- or N, and Y is -CR₄ or N;
R₄ is H, C1-C6 alkyl or halogen; and
Z₁, Z₂, Q₁ and Q₂ are not all N, and when X_̅ _̅ _̅Y is X=Y, either X or Y is N.

3. The compound represented by formula I, the stereoisomer thereof, the pharmaceutically acceptable salt thereof, the hydrate thereof or the solvate thereof of claim 1,
wherein in the formula I,
L₁ is a single bond, -(C1-C6 alkylene)- or -(C=O)-;
R₁ is H, methoxy, cyclopentanyl, cyclohexanyl, cyclohexenyl, phenyl, pyridinyl, pyrazolyl, pyrimidinyl or thiophenyl;
in the Ri, at least one H of methoxy, cyclohexanyl, cyclohexenyl, phenyl, pyridinyl, pyrazolyl, pyrimidinyl or thiophenyl may be each independently substituted or unsubstituted with methyl, isopropyl, tert-butyl, sec-butyl, methoxy, isobutoxy, cyclohexyl, CF₂H, CF₃, -OCF₃, -OH, -CN, NH₂ or halogen;
Z₁ is Nor CH;
Z₂ is N or CRa;
Ra is H or methoxy;
Q₁ is N, CH or CRbi and Q₂ is N, CH or CRb₂, in which at least one of Q₁ and Q₂ is CRb₁ or CRb₂;
Rb₁ is 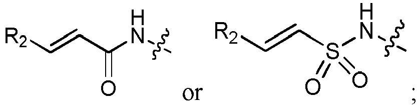
Rb₂ is 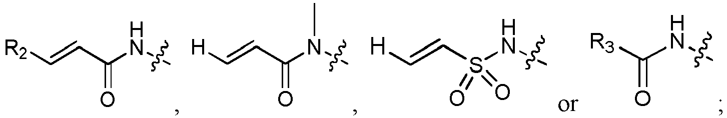
R₂ is H or methyl;
R₃ is isopropyl;
X_̅ _̅ _̅Y is X-Y or X=Y;
when X_̅ _̅ _̅Y is X-Y, X and Y are -CH₂-;
when X_̅ _̅ _̅Y is X=Y, X is -CH- or N, and Y is -CR₄ or N;
R₄ is H, methyl or halogen; and
Z₁, Z₂, Q₁ and Q₂ are not all N, and when X_̅ _̅ _̅Y is X=Y, either X or Y is N.

4. The compound represented by formula I, the stereoisomer thereof, the pharmaceutically acceptable salt thereof, the hydrate thereof or the solvate thereof of claim 1, wherein the compound represented by the formula I is represented by formula II below: 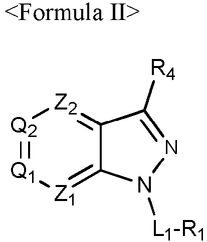 wherein in the formula II,
L₁ is a single bond or -(C1-C6 alkylene)-;
Ri is H, C1-C6 alkoxy, C3-C8 cycloalkyl, C3-C8 cycloalkenyl, 6- to 14-membered aryl, or 5- to 12-membered heteroaryl comprising in a ring 1 to 3 heteroatoms independently selected from the group consisting of N, O and S;
in the Ri, at least one H of C1-C6 alkoxy, C3-C8 cycloalkyl, C3-C8 cycloalkenyl, 6-to 14-membered aryl, or 5- to 12-membered heteroaryl comprising in a ring 1 to 3 heteroatoms independently selected from the group consisting of N, O and S may be each independently substituted or unsubstituted with C1-C6 alkyl, C1-C6 alkoxy, CF₂H, CF₃, -OCF₃, -OH, -CN, - NRxRy or halogen, in which Rx and Ry are each independently H or C1-C6 alkyl;
Z₁ and Z₂ are each independently N or CH;
Q₁ and Q₂ are each independently N, CH or CRb, in which at least one of Q₁ and Q₂ is CRb;
Rb is 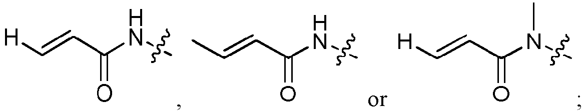
and
R₄ is H, C1-C6 alkyl or halogen.

5. The compound represented by formula I, the stereoisomer thereof, the pharmaceutically acceptable salt thereof, the hydrate thereof or the solvate thereof of claim 1, wherein the compound represented by the formula I is represented by formula III below: 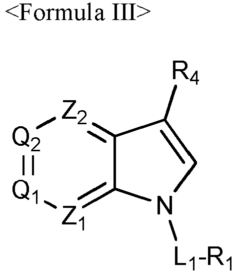 wherein in the formula III,
L₁ is a single bond, -(C1-C6 alkylene)- or -(C=O)-;
R₁ is H, C3-C8 cycloalkyl, 6- to 14-membered aryl, or 5- to 12-membered heteroaryl comprising in a ring 1 to 3 heteroatoms independently selected from the group consisting of N, O and S;
in the Ri, at least one H of C3-C8 cycloalkyl, 6- to 14-membered aryl, or 5- to 12-membered heteroaryl comprising in a ring 1 to 3 heteroatoms independently selected from the group consisting of N, O and S may be each independently substituted or unsubstituted with C1-C6 alkyl, C1-C6 alkoxy, C3-C8 cycloalkyl, CF₂H, CF₃, -OCF₃, or halogen;
Z₁ is Nor CH;
Z₂ is N or CRa;
Ra is H or C1-C6 alkoxy;
Q₁ is N or CH;
Q₂ is CRb;
Rb is 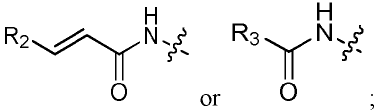
R₂ is H or C1-C6 alkyl;
R₃ is C1-C6 alkyl;
R₄ is H, C1-C6 alkyl or halogen; and
at least one of Z₁, Z₂, Q₁ and Q₂ is N.

6. The compound represented by formula I, the stereoisomer thereof, the pharmaceutically acceptable salt thereof, the hydrate thereof or the solvate thereof of claim 1, wherein the compound represented by the formula I is at least one selected from the group consisting of compounds shown in a following table:
| **Compound No.** | **Structure** | **Compound No.** | **Structure** |
|---|---|---|---|
| **1** | 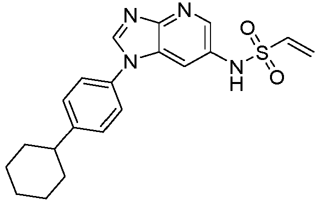 | **2** | 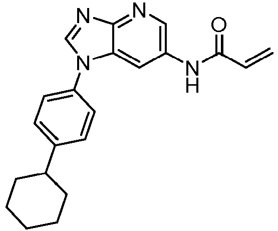 |
| **3** | 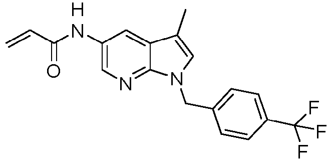 | **4** | 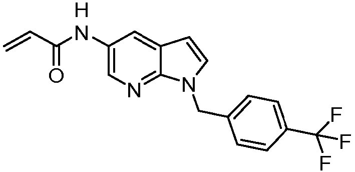 |
| **5** | 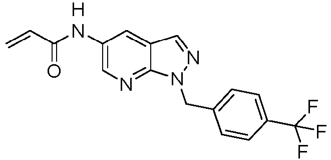 | **6** | 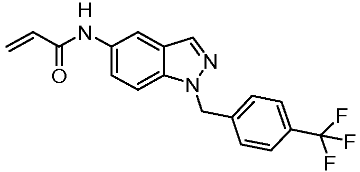 |
| **7** | 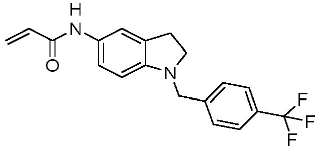 | **8** | 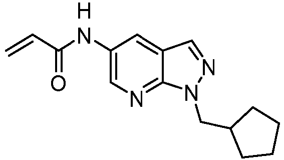 |
| **9** | 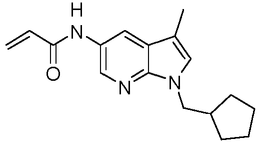 | **10** | 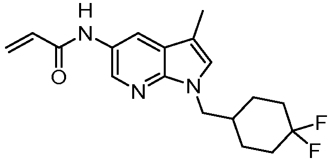 |
| **11** | 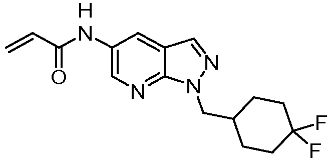 | **12** | 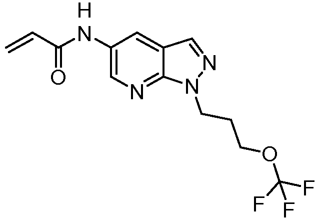 |
| **13** | 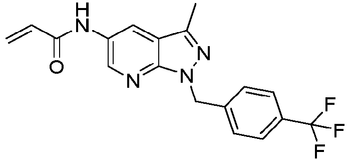 | **14** | 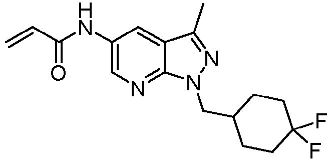 |
| **15** | 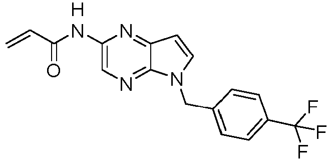 | **16** | 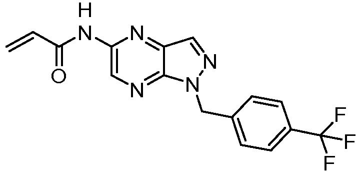 |
| **17** | 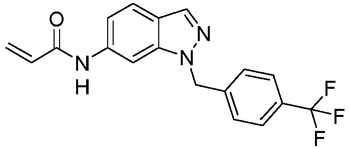 | **18** | 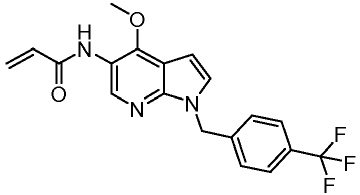 |
| **19** | 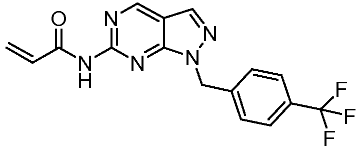 | **20** | 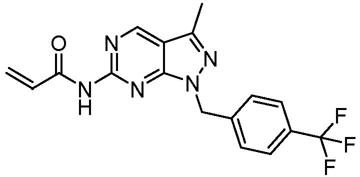 |
| **21** | 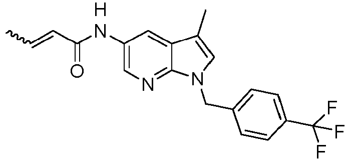 | **22** | 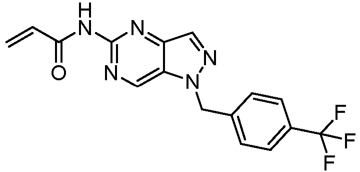 |
| **23** | 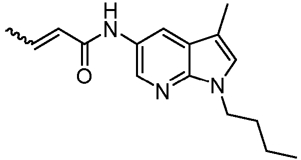 | **24** | 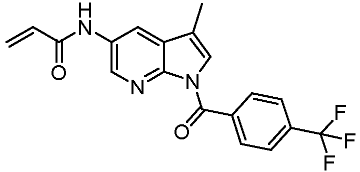 |
| **25** | 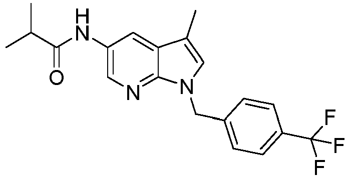 | **26** | 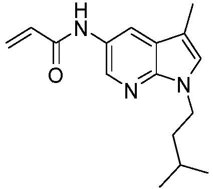 |
| **27** | 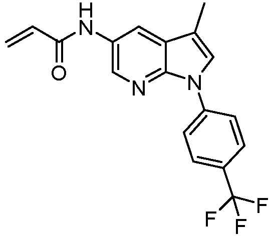 | **28** | 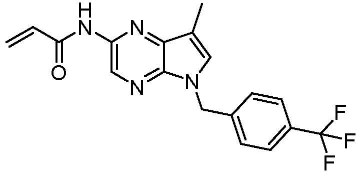 |
| **29** | 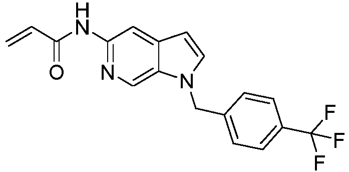 | **30** | 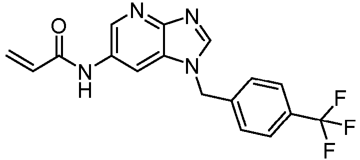 |
| **31** | 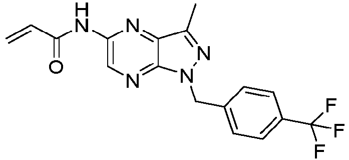 | **32** | 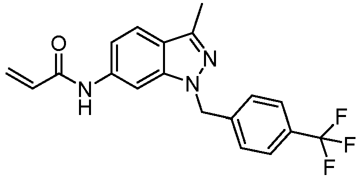 |
| **33** | 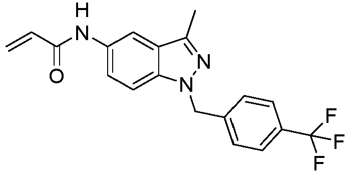 | **34** | 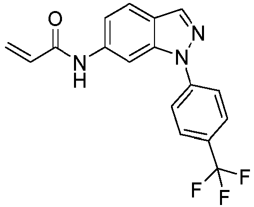 |
| **35** | 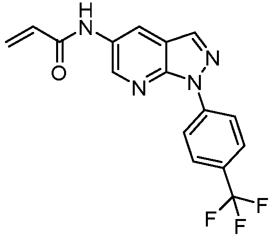 | **36** | 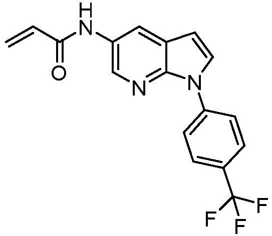 |
| **37** | 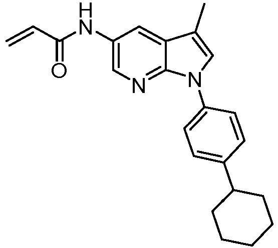 | **38** | 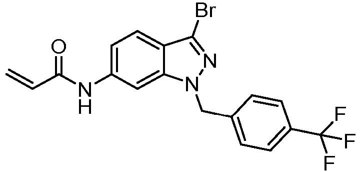 |
| **39** | 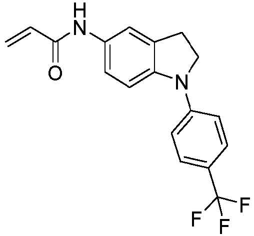 | **40** | 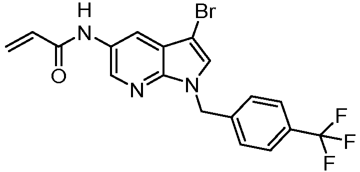 |
| **41** | 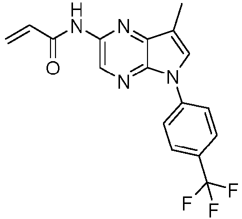 | **42** | 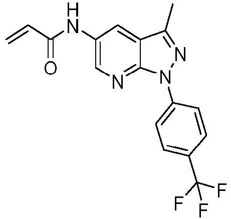 |
| **43** | 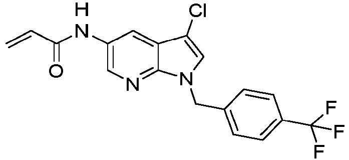 | **44** | 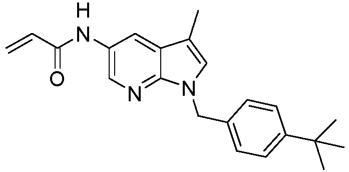 |
| **45** | 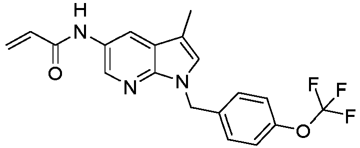 | **46** | 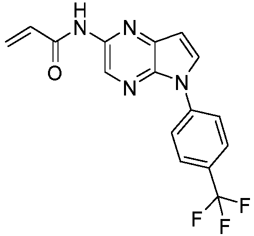 |
| **47** | 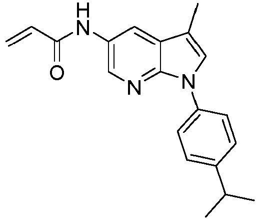 | **48** | 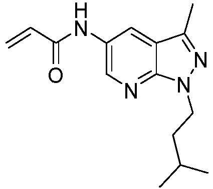 |
| **49** | 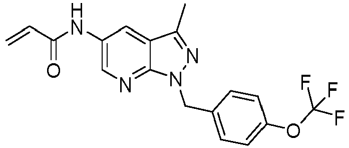 | **50** | 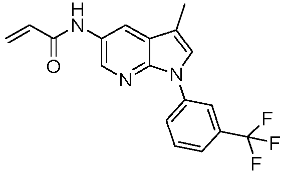 |
| **51** | 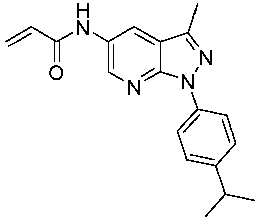 | **52** | 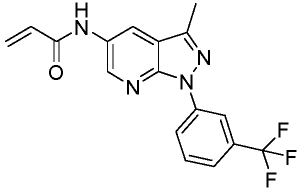 |
| **53** | 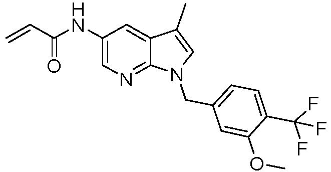 | **54** | 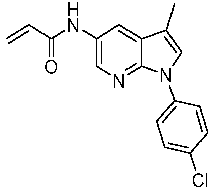 |
| **55** | 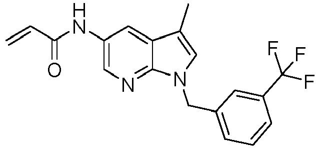 | **56** | 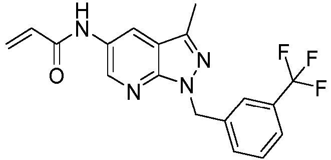 |
| **57** | 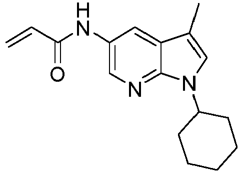 | **58** | 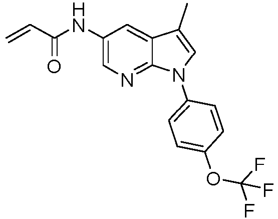 |
| **59** | 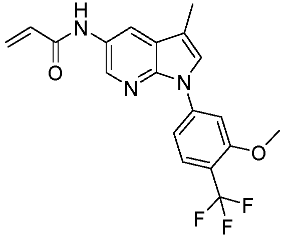 | **60** | 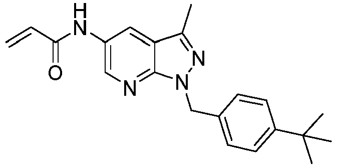 |
| **61** | 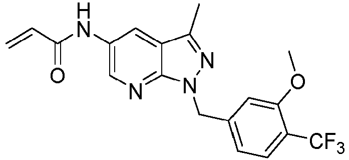 | **62** | 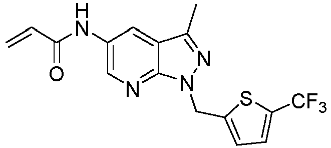 |
| **63** | 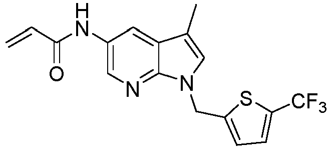 | **64** | 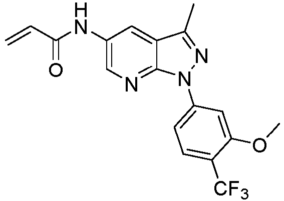 |
| **65** | 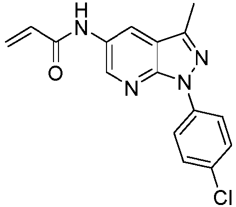 | **66** | 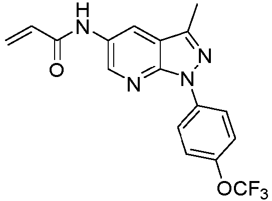 |
| **67** | 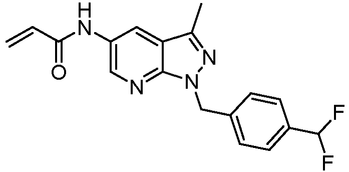 | **68** | 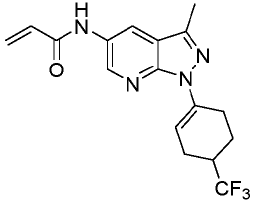 |
| **69** | 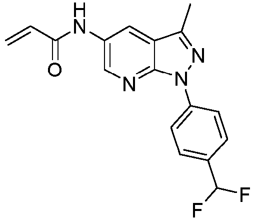 | **70** | 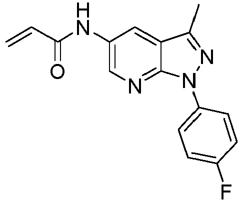 |
| **71** | 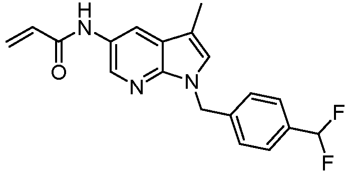 | **72** | 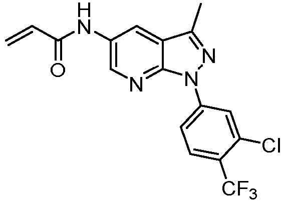 |
| **73** | 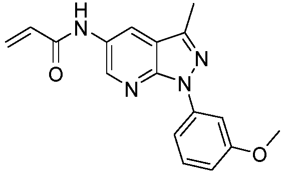 | **74** | 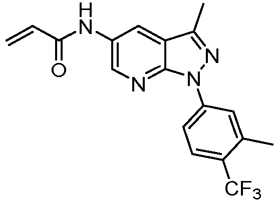 |
| **75** | 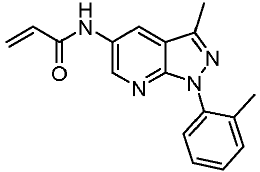 | **76** | 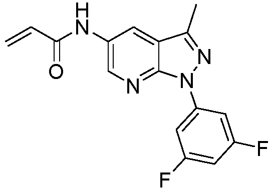 |
| **77** | 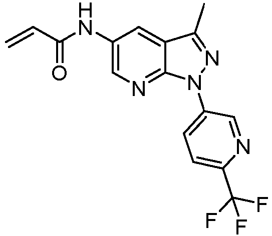 | **78** | 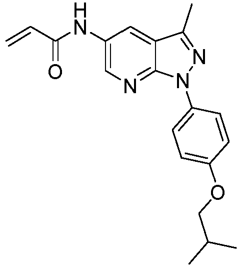 |
| **79** | 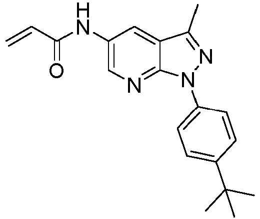 | **80** | 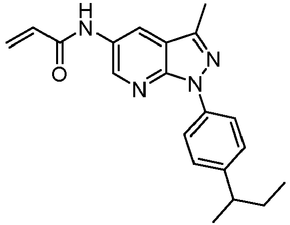 |
| **81** | 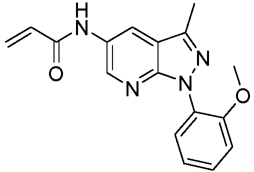 | **82** | 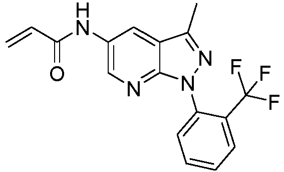 |
| **83** | 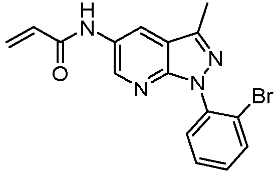 | **84** | 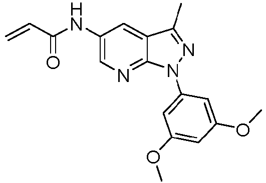 |
| **85** | 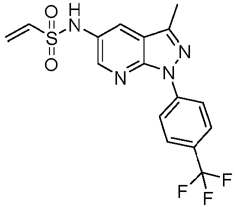 | **86** | 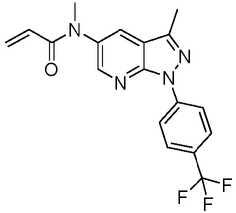 |
| **87** | 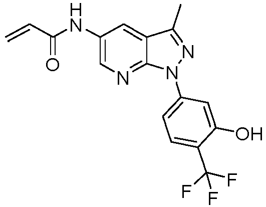 | **88** | 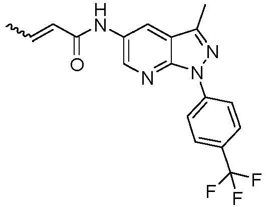 |
| **89** | 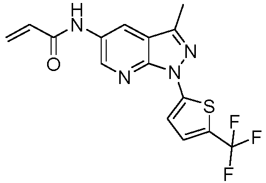 | **90** | 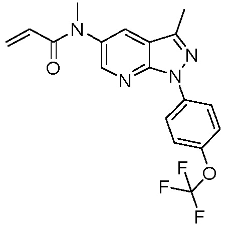 |
| **91** | 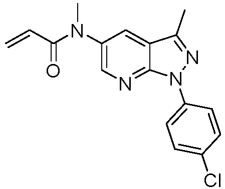 | **92** | 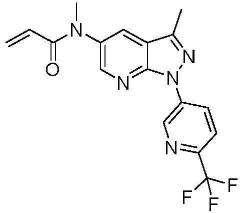 |
| **93** | 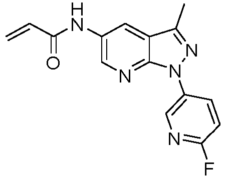 | **94** | 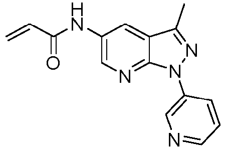 |
| **95** | 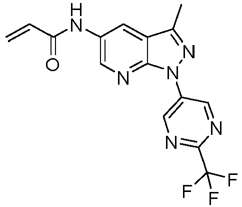 | **96** | 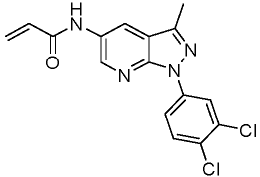 |
| **97** | 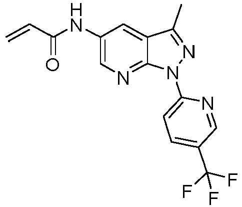 | **98** | 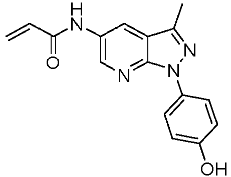 |
| **99** | 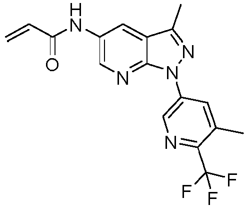 | **100** | 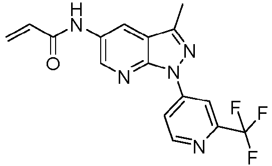 |
| **101** | 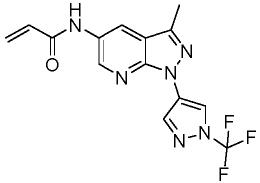 | **102** | 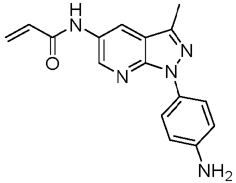 |
| **103** | 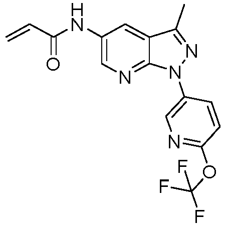 | **104** | 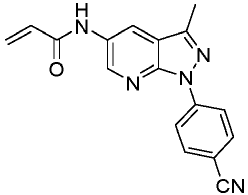 |
| **105** | 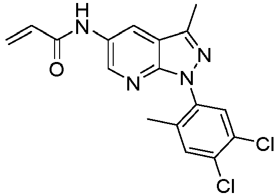 | | |

7. A pharmaceutical composition comprising the compound according to any one of claims 1 to 6, the stereoisomer thereof, the pharmaceutically acceptable salt thereof, the hydrate thereof or the solvate thereof as an active ingredient.

8. The pharmaceutical composition of claim 7, wherein the pharmaceutical composition is used for preventing or treating cancer.

9. A method for preventing or treating cancer, the method comprising: administering the compound according to any one of claims 1 to 6, the stereoisomer thereof, a pharmaceutically acceptable salt thereof, the hydrate thereof or the solvate thereof into an individual.

10. A use of the compound according to any one of claims 1 to 6, the stereoisomer thereof, the pharmaceutically acceptable salt thereof, the hydrate thereof or the solvate thereof for preventing or treating cancer.

11. A use of the compound according to any one of claims 1 to 6, a stereoisomer thereof, the pharmaceutically acceptable salt thereof, the hydrate thereof or the solvate thereof in preparation of a medicament for preventing or treating cancer.
